# EUROPEAN PATENT SPECIFICATION

(11) **EP 2 046 949 B1**
(45) Date of publication and mention of the grant of the patent: **15.01.2014**
(21) Application number: 07768453.8
(22) Date of filing: 12.07.2007
(51) Int. Cl.: C12N 9/04

(54) **A METHOD FOR PRODUCING AN L-AMINO ACID USING A BACTERIUM OF THE ENTEROBACTERIACEAE FAMILY**
VERFAHREN ZUR HERSTELLUNG EINER L-AMINOSÄURE UNTER VERWENDUNG EINES BAKTERIUMS DER FAMILIE ENTEROBACTERIACEAE
PROCÉDÉ PERMETTANT DE PRODUIRE UN ACIDE L-AMINÉ AU MOYEN D'UN BACTÉRIE DE LA FAMILLE ENTEROBACTERIACEAE

(30) Priority: 19.07.2006 RU 2006125964; 19.01.2007 US 885671 P
(43) Date of publication of application: 15.04.2009
(73) Proprietor: Ajinomoto Co., Inc., Tokyo 104-8315 (JP)
(72) Inventor: PTITSYN, Leonid Romanovich, Moscow 115404 (RU); ALTMAN, Irina Borisovna, Moscow 119435 (RU); KOTLIAROVA, Veronika Aleksandrovna, Moscow 117574 (RU); MOKHOVA, Olga, Nikolaevna, Moscow Region 141292 (RU); YAMPOLSKAYA, Tatyana Abramovna, Moscow 123364 (RU); KOZLOV, Yury Ivanovich, Moscow 117574 (RU); TERASHITA, Masaru, Kawasaki-shi Kanagawa 210-8681 (JP); USUDA, Yoshihiro, Kawasaki-shi Kanagawa 210-8681 (JP); MATSUI, Kazuhiko, Kawasaki-shi Kanagawa 210-8681 (JP)
(74) Representative: HOFFMANN EITLE
(86) International application number: PCT/JP2007/064304
(87) International publication number: WO 2008/010565

(56) References cited:
- US-A1- 2006 063 238
- HOLLAND-STALEY CAROL A ET AL: "Aerobic activity of Escherichia coli alcohol dehydrogenase is determined by a single amino acid" JOURNAL OF BACTERIOLOGY, vol. 182, no. 21, November 2000 (2000-11), pages 6049-6054, XP002472512 ISSN: 0021-9193 cited in the application
- MEMBRILLO-HERNANDEZ JORGE ET AL: "Evolution of the adhE gene product of Escherichia coli from a functional reductase to a dehydrogenase. Genetic and biochemical studies of the mutant proteins" JOURNAL OF BIOLOGICAL CHEMISTRY, vol. 275, no. 43, 27 October 2000 (2000-10-27), pages 33869-33875, XP002472513 ISSN: 0021-9258 cited in the application
- LEONARDO MICHAEL R ET AL: "Anaerobic regulation of the adhE gene, encoding the fermentative alcohol dehydrogenase of Escherichia coli" JOURNAL OF BACTERIOLOGY, vol. 175, no. 3, 1993, pages 870-878, XP002472514 ISSN: 0021-9193 cited in the application

### SEQUENCE LISTING

<110> Ajinomoto Co., Inc.
<120> A METHOD FOR PRODUCING AN L-AMINO ACID USING A BACTERIUM OF THE ENTEROBACTERIACEAE FAMILY
<130> C666-C7177
<150> RU2006125964
   <151> 2006-07-19
<150> US60/885671
   <151> 2007-01-19
<160> 58
<170> Patent In version 3.1
<210> 1
   <211> 2676
   <212> DNA
   <213> Escherichia coli
<220>
   <221> CDS
   <222> (1)..(2676)
<400> 1
   atg gct gtt act aat gtc gct gaa ctt aac gca ctc gta gag cgt gta        48
   Met Ala Val Thr Asn Val Ala Glu Leu Asn Ala Leu Val Glu Arg Val
   1               5                   10                  15
   aaa aaa gcc cag cgt gaa tat gcc agt ttc act caa gag caa gta gac        96
   Lys Lys Ala Gln Arg Glu Tyr Ala Ser Phe Thr Gln Glu Gln Val Asp
               20                  25                  30
   aaa atc ttc cgc gcc gcc gct ctg gct gct gca gat gct cga atc cca       144
   Lys Ile Phe Arg Ala Ala Ala Leu Ala Ala Ala Asp Ala Arg Ile Pro
           35                  40                  45
   ctc gcg aaa atg gcc gtt gcc gaa tcc ggc atg ggt atc gtc gaa gat       192
   Leu Ala Lys Met Ala Val Ala Glu Ser Gly Met Gly Ile Val Glu Asp
       50                  55                  60
   aaa gtg atc aaa aac cac ttt gct tct gaa tat atc tac aac gcc tat      240
   Lys Val Ile Lys Asn His Phe Ala Ser Glu Tyr Ile Tyr Asn Ala Tyr
   65                  70                  75                  80
   aaa gat gaa aaa acc tgt ggt gtt ctg tct gaa gac gac act ttt ggt      288
   Lys Asp Glu Lys Thr Cys Gly Val Leu Ser Glu Asp Asp Thr Phe Gly
                   85                  90                  95
   acc atc act atc gct gaa cca atc ggt att att tgc ggt atc gtt ccg      336
   Thr Ile Thr Ile Ala Glu Pro Ile Gly Ile Ile Cys Gly Ile Val Pro
               100                 105                 110
   acc act aac ccg act tca act gct atc ttc aaa tcg ctg atc agt ctg      384
   Thr Thr Asn Pro Thr Ser Thr Ala Ile Phe Lys Ser Leu Ile Ser Leu
           115                 120                 125
   aag acc cgt aac gcc att atc ttc tcc ccg cac ccg cgt gca aaa gat      432
   Lys Thr Arg Asn Ala Ile Ile Phe Ser Pro His Pro Arg Ala Lys Asp
       130                 135                 140
   gcc acc aac aaa gcg gct gat atc gtt ctg cag gct gct atc gct gcc      480
   Ala Thr Asn Lys Ala Ala Asp Ile Val Leu Gln Ala Ala Ile Ala Ala
   145                 150                 155                 160
   ggt gct ccg aaa gat ctg atc ggc tgg atc gat caa cct tct gtt gaa      528
   Gly Ala Pro Lys Asp Leu Ile Gly Trp Ile Asp Gln Pro Ser Val Glu
                   165                 170                 175
   ctg tct aac gca ctg atg cac cac cca gac atc aac ctg atc ctc gcg      576
   Leu Ser Asn Ala Leu Met His His Pro Asp Ile Asn Leu Ile Leu Ala
               180                 185                 190
   act ggt ggt ccg ggc atg gtt aaa gcc gca tac agc tcc ggt aaa cca      624
   Thr Gly Gly Pro Gly Met Val Lys Ala Ala Tyr Ser Ser Gly Lys Pro
           195                 200                 205
   gct atc ggt gta ggc gcg ggc aac act cca gtt gtt atc gat gaa act      672
   Ala Ile Gly Val Gly Ala Gly Asn Thr Pro Val Val Ile Asp Glu Thr
       210                 215                 220
   gct gat atc aaa cgt gca gtt gca tct gta ctg atg tcc aaa acc ttc      720
   Ala Asp Ile Lys Arg Ala Val Ala Ser Val Leu Met Ser Lys Thr Phe
   225                 230                 235                 240
   gac aac ggc gta atc tgt gct tct gaa cag tct gtt gtt gtt gtt gac      768
   Asp Asn Gly Val Ile Cys Ala Ser Glu Gln Ser Val Val Val Val Asp
                   245                 250                 255
   tct gtt tat gac gct gta cgt gaa cgt ttt gca acc cac ggc ggc tat      816
   Ser Val Tyr Asp Ala Val Arg Glu Arg Phe Ala Thr His Gly Gly Tyr
               260                 265                 270
   ctg ttg cag ggt aaa gag ctg aaa gct gtt cag gat gtt atc ctg aaa      864
   Leu Leu Gln Gly Lys Glu Leu Lys Ala Val Gln Asp Val Ile Leu Lys
           275                 280                 285
   aac ggt gcg ctg aac gcg gct atc gtt ggt cag cca gcc tat aaa att      912
   Asn Gly Ala Leu Asn Ala Ala Ile Val Gly Gln Pro Ala Tyr Lys Ile
       290                 295                 300
   gct gaa ctg gca ggc ttc tct gta cca gaa aac acc aag att ctg atc      960
   Ala Glu Leu Ala Gly Phe Ser Val Pro Glu Asn Thr Lys Ile Leu Ile
   305                 310                 315                 320
   ggt gaa gtg acc gtt gtt gat gaa agc gaa ccg ttc gca cat gaa aaa     1008
   Gly Glu Val Thr Val Val Asp Glu Ser Glu Pro Phe Ala His Glu Lys
                   325                 330                 335
   ctg tcc ccg act ctg gca atg tac cgc gct aaa gat ttc gaa gac gcg     1056
   Leu Ser Pro Thr Leu Ala Met Tyr Arg Ala Lys Asp Phe Glu Asp Ala
               340                 345                 350
   gta gaa aaa gca gag aaa ctg gtt gct atg ggc ggt atc ggt cat acc     1104
   Val Glu Lys Ala Glu Lys Leu Val Ala Met Gly Gly Ile Gly His Thr
           355                 360                 365
   tct tgc ctg tac act gac cag gat aac caa ccg gct cgc gtt tct tac     1152
   Ser Cys Leu Tyr Thr Asp Gln Asp Asn Gln Pro Ala Arg Val Ser Tyr
       370                 375                 380
   ttc ggt cag aaa atg aaa acg gcg cgt atc ctg att aac acc cca gcg     1200
   Phe Gly Gln Lys Met Lys Thr Ala Arg Ile Leu Ile Asn Thr Pro Ala
   385                 390                 395                 400
   tct cag ggt ggt atc ggt gac ctg tat aac ttc aaa ctc gca cct tcc     1248
   Ser Gln Gly Gly Ile Gly Asp Leu Tyr Asn Phe Lys Leu Ala Pro Ser
                   405                 410                 415
   ctg act ctg ggt tgt ggt tct tgg ggt ggt aac tcc atc tct gaa aac     1296
   Leu Thr Leu Gly Cys Gly Ser Trp Gly Gly Asn Ser Ile Ser Glu Asn
               420                 425                 430
   gtt ggt ccg aaa cac ctg atc aac aag aaa acc gtt gct aag cga gct     1344
   Val Gly Pro Lys His Leu Ile Asn Lys Lys Thr Val Ala Lys Arg Ala
           435                 440                 445
   gaa aac atg ttg tgg cac aaa ctt ccg aaa tct atc tac ttc cgc cgt     1392
   Glu Asn Met Leu Trp His Lys Leu Pro Lys Ser Ile Tyr Phe Arg Arg
       450                 455                 460
   ggc tcc ctg cca atc gcg ctg gat gaa gtg att act gat ggc cac aaa     1440
   Gly Ser Leu Pro Ile Ala Leu Asp Glu Val Ile Thr Asp Gly His Lys
   465                 470                 475                 480
   cgt gcg ctc atc gtg act gac cgc ttc ctg ttc aac aat ggt tat gct     1488
   Arg Ala Leu Ile Val Thr Asp Arg Phe Leu Phe Asn Asn Gly Tyr Ala
                   485                 490                 495
   gat cag atc act tcc gta ctg aaa gca gca ggc gtt gaa act gaa gtc     1536
   Asp Gln Ile Thr Ser Val Leu Lys Ala Ala Gly Val Glu Thr Glu Val
               500                 505                 510
   ttc ttc gaa gta gaa gcg gac ccg acc ctg agc atc gtt cgt aaa ggt     1584
   Phe Phe Glu Val Glu Ala Asp Pro Thr Leu Ser Ile Val Arg Lys Gly
           515                 520                 525
   gca gaa ctg gca aac tcc ttc aaa cca gac gtg att atc gcg ctg ggt     1632
   Ala Glu Leu Ala Asn Ser Phe Lys Pro Asp Val Ile Ile Ala Leu Gly
       530                 535                 540
   ggt ggt tcc ccg atg gac gcc gcg aag atc atg tgg gtt atg tac gaa     1680
   Gly Gly Ser Pro Met Asp Ala Ala Lys Ile Met Trp Val Met Tyr Glu
   545                 550                 555                 560
   cat ccg gaa act cac ttc gaa gag ctg gcg ctg cgc ttt atg gat atc     1728
   His Pro Glu Thr His Phe Glu Glu Leu Ala Leu Arg Phe Met Asp Ile
                   565                 570                 575
   cgt aaa cgt atc tac aag ttc ccg aaa atg ggc gtg aaa gcg aaa atg     1776
   Arg Lys Arg Ile Tyr Lys Phe Pro Lys Met Gly Val Lys Ala Lys Met
               580                 585                 590
   atc gct gtc acc acc act tct ggt aca ggt tct gaa gtc act ccg ttt     1824
   Ile Ala Val Thr Thr Thr Ser Gly Thr Gly Ser Glu Val Thr Pro Phe
           595                 600                 605
   gcg gtt gta act gac gac gct act ggt cag aaa tat ccg ctg gca gac     1872
   Ala Val Val Thr Asp Asp Ala Thr Gly Gln Lys Tyr Pro Leu Ala Asp
       610                 615                 620
   tat gcg ctg act ccg gat atg gcg att gtc gac gcc aac ctg gtt atg     1920
   Tyr Ala Leu Thr Pro Asp Met Ala Ile Val Asp Ala Asn Leu Val Met
   625                 630                 635                 640
   gac atg ccg aag tcc ctg tgt gct ttc ggt ggt ctg gac gca gta act     1968
   Asp Met Pro Lys Ser Leu Cys Ala Phe Gly Gly Leu Asp Ala Val Thr
                   645                 650                 655
   cac gcc atg gaa gct tat gtt tct gta ctg gca tct gag ttc tct gat     2016
   His Ala Met Glu Ala Tyr Val Ser Val Leu Ala Ser Glu Phe Ser Asp
               660                 665                 670
   ggt cag gct ctg cag gca ctg aaa ctg ctg aaa gaa tat ctg cca gcg     2064
   Gly Gln Ala Leu Gln Ala Leu Lys Leu Leu Lys Glu Tyr Leu Pro Ala
           675                 680                 685
   tcc tac cac gaa ggg tct aaa aat ccg gta gcg cgt gaa cgt gtt cac     2112
   Ser Tyr His Glu Gly Ser Lys Asn Pro Val Ala Arg Glu Arg Val His
       690                 695                 700
   agt gca gcg act atc gcg ggt atc gcg ttt gcg aac gcc ttc ctg ggt      2160
   Ser Ala Ala Thr Ile Ala Gly Ile Ala Phe Ala Asn Ala Phe Leu Gly
   705                 710                 715                 720
   gta tgt cac tca atg gcg cac aaa ctg ggt tcc cag ttc cat att ccg      2208
   Val Cys His Ser Met Ala His Lys Leu Gly Ser Gln Phe His Ile Pro
                   725                 730                 735
   cac ggt ctg gca aac gcc ctg ctg att tgt aac gtt att cgc tac aat      2256
   His Gly Leu Ala Asn Ala Leu Leu Ile Cys Asn Val Ile Arg Tyr Asn
               740                 745                 750
   gcg aac gac aac ccg acc aag cag act gca ttc agc cag tat gac cgt      2304
   Ala Asn Asp Asn Pro Thr Lys Gln Thr Ala Phe Ser Gln Tyr Asp Arg
           755                 760                 765
   ccg cag gct cgc cgt cgt tat gct gaa att gcc gac cac ttg ggt ctg      2352
   Pro Gln Ala Arg Arg Arg Tyr Ala Glu Ile Ala Asp His Leu Gly Leu
       770                 775                 780
   agc gca ccg ggc gac cgt act gct gct aag atc gag aaa ctg ctg gca      2400
   Ser Ala Pro Gly Asp Arg Thr Ala Ala Lys Ile Glu Lys Leu Leu Ala
   785                 790                 795                 800
   tgg ctg gaa acg ctg aaa gct gaa ctg ggt att ccg aaa tct atc cgt      2448
   Trp Leu Glu Thr Leu Lys Ala Glu Leu Gly Ile Pro Lys Ser Ile Arg
                   805                 810                 815
   gaa gct ggc gtt cag gaa gca gac ttc ctg gcg aac gtg gat aaa ctg      2496
   Glu Ala Gly Val Gln Glu Ala Asp Phe Leu Ala Asn Val Asp Lys Leu
               820                 825                 830
   tct gaa gat gca ttc gat gac cag tgc acc ggc gct aac ccg cgt tac      2544
   Ser Glu Asp Ala Phe Asp Asp Gln Cys Thr Gly Ala Asn Pro Arg Tyr
           835                 840                 845
   ccg ctg atc tcc gag ctg aaa cag att ctg ctg gat acc tac tac ggt      2592
   Pro Leu Ile Ser Glu Leu Lys Gln Ile Leu Leu Asp Thr Tyr Tyr Gly
       850                 855                 860
   cgt gat tat gta gaa ggt gaa act gca gcg aag aaa gaa gct gct ccg      2640
   Arg Asp Tyr Val Glu Gly Glu Thr Ala Ala Lys Lys Glu Ala Ala Pro
   865                 870                 875                 880
   gct aaa gct gag aaa aaa gcg aaa aaa tcc gct taa                      2676
   Ala Lys Ala Glu Lys Lys Ala Lys Lys Ser Ala
                   885                 890
<210> 2
   <211> 891
   <212> PRT
   <213> Escherichia coli
<400> 2
   Met Ala Val Thr Asn Val Ala Glu Leu Asn Ala Leu Val Glu Arg Val
   1               5                   10                  15
   Lys Lys Ala Gln Arg Glu Tyr Ala Ser Phe Thr Gln Glu Gln Val Asp
               20                  25                  30
   Lys Ile Phe Arg Ala Ala Ala Leu Ala Ala Ala Asp Ala Arg Ile Pro
           35                  40                  45
   Leu Ala Lys Met Ala Val Ala Glu Ser Gly Met Gly Ile Val Glu Asp
       50                  55                  60
   Lys Val Ile Lys Asn His Phe Ala Ser Glu Tyr Ile Tyr Asn Ala Tyr
   65                  70                  75                  80
   Lys Asp Glu Lys Thr Cys Gly Val Leu Ser Glu Asp Asp Thr Phe Gly
                   85                  90                  95
   Thr Ile Thr Ile Ala Glu Pro Ile Gly Ile Ile Cys Gly Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Ile Phe Ser Pro His Pro Arg Ala Lys Asp
       130                 135                 140
   Ala Thr Asn Lys Ala Ala Asp Ile Val Leu Gln Ala Ala Ile Ala Ala
   145                 150                 155                 160
   Gly Ala Pro Lys Asp Leu Ile Gly Trp Ile Asp Gln Pro Ser Val Glu
                   165                 170                 175
   Leu Ser Asn Ala Leu Met His His Pro Asp Ile Asn Leu Ile Leu Ala
               180                 185                 190
   Thr Gly Gly Pro Gly Met Val Lys Ala Ala Tyr Ser Ser Gly Lys Pro
           195                 200                 205
   Ala Ile Gly Val Gly Ala Gly Asn Thr Pro Val Val Ile Asp Glu Thr
       210                 215                 220
   Ala Asp Ile Lys Arg Ala Val Ala Ser Val Leu Met Ser Lys Thr Phe
   225                 230                 235                 240
   Asp Asn Gly Val Ile Cys Ala Ser Glu Gln Ser Val Val Val Val Asp
                   245                 250                 255
   Ser Val Tyr Asp Ala Val Arg Glu Arg Phe Ala Thr His Gly Gly Tyr
               260                 265                 270
   Leu Leu Gln Gly Lys Glu Leu Lys Ala Val Gln Asp Val Ile Leu Lys
           275                 280                 285
   Asn Gly Ala Leu Asn Ala Ala Ile Val Gly Gln Pro Ala Tyr Lys Ile
       290                 295                 300
   Ala Glu Leu Ala Gly Phe Ser Val Pro Glu Asn Thr Lys Ile Leu Ile
   305                 310                 315                 320
   Gly Glu Val Thr Val Val Asp Glu Ser Glu Pro Phe Ala His Glu Lys
                   325                 330                 335
   Leu Ser Pro Thr Leu Ala Met Tyr Arg Ala Lys Asp Phe Glu Asp Ala
               340                 345                 350
   Val Glu Lys Ala Glu Lys Leu Val Ala Met Gly Gly Ile Gly His Thr
           355                 360                 365
   Ser Cys Leu Tyr Thr Asp Gln Asp Asn Gln Pro Ala Arg Val Ser Tyr
       370                 375                 380
   Phe Gly Gln Lys Met Lys Thr Ala Arg Ile Leu Ile Asn Thr Pro Ala
   385                 390                 395                 400
   Ser Gln Gly Gly Ile Gly Asp Leu Tyr Asn Phe Lys Leu Ala Pro Ser
                   405                 410                 415
   Leu Thr Leu Gly Cys Gly Ser Trp Gly Gly Asn Ser Ile Ser Glu Asn
               420                 425                 430
   Val Gly Pro Lys His Leu Ile Asn Lys Lys Thr Val Ala Lys Arg Ala
           435                 440                 445
   Glu Asn Met Leu Trp His Lys Leu Pro Lys Ser Ile Tyr Phe Arg Arg
       450                 455                 460
   Gly Ser Leu Pro Ile Ala Leu Asp Glu Val Ile Thr Asp Gly His Lys
   465                 470                 475                 480
   Arg Ala Leu Ile Val Thr Asp Arg Phe Leu Phe Asn Asn Gly Tyr Ala
                   485                 490                 495
   Asp Gln Ile Thr Ser Val Leu Lys Ala Ala Gly Val Glu Thr Glu Val
               500                 505                 510
   Phe Phe Glu Val Glu Ala Asp Pro Thr Leu Ser Ile Val Arg Lys Gly
           515                 520                 525
   Ala Glu Leu Ala Asn Ser Phe Lys Pro Asp Val Ile Ile Ala Leu Gly
       530                 535                 540
   Gly Gly Ser Pro Met Asp Ala Ala Lys Ile Met Trp Val Met Tyr Glu
   545                 550                 555                 560
   His Pro Glu Thr His Phe Glu Glu Leu Ala Leu Arg Phe Met Asp Ile
                   565                 570                 575
   Arg Lys Arg Ile Tyr Lys Phe Pro Lys Met Gly Val Lys Ala Lys Met
               580                 585                 590
   Ile Ala Val Thr Thr Thr Ser Gly Thr Gly Ser Glu Val Thr Pro Phe
   595 600 605
   Ala Val Val Thr Asp Asp Ala Thr Gly Gln Lys Tyr Pro Leu Ala Asp
   610 615 620
   Tyr Ala Leu Thr Pro Asp Met Ala Ile Val Asp Ala Asn Leu Val Met
   625 630 635 640
   Asp Met Pro Lys Ser Leu Cys Ala Phe Gly Gly Leu Asp Ala Val Thr
   645 650 655
   His Ala Met Glu Ala Tyr Val Ser Val Leu Ala Ser Glu Phe Ser Asp
   660 665 670
   Gly Gln Ala Leu Gln Ala Leu Lys Leu Leu Lys Glu Tyr Leu Pro Ala
   675 680 685
   Ser Tyr His Glu Gly Ser Lys Asn Pro Val Ala Arg Glu Arg Val His
   690 695 700
   Ser Ala Ala Thr Ile Ala Gly Ile Ala Phe Ala Asn Ala Phe Leu Gly
   705 710 715 720
   Val Cys His Ser Met Ala His Lys Leu Gly Ser Gln Phe His Ile Pro
   725 730 735
   His Gly Leu Ala Asn Ala Leu Leu Ile Cys Asn Val Ile Arg Tyr Asn
   740 745 750
   Ala Asn Asp Asn Pro Thr Lys Gln Thr Ala Phe Ser Gln Tyr Asp Arg
   755 760 765
   Pro Gln Ala Arg Arg Arg Tyr Ala Glu Ile Ala Asp His Leu Gly Leu
   770 775 780
   Ser Ala Pro Gly Asp Arg Thr Ala Ala Lys Ile Glu Lys Leu Leu Ala
   785 790 795 800
   Trp Leu Glu Thr Leu Lys Ala Glu Leu Gly Ile Pro Lys Ser Ile Arg
   805 810 815
   Glu Ala Gly Val Gln Glu Ala Asp Phe Leu Ala Asn Val Asp Lys Leu
   820 825 830
   Ser Glu Asp Ala Phe Asp Asp Gln Cys Thr Gly Ala Asn Pro Arg Tyr
   835 840 845
   Pro Leu Ile Ser Glu Leu Lys Gln Ile Leu Leu Asp Thr Tyr Tyr Gly
   850 855 860
   Arg Asp Tyr Val Glu Gly Glu Thr Ala Ala Lys Lys Glu Ala Ala Pro
   865 870 875 880
   Ala Lys Ala Glu Lys Lys Ala Lys Lys Ser Ala
   885 890
<210> 3
   <211> 70
   <212> DNA
   <213> Artificial
<220>
   <223> primer P1
<400> 3
   gatgaaagcg ttatccaaac tgaaagcgga agaggccgac gcactttgcg ccgaataaat 60
   acctgtgacg 70
<210> 4
   <211> 69
   <212> DNA
   <213> Artificial
<220>
   <223> primer P2
<400> 4
   ttaatcccag ctcagaataa ctttcccgga ctttacgccc cgccctgcca ctcatcgcag 60
   tactgttgt 69
<210> 5
   <211> 18
   <212> DNA
   <213> Artificial
<220>
   <223> primer P3
<400> 5
   cggtcatgct tggtgatg 18
<210> 6
   <211> 21
   <212> DNA
   <213> Artificial
<220>
   <223> primer P4
<400> 6
   ttaatcccag ctcagaataa c 21
<210> 7
   <211> 183
   <212> DNA
   <213> Artificial
<220>
   <223> hybrid promoter
<400> 7
   ctagatctct cacctaccaa acaatgcccc cctgcaaaaa ataaattcat aaaaaacata 60
   cagataacca tctgcggtga taaattatct ctggcggtgt tgacaattaa tcatcggctc 120
   gtataatgtg tggaattgtg agcggtttaa cattatcagg agagcattat ggctgttact 180
   aat 183
<210> 8
   <211> 69
   <212> DNA
   <213> Artificial
<220>
   <223> primer P5
<400> 8
   cgttattgtt atctagttgt gcaaaacatg ctaatgtagc attacgcccc gccctgccac 60
   tcatcgcag 69
<210> 9
   <211> 58
   <212> DNA
   <213> Artificial
<220>
   <223> primer P6
<400> 9
   attagtaaca gccataatgc tctcctgata atgttaaacc gctcacaatt ccacacat 58
<210> 10
   <211> 24
   <212> DNA
   <213> Artificial
<220>
   <223> primer P7
<400> 10
   acttgttctt gagtgaaact ggca 24
<210> 11
   <211> 22
   <212> DNA
   <213> Artificial
<220>
   <223> primer P8
<400> 11
   aagacgcgct gacaatacgc ct 22
<210> 12
   <211> 69
   <212> DNA
   <213> Artificial
<220>
   <223> primer P9
<400> 12
   cgttattgtt atctagttgt gcaaaacatg ctaatgtagc atcagaaaaa ctcatcgagc 60
   atcaaatga 69
<210> 13
   <211> 65
   <212> DNA
   <213> Artificial
<220>
   <223> primer P10
<400> 13
   agccggagca gcttctttct tcgctgcagt ttcaccttct acgttgtgtc tcaaaatctc 60
   tgatg 65
<210> 14
   <211> 24
   <212> DNA
   <213> Artificial
<220>
   <223> primer P11
<400> 14
   aagacgcgct gacaatacgc cttt 24
<210> 15
   <211> 24
   <212> DNA
   <213> Artificial
<220>
   <223> primer P12
<400> 15
   aaggggccgt ttatgttgcc agac 24
<210> 16
   <211> 69
   <212> DNA
   <213> Artificial
<220>
   <223> primer P13
<400> 16
   catgtgggtt atgtacgaac atccggaaac tcacttcgaa aagtcggcgc tgcgctttat 60
   ggatatccg 69
<210> 17
   <211> 69
   <212> DNA
   <213> Artificial
<220>
   <223> primer P14
<400> 17
   atggacgccg cgaagatcat gtgggttatg tacgaacatc ccgttgtgtc tcaaaatctc 60
   tgatgttac 69
<210> 18
   <211> 67
   <212> DNA
   <213> Artificial
<220>
   <223> primer P15
<400> 18
   cattttcggg aacttgtaga tacgtttacg gatatccatt cagaaaaact catcgagcat 60
   caaatga 67
<210> 19
   <211> 23
   <212> DNA
   <213> Artificial
<220>
   <223> primer P16
<400> 19
   cttcgaagta gaagcggacc cga 23
<210> 20
   <211> 27
   <212> DNA
   <213> Artificial
<220>
   <223> primer P17
<400> 20
   ccagaagtgg tggtgacagc gatcatt 27
<210> 21
   <211> 75
   <212> DNA
   <213> Artificial
<220>
   <223> primer P18
<400> 21
   atggacgccg cgaagatcat gtgggttatg tacgaacatc cggaaactca cttcgaaaag 60
   ctggcgctgc gcttt 75
<210> 22
   <211> 73
   <212> DNA
   <213> Artificial
<220>
   <223> primer P19
<400> 22
   cattttcggg aacttgtaga tacgtttacg gatatccata aagcgcagcg ccagcttttc 60
   gaagtgagtt tcc 73
<210> 23
   <211> 31
   <212> DNA
   <213> Artificial
<220>
   <223> primer P20
<400> 23
   atcgaattca agacgcgctg acaatacgcc t 31
<210> 24
   <211> 27
   <212> DNA
   <213> Artificial
<220>
   <223> primer P21
<400> 24
   cacgctctac gagtgcgtta agttcag 27
<210> 25
   <211> 69
   <212> DNA
   <213> Artificial
<220>
   <223> primer P22
<400> 25
   aagcaaaagc cggataatgt tagccataaa taaggttgaa atcagaaaaa ctcatcgagc 60
   atcaaatga 69
<210> 26
   <211> 30
   <212> DNA
   <213> Artificial
<220>
   <223> primer P23
<400> 26
   ttcgaattcg ttgtgtctca aaatctccga 30
<210> 27
   <211> 21
   <212> DNA
   <213> Artificial
<220>
   <223> primer P24
<400> 27
   cgtcttcaga cagaacacca c 21
<210> 28
   <211> 23
   <212> DNA
   <213> Artificial
<220>
   <223> primer P25
<400> 28
   atgcttgatg gtcggaagag gca 23
<210> 29
   <211> 2688
   <212> DNA
   <213> Pantoea ananatis
<220>
   <221> CDS
   <222> (1)..(2688)
<400> 29
   atg gcc gtt act aat gtc gct gaa ctc aat gca ctg gtt gaa cgt gta       48
   Met Ala Val Thr Asn Val Ala Glu Leu Asn Ala Leu Val Glu Arg Val
   1               5                   10                  15
   aaa aaa gcc cag caa gaa ttc gcc aat ttt tct caa caa cag gtc gat       96
   Lys Lys Ala Gln Gln Glu Phe Ala Asn Phe Ser Gln Gln Gln Val Asp
               20                  25                  30
   gcc atc ttc cgc gca gcc gca ctg gcc gcc gcg gat gcc cga att cca      144
   Ala Ile Phe Arg Ala Ala Ala Leu Ala Ala Ala Asp Ala Arg Ile Pro
           35                  40                  45
   ctc gct aaa atg gcg gtg gcg gaa tcg ggc atg ggc att gtt gaa gac      192
   Leu Ala Lys Met Ala Val Ala Glu Ser Gly Met Gly Ile Val Glu Asp
       50                  55                  60
   aaa gtc att aaa aat cac ttc gct tct gaa tac atc tac aac gcc tat      240
   Lys Val Ile Lys Asn His Phe Ala Ser Glu Tyr Ile Tyr Asn Ala Tyr
   65                  70                  75                  80
   aag gat gag aaa acc tgc ggc gta ctg gac acc gat gat acg ttt ggc      288
   Lys Asp Glu Lys Thr Cys Gly Val Leu Asp Thr Asp Asp Thr Phe Gly
                   85                  90                  95
   acc atc acc atc gct gaa ccc atc ggc ctg att tgc ggt att gtc ccc      336
   Thr Ile Thr Ile Ala Glu Pro Ile Gly Leu Ile Cys Gly Ile Val Pro
               100                 105                 110
   acc act aac cct acc tcg acc gca att ttt aag gca ctt atc agc ctt      384
   Thr Thr Asn Pro Thr Ser Thr Ala Ile Phe Lys Ala Leu Ile Ser Leu
           115                 120                 125
   aaa acc cgc aac ggg att atc ttc tcc ccc cat cct cga gcc aaa gat      432
   Lys Thr Arg Asn Gly Ile Ile Phe Ser Pro His Pro Arg Ala Lys Asp
       130                 135                 140
   gcg acg aac aaa gcg gcg gat att gtc ctg cag gca gcg att gcc gct      480
   Ala Thr Asn Lys Ala Ala Asp Ile Val Leu Gln Ala Ala Ile Ala Ala
   145                 150                 155                 160
   ggc gcg ccc aaa gac att ata ggc tgg att gat gca cct tct gtg gaa      528
   Gly Ala Pro Lys Asp Ile Ile Gly Trp Ile Asp Ala Pro Ser Val Glu
                   165                 170                 175
   ctg tcc aat cag ttg atg cac cat cct gat att aac ctg att ctg gcg      576
   Leu Ser Asn Gln Leu Met His His Pro Asp Ile Asn Leu Ile Leu Ala
               180                 185                 190
   acg ggt ggc ccc ggc atg gtc aaa gcc gcc tac agc tca ggt aag ccg      624
   Thr Gly Gly Pro Gly Met Val Lys Ala Ala Tyr Ser Ser Gly Lys Pro
           195                 200                 205
   gcg att ggc gtg ggg gcc ggt aac acg ccc gtt gtc atc gat gaa aca      672
   Ala Ile Gly Val Gly Ala Gly Asn Thr Pro Val Val Ile Asp Glu Thr
       210                 215                 220
   gct gat gtt aaa cgc gcc gtt gcc tcc atc ctg atg tca aaa acg ttt      720
   Ala Asp Val Lys Arg Ala Val Ala Ser Ile Leu Met Ser Lys Thr Phe
   225                 230                 235                 240
   gat aac ggt gtg atc tgt gcc tct gaa cag tcg gtt atc gtg gtg gat      768
   Asp Asn Gly Val Ile Cys Ala Ser Glu Gln Ser Val Ile Val Val Asp
                   245                 250                 255
   gcc gtc tac gac gcc gtg cgc gag cgc ttc gcc agc cat ggt ggc tat      816
   Ala Val Tyr Asp Ala Val Arg Glu Arg Phe Ala Ser His Gly Gly Tyr
               260                 265                 270
   ttg ctt cag gga cag gag ctg agt gcg gta caa aat atc att cta aaa      864
   Leu Leu Gln Gly Gln Glu Leu Ser Ala Val Gln Asn Ile Ile Leu Lys
           275                 280                 285
   aac ggt ggg ctt aac gcc gcc att gtg ggc cag cct gcg gtg aag att      912
   Asn Gly Gly Leu Asn Ala Ala Ile Val Gly Gln Pro Ala Val Lys Ile
       290                 295                 300
   gcg gag atg gcc ggc atc agc gta cct ggt gaa acc aaa atc ctg att      960
   Ala Glu Met Ala Gly Ile Ser Val Pro Gly Glu Thr Lys Ile Leu Ile
   305                 310                 315                 320
   ggc gaa gtt gaa cgg gtc gat gaa tca gaa cct ttc gct cat gaa aaa     1008
   Gly Glu Val Glu Arg Val Asp Glu Ser Glu Pro Phe Ala His Glu Lys
                   325                 330                 335
   ctg tcg ccg aca ctg gcg atg tac cgt gct aaa gat tat cag gat gcc     1056
   Leu Ser Pro Thr Leu Ala Met Tyr Arg Ala Lys Asp Tyr Gln Asp Ala
               340                 345                 350
   gtc agc aaa gcg gag aaa ctg gtg gcg atg ggc ggt att ggt cat acg      1104
   Val Ser Lys Ala Glu Lys Leu Val Ala Met Gly Gly Ile Gly His Thr
           355                 360                 365
   tca tgc ctg tat acc gac cag gac aat cag aca gcg cgc gtg cac tat      1152
   Ser Cys Leu Tyr Thr Asp Gln Asp Asn Gln Thr Ala Arg Val His Tyr
       370                 375                 380
   ttt ggc gac aag atg aaa aca gcc cgc att ctg atc aac acg cca gct      1200
   Phe Gly Asp Lys Met Lys Thr Ala Arg Ile Leu Ile Asn Thr Pro Ala
   385                 390                 395                 400
   tct cag ggc ggt att ggt gat tta tat aac ttc aaa ctc gcc cct tct      1248
   Ser Gln Gly Gly Ile Gly Asp Leu Tyr Asn Phe Lys Leu Ala Pro Ser
                   405                 410                 415
   ctg aca ctg ggt tgt ggt tcc tgg ggc ggt aac tcc att tct gaa aac      1296
   Leu Thr Leu Gly Cys Gly Ser Trp Gly Gly Asn Ser Ile Ser Glu Asn
               420                 425                 430
   gtg ggg ccc aaa cat ctc atc aac aag aaa acc gtc gct aag cga gct      1344
   Val Gly Pro Lys His Leu Ile Asn Lys Lys Thr Val Ala Lys Arg Ala
           435                 440                 445
   gaa aat atg ttg tgg cat aaa ctt ccg aag tcc att tac ttt cgt cgc      1392
   Glu Asn Met Leu Trp His Lys Leu Pro Lys Ser Ile Tyr Phe Arg Arg
       450                 455                 460
   ggc tct tta ccc att gcg ctt gaa gag atc gcc act gac ggt gcc aaa      1440
   Gly Ser Leu Pro Ile Ala Leu Glu Glu Ile Ala Thr Asp Gly Ala Lys
   465                 470                 475                 480
   cgc gcg ttt gtg gtg act gac cgc ttc ctg ttt aac aac ggt tat gcc      1488
   Arg Ala Phe Val Val Thr Asp Arg Phe Leu Phe Asn Asn Gly Tyr Ala
                   485                 490                 495
   gat cag gtc acc cgc gtt tta aaa tct cac ggc atc gaa acc gaa gtt      1536
   Asp Gln Val Thr Arg Val Leu Lys Ser His Gly Ile Glu Thr Glu Val
               500                 505                 510
   ttc ttt gag gtt gaa gcg gat ccc acc tta agc atc gtg cgt aaa ggt      1584
   Phe Phe Glu Val Glu Ala Asp Pro Thr Leu Ser Ile Val Arg Lys Gly
           515                 520                 525
   gca gaa cag atg aac agc ttt aag cca gac gtg atc atc gcc ctg ggc      1632
   Ala Glu Gln Met Asn Ser Phe Lys Pro Asp Val Ile Ile Ala Leu Gly
       530                 535                 540
   ggc ggt tcg ccg atg gat gca gcc aaa atc atg tgg gtc atg tat gag      1680
   Gly Gly Ser Pro Met Asp Ala Ala Lys Ile Met Trp Val Met Tyr Glu
   545                 550                 555                 560
   cat cca gaa acc cat ttt gaa gag ctg gca ctg cgg ttt atg gat att      1728
   His Pro Glu Thr His Phe Glu Glu Leu Ala Leu Arg Phe Met Asp Ile
                   565                 570                 575
   cgc aaa cgt atc tat aag ttc cct aaa atg ggc gtg aaa gcg cgc atg      1776
   Arg Lys Arg Ile Tyr Lys Phe Pro Lys Met Gly Val Lys Ala Arg Met
               580                 585                 590
   gtg gcc att acg aca acc tca ggc aca ggt tca gaa gtg acg cct ttt      1824
   Val Ala Ile Thr Thr Thr Ser Gly Thr Gly Ser Glu Val Thr Pro Phe
           595                 600                 605
   gcc gtg gta acg gat gac gcg acc gga cag aaa tac ccg ctg gcc gat      1872
   Ala Val Val Thr Asp Asp Ala Thr Gly Gln Lys Tyr Pro Leu Ala Asp
       610                 615                 620
   tat gcg ctg acg ccg gat atg gct atc gtt gat gcc aac ctg gtc atg      1920
   Tyr Ala Leu Thr Pro Asp Met Ala Ile Val Asp Ala Asn Leu Val Met
   625                 630                 635                 640
   gat atg cca cgt tca ctt tgt gcc ttc ggc ggt ctg gat gcg gtg acg      1968
   Asp Met Pro Arg Ser Leu Cys Ala Phe Gly Gly Leu Asp Ala Val Thr
                   645                 650                 655
   cac gcg ctg gaa gcc tat gtg tcc gtt ctg gcc aat gaa tac tcc gat      2016
   His Ala Leu Glu Ala Tyr Val Ser Val Leu Ala Asn Glu Tyr Ser Asp
               660                 665                 670
   ggt cag gcc ctg cag gcg ctt aag ctg ctt aaa gag aac tta ccg gcg      2064
   Gly Gln Ala Leu Gln Ala Leu Lys Leu Leu Lys Glu Asn Leu Pro Ala
           675                 680                 685
   agt tat gca gaa ggt gca aaa aat ccg gtt gcc cgt gaa cgt gta cat      2112
   Ser Tyr Ala Glu Gly Ala Lys Asn Pro Val Ala Arg Glu Arg Val His
       690                 695                 700
   aat gcc gcc acc atc gcc ggt atc gcc ttt gct aac gcc ttc ctc ggg      2160
   Asn Ala Ala Thr Ile Ala Gly Ile Ala Phe Ala Asn Ala Phe Leu Gly
   705                 710                 715                 720
   gtt tgt cac tca atg gcg cat aag ctt ggc tct gag ttc cat att cct      2208
   Val Cys His Ser Met Ala His Lys Leu Gly Ser Glu Phe His Ile Pro
                   725                 730                 735
   cat gga ctg gct aac tcg ctg ctg att tcc aac gtt att cgc tat aac      2256
   His Gly Leu Ala Asn Ser Leu Leu Ile Ser Asn Val Ile Arg Tyr Asn
               740                 745                 750
   gcc aat gac aac cct act aag caa acc gca ttc agc cag tac gat cgt      2304
   Ala Asn Asp Asn Pro Thr Lys Gln Thr Ala Phe Ser Gln Tyr Asp Arg
           755                 760                 765
   ccc cag gcg cgt cgt cgt tat gct gaa att gcg gat cat ctt ggt ctc      2352
   Pro Gln Ala Arg Arg Arg Tyr Ala Glu Ile Ala Asp His Leu Gly Leu
       770                 775                 780
   acc gcg acg ggc gac cgc act gcc cag aaa att gag aag ctg ctg gta      2400
   Thr Ala Thr Gly Asp Arg Thr Ala Gln Lys Ile Glu Lys Leu Leu Val
   785                 790                 795                 800
   tgg ctg gat gag atc aaa acg gaa ctg ggt att ccg gca tcg att cgt      2448
   Trp Leu Asp Glu Ile Lys Thr Glu Leu Gly Ile Pro Ala Ser Ile Arg
                   805                 810                 815
   gaa gcc ggt gtg cag gag gca gac ttc ctg gcg aaa gtc gat aaa ctg      2496
   Glu Ala Gly Val Gln Glu Ala Asp Phe Leu Ala Lys Val Asp Lys Leu
               820                 825                 830
   gcg gat gat gcc ttt gat gac cag tgt act ggc gcg aat cca cgt tat      2544
   Ala Asp Asp Ala Phe Asp Asp Gln Cys Thr Gly Ala Asn Pro Arg Tyr
           835                 840                 845
   ccg ctg att gcc gaa ctc aaa cag ctg atg ctg gac agc tac tac gga      2592
   Pro Leu Ile Ala Glu Leu Lys Gln Leu Met Leu Asp Ser Tyr Tyr Gly
       850                 855                 860
   cgc aaa ttt gtc gag ccg ttc gcc agt gcc gcc gag gct gcc cag gct      2640
   Arg Lys Phe Val Glu Pro Phe Ala Ser Ala Ala Glu Ala Ala Gln Ala
   865                 870                 875                 880
   cag cct gtc agt gac agc aaa gcg gcg aag aaa gct aaa aaa gcc tag      2688
   Gln Pro Val Ser Asp Ser Lys Ala Ala Lys Lys Ala Lys Lys Ala
                   885                 890                 895
<210> 30
   <211> 895
   <212> PRT
   <213> Pantoea ananatis
<400> 30
   Met Ala Val Thr Asn Val Ala Glu Leu Asn Ala Leu Val Glu Arg Val
   1               5                   10                  15
   Lys Lys Ala Gln Gln Glu Phe Ala Asn Phe Ser Gln Gln Gln Val Asp
               20                  25                  30
   Ala Ile Phe Arg Ala Ala Ala Leu Ala Ala Ala Asp Ala Arg Ile Pro
           35                  40                  45
   Leu Ala Lys Met Ala Val Ala Glu Ser Gly Met Gly Ile Val Glu Asp
       50                  55                  60
   Lys Val Ile Lys Asn His Phe Ala Ser Glu Tyr Ile Tyr Asn Ala Tyr
   65 70 75 80
   Lys Asp Glu Lys Thr Cys Gly Val Leu Asp Thr Asp Asp Thr Phe Gly
   85 90 95
   Thr Ile Thr Ile Ala Glu Pro Ile Gly Leu Ile Cys Gly Ile Val Pro
   100 105 110
   Thr Thr Asn Pro Thr Ser Thr Ala Ile Phe Lys Ala Leu Ile Ser Leu
   115 120 125
   Lys Thr Arg Asn Gly Ile Ile Phe Ser Pro His Pro Arg Ala Lys Asp
   130 135 140
   Ala Thr Asn Lys Ala Ala Asp Ile Val Leu Gln Ala Ala Ile Ala Ala
   145 150 155 160
   Gly Ala Pro Lys Asp Ile Ile Gly Trp Ile Asp Ala Pro Ser Val Glu
   165 170 175
   Leu Ser Asn Gln Leu Met His His Pro Asp Ile Asn Leu Ile Leu Ala
   180 185 190
   Thr Gly Gly Pro Gly Met Val Lys Ala Ala Tyr Ser Ser Gly Lys Pro
   195 200 205
   Ala Ile Gly Val Gly Ala Gly Asn Thr Pro Val Val Ile Asp Glu Thr
   210 215 220
   Ala Asp Val Lys Arg Ala Val Ala Ser Ile Leu Met Ser Lys Thr Phe
   225 230 235 240
   Asp Asn Gly Val Ile Cys Ala Ser Glu Gln Ser Val Ile Val Val Asp
   245 250 255
   Ala Val Tyr Asp Ala Val Arg Glu Arg Phe Ala Ser His Gly Gly Tyr
   260 265 270
   Leu Leu Gln Gly Gln Glu Leu Ser Ala Val Gln Asn Ile Ile Leu Lys
   275 280 285
   Asn Gly Gly Leu Asn Ala Ala Ile Val Gly Gln Pro Ala Val Lys Ile
   290 295 300
   Ala Glu Met Ala Gly Ile Ser Val Pro Gly Glu Thr Lys Ile Leu Ile
   305 310 315 320
   Gly Glu Val Glu Arg Val Asp Glu Ser Glu Pro Phe Ala His Glu Lys
   325 330 335
   Leu Ser Pro Thr Leu Ala Met Tyr Arg Ala Lys Asp Tyr Gln Asp Ala
   340 345 350
   Val Ser Lys Ala Glu Lys Leu Val Ala Met Gly Gly Ile Gly His Thr
   355 360 365
   Ser Cys Leu Tyr Thr Asp Gln Asp Asn Gln Thr Ala Arg Val His Tyr
   370 375 380
   Phe Gly Asp Lys Met Lys Thr Ala Arg Ile Leu Ile Asn Thr Pro Ala
   385 390 395 400
   Ser Gln Gly Gly Ile Gly Asp Leu Tyr Asn Phe Lys Leu Ala Pro Ser
   405 410 415
   Leu Thr Leu Gly Cys Gly Ser Trp Gly Gly Asn Ser Ile Ser Glu Asn
   420 425 430
   Val Gly Pro Lys His Leu Ile Asn Lys Lys Thr Val Ala Lys Arg Ala
   435 440 445
   Glu Asn Met Leu Trp His Lys Leu Pro Lys Ser Ile Tyr Phe Arg Arg
   450 455 460
   Gly Ser Leu Pro Ile Ala Leu Glu Glu Ile Ala Thr Asp Gly Ala Lys
   465 470 475 480
   Arg Ala Phe Val Val Thr Asp Arg Phe Leu Phe Asn Asn Gly Tyr Ala
   485 490 495
   Asp Gln Val Thr Arg Val Leu Lys Ser His Gly Ile Glu Thr Glu Val
   500 505 510
   Phe Phe Glu Val Glu Ala Asp Pro Thr Leu Ser Ile Val Arg Lys Gly
   515 520 525
   Ala Glu Gln Met Asn Ser Phe Lys Pro Asp Val Ile Ile Ala Leu Gly
   530 535 540
   Gly Gly Ser Pro Met Asp Ala Ala Lys Ile Met Trp Val Met Tyr Glu
   545 550 555 560
   His Pro Glu Thr His Phe Glu Glu Leu Ala Leu Arg Phe Met Asp Ile
   565 570 575
   Arg Lys Arg Ile Tyr Lys Phe Pro Lys Met Gly Val Lys Ala Arg Met
   580 585 590
   Val Ala Ile Thr Thr Thr Ser Gly Thr Gly Ser Glu Val Thr Pro Phe
   595 600 605
   Ala Val Val Thr Asp Asp Ala Thr Gly Gln Lys Tyr Pro Leu Ala Asp
   610 615 620
   Tyr Ala Leu Thr Pro Asp Met Ala Ile Val Asp Ala Asn Leu Val Met
   625 630 635 640
   Asp Met Pro Arg Ser Leu Cys Ala Phe Gly Gly Leu Asp Ala Val Thr
   645 650 655
   His Ala Leu Glu Ala Tyr Val Ser Val Leu Ala Asn Glu Tyr Ser Asp
   660 665 670
   Gly Gln Ala Leu Gln Ala Leu Lys Leu Leu Lys Glu Asn Leu Pro Ala
   675 680 685
   Ser Tyr Ala Glu Gly Ala Lys Asn Pro Val Ala Arg Glu Arg Val His
   690 695 700
   Asn Ala Ala Thr Ile Ala Gly Ile Ala Phe Ala Asn Ala Phe Leu Gly
   705                 710                 715                 720
   Val Cys His Ser Met Ala His Lys Leu Gly Ser Glu Phe His Ile Pro
                   725                 730                 735
   His Gly Leu Ala Asn Ser Leu Leu Ile Ser Asn Val Ile Arg Tyr Asn
               740                 745                 750
   Ala Asn Asp Asn Pro Thr Lys Gln Thr Ala Phe Ser Gln Tyr Asp Arg
           755                 760                 765
   Pro Gln Ala Arg Arg Arg Tyr Ala Glu Ile Ala Asp His Leu Gly Leu
       770                 775                 780
   Thr Ala Thr Gly Asp Arg Thr Ala Gln Lys Ile Glu Lys Leu Leu Val
   785                 790                 795                 800
   Trp Leu Asp Glu Ile Lys Thr Glu Leu Gly Ile Pro Ala Ser Ile Arg
                   805                 810                 815
   Glu Ala Gly Val Gln Glu Ala Asp Phe Leu Ala Lys Val Asp Lys Leu
               820                 825                 830
   Ala Asp Asp Ala Phe Asp Asp Gln Cys Thr Gly Ala Asn Pro Arg Tyr
           835                 840                 845
   Pro Leu Ile Ala Glu Leu Lys Gln Leu Met Leu Asp Ser Tyr Tyr Gly
       850                 855                 860
   Arg Lys Phe Val Glu Pro Phe Ala Ser Ala Ala Glu Ala Ala Gln Ala
   865                 870                 875                 880
   Gln Pro Val Ser Asp Ser Lys Ala Ala Lys Lys Ala Lys Lys Ala
                   885                 890                 895
<210> 31
   <211> 69
   <212> DNA
   <213> Artificial
<220>
   <223> primer P26
<400> 31
   tgcacaataa tgttgtatca accaccatat cgggtgactt atcagaaaaa ctcatcgagc    60
   atcaaatga                                                            69
<210> 32
   <211> 64
   <212> DNA
   <213> Artificial
<220>
   <223> primer P27
<400> 32
   gcaacatttt ccccgccgtc agaaacgacg gggcagagat tcgttgtgtc tcaaaatctc 60
   gtat 64
<210> 33
   <211> 24
   <212> DNA
   <213> Artificial
<220>
   <223> primer P28
<400> 33
   aatcccgctc tttcataaca ttat 24
<210> 34
   <211> 23
   <212> DNA
   <213> Artificial
<220>
   <223> primer P29
<400> 34
   attaatcgca ggggaaagca ggg 23
<210> 35
   <211> 42
   <212> DNA
   <213> Artificial
<220>
   <223> primer P30
<400> 35
   atcatgcaaa gaggtgtgcc gtggtaaagg aacgtaaaac cg 42
<210> 36
   <211> 42
   <212> DNA
   <213> Artificial
<220>
   <223> primer P31
<400> 36
   atcatgcaaa gaggtgtgcc gtggtaaagg aacgtaaaac cg 42
<210> 37
   <211> 30
   <212> DNA
   <213> Artificial
<220>
   <223> primer P32
<400> 37
   gttggatcct gacatgcctc tcccgagcaa 30
<210> 38
   <211> 24
   <212> DNA
   <213> Artificial
<220>
   <223> primer P33
<400> 38
   ccacggcaca cctctttgca tgat 24
<210> 39
   <211> 61
   <212> DNA
   <213> Artificial
<220>
   <223> primer P34
<400> 39
   ttcacctttc ctcctgttta ttcttattac ccctgaagcc tgctttttta tactaagttg 60
   g 61
<210> 40
   <211> 64
   <212> DNA
   <213> Artificial
<220>
   <223> primer P35
<400> 40
   acatgttggg ctgtaaattg cgcattgaga tcattccgct caagttagta taaaaaagct 60
   gaac 64
<210> 41
   <211> 21
   <212> DNA
   <213> Artificial
<220>
   <223> primer P36
<400> 41
   ttgctgtaag ttgtgggatt c 21
<210> 42
   <211> 20
   <212> DNA
   <213> Artificial
<220>
   <223> primer P37
<400> 42
   tccaggttcc cactgatttc 20
<210> 43
   <211> 64
   <212> DNA
   <213> Artificial
<220>
   <223> primer P38
<400> 43
   gtttctcaag attcaggacg gggaactaac tatgaatgaa gcctgctttt ttatactaag 60
   ttgg 64
<210> 44
   <211> 64
   <212> DNA
   <213> Artificial
<220>
   <223> primer P39
<400> 44
   tcagctttct tcgtggtcat ttttatattc cttttgcgct caagttagta taaaaaagct 60
   gaac 64
<210> 45
   <211> 20
   <212> DNA
   <213> Artificial
<220>
   <223> primer P40
<400> 45
   tggtcgtgat tagcgtggtg 20
<210> 46
   <211> 20
   <212> DNA
   <213> Artificial
<220>
   <223> primer P41
<400> 46
   cacatgcacc ttcgcgtctt 20
<210> 47
   <211> 64
   <212> DNA
   <213> Artificial
<220>
   <223> primer P42
<400> 47
   ccgcaggcga ctgacgaaac ctcgctccgg cggggtcgct caagttagta taaaaaagct 60
   gaac 64
<210> 48
   <211> 64
   <212> DNA
   <213> Artificial
<220>
   <223> primer P43
<400> 48
   tgcccgaact tgccatgctc cagtctcctt cttctgagct gtttccttct agacggccaa 60
   tgct 64
<210> 49
   <211> 1968
   <212> DNA
   <213> artificial
<220>
   <223> DNA fragment containing cat gene and PL promoter
<400> 49
   ccgcaggcga ctgacgaaac ctcgctccgg cggggtcgct caagttagta taaaaaagct 60
   gaacgagaaa cgtaaaatga tataaatatc aatatattaa attagatttt gcataaaaaa 120
   cagactacat aatactgtaa aacacaacat atgcagtcac tatgaatcaa ctacttagat 180
   ggtattagtg acctgtaaca gactgcagtg gtcgaaaaaa aaagcccgca ctgtcaggtg 240
   cgggcttttt tctgtgttaa gcttcgacga atttctgcca ttcatccgct tattatcact 300
   tattcaggcg tagcaccagg cgtttaaggg caccaataac tgccttaaaa aaattacgcc 360
   ccgccctgcc actcatcgca gtactgttgt aattcattaa gcattctgcc gacatggaag 420
   ccatcacaga cggcatgatg aacctgaatc gccagcggca tcagcacctt gtcgccttgc 480
   gtataatatt tgcccatggt gaaaacgggg gcgaagaagt tgtccatatt ggccacgttt 540
   aaatcaaaac tggtgaaact cacccaggga ttggctgaga cgaaaaacat attctcaata 600
   aaccctttag ggaaataggc caggttttca ccgtaacacg ccacatcttg cgaatatatg 660
   tgtagaaact gccggaaatc gtcgtggtat tcactccaga gcgatgaaaa cgtttcagtt 720
   tgctcatgga aaacggtgta acaagggtga acactatccc atatcaccag ctcaccgtct 780
   ttcattgcca tacggaattc cggatgagca ttcatcaggc gggcaagaat gtgaataaag 840
   gccggataaa acttgtgctt atttttcttt acggtcttta aaaaggccgt aatatccagc 900
   tgaacggtct ggttataggt acattgagca actgactgaa atgcctcaaa atgttcttta 960
   cgatgccatt gggatatatc aacggtggta tatccagtga tttttttctc cattttagct 1020
   tccttagctc ctgaaaatct cggatccgat atctagctag agcgcccggt tgacgctgct 1080
   agtgttacct agcgatttgt atcttactgc atgttacttc atgttgtcaa tacctgtttt 1140
   tcgtgcgact tatcaggctg tctacttatc cggagatcca caggacgggt gtggtcgcca 1200
   tgatcgcgta gtcgatagtg gctccaagta gcgaagcgag caggactggg cggcggccaa 1260
   agcggtcgga cagtgctccg agaacgggtg cgcatagaaa ttgcatcaac gcatatagcg 1320
   ctagcagcac gccatagtga ctggcgatgc tgtcggaatg gacgatatcc cgcaagaggc 1380
   ccggcagtac cggcataacc aagcctatgc ctacagcatc cagggtgacg gtgccgagga 1440
   tgacgatgag cgcattgtta gatttcatac acggtgcctg actgcgttag caatttaact 1500
   gtgataaact accgcattaa agcttatcga tgataagctg tcaaacatga gaattcgaaa 1560
   tcaaataatg attttatttt gactgatagt gacctgttcg ttgcaacaaa ttgataagca 1620
   atgctttttt ataatgccaa cttagtataa aaaagcaggc ttcaagatct tcacctacca 1680
   aacaatgccc ccctgcaaaa aataaattca tataaaaaac atacagataa ccatctgcgg 1740
   tgataaatta tctctggcgg tgttgacata aataccactg gcggtgatac tgagcacatc 1800
   agcaggacgc actgaccacc atgaaggtga cgctcttaaa aattaagccc tgaagaaggg 1860
   cagcattcaa agcagaaggc tttggggtgt gtgatacgaa acgaagcatt ggccgtctag 1920
   aaggaaacag ctcagaagaa ggagactgga gcatggcaag ttcgggca 1968
<210> 50
   <211> 4971
   <212> DNA
   <213> DNA fragment
<400> 50
   aatctgatcc ttcactgccc gtgcacggct ctcattttcg acaaacggca gcgtgatgct     60
   gctgatagtg acgggaaatt gtgacattac tgcgtgtcct aatacctccg cagttattgc    120
   cgtaccatca gaaatataaa aaacgtggcg atcaacagca ttatccattt tgttcttccc    180
   gtgatgcaga cataattcta tgaaagcata aattaaaaac gcacagaagc gtagaacgtt    240
   atgtctggtt tataaaatga accttcaatt ttatttttta tgaaaacagc atttcatttt    300
   tatggtttcg tttataccga tggtttatgt ggaaattgtc gaagagagca gatttgcgca    360
   acgctgggat cagtcttaaa aagtaaaaaa atatatttgc ttgaacgatt caccgttttt    420
   ttcatccggt taaatatgca aagataaatg cgcagaaatg tgtttctcaa accgttcatt    480
   tatcacaaaa ggattgttcg atgtccaaca atggctcgta accgctggtg ctttggtcgc    540
   tcaagttagt ataaaaaagc tgaacgagaa acgtaaaatg atataaatat caatatatta    600
   aattagattt tgcataaaaa acagactaca taatactgta aaacacaaca tatgcagtca    660
   ctatgaatca actacttaga tggtattagt gacctgtaac agactgcagt ggtcgaaaaa    720
   aaaagcccgc actgtcaggt gcgggctttt ttctgtgtta agcttcgacg aatttctgcc    780
   attcatccgc ttattatcac ttattcaggc gtagcaccag gcgtttaagg gcaccaataa    840
   ctgccttaaa aaaattacgc cccgccctgc cactcatcgc agtactgttg taattcatta    900
   agcattctgc cgacatggaa gccatcacag acggcatgat gaacctgaat cgccagcggc    960
   atcagcacct tgtcgccttg cgtataatat ttgcccatgg tgaaaacggg ggcgaagaag   1020
   ttgtccatat tggccacgtt taaatcaaaa ctggtgaaac tcacccaggg attggctgag   1080
   acgaaaaaca tattctcaat aaacccttta gggaaatagg ccaggttttc accgtaacac   1140
   gccacatctt gcgaatatat gtgtagaaac tgccggaaat cgtcgtggta ttcactccag   1200
   agcgatgaaa acgtttcagt ttgctcatgg aaaacggtgt aacaagggtg aacactatcc 1260
   catatcacca gctcaccgtc tttcattgcc atacggaatt ccggatgagc attcatcagg 1320
   cgggcaagaa tgtgaataaa ggccggataa aacttgtgct tatttttctt tacggtcttt 1380
   aaaaaggccg taatatccag ctgaacggtc tggttatagg tacattgagc aactgactga 1440
   aatgcctcaa aatgttcttt acgatgccat tgggatatat caacggtggt atatccagtg 1500
   atttttttct ccattttagc ttccttagct cctgaaaatc tcggatccga tatctagcta 1560
   gagcgcccgg ttgacgctgc tagtgttacc tagcgatttg tatcttactg catgttactt 1620
   catgttgtca atacctgttt ttcgtgcgac ttatcaggct gtctacttat ccggagatcc 1680
   acaggacggg tgtggtcgcc atgatcgcgt agtcgatagt ggctccaagt agcgaagcga 1740
   gcaggactgg gcggcggcca aagcggtcgg acagtgctcc gagaacgggt gcgcatagaa 1800
   attgcatcaa cgcatatagc gctagcagca cgccatagtg actggcgatg ctgtcggaat 1860
   ggacgatatc ccgcaagagg cccggcagta ccggcataac caagcctatg cctacagcat 1920
   ccagggtgac ggtgccgagg atgacgatga gcgcattgtt agatttcata cacggtgcct 1980
   gactgcgtta gcaatttaac tgtgataaac taccgcatta aagcttatcg atgataagct 2040
   gtcaaacatg agaattcgaa atcaaataat gattttattt tgactgatag tgacctgttc 2100
   gttgcaacaa attgataagc aatgcttttt tataatgcca acttagtata aaaaagcagg 2160
   cttcaagatc ttcacctacc aaacaatgcc cccctgcaaa aaataaattc atataaaaaa 2220
   catacagata accatctgcg gtgataaatt atctctggcg gtgttgacat aaataccact 2280
   ggcggtgata ctgagcacat cagcaggacg cactgaccac catgaaggtg acgctcttaa 2340
   aaattaagcc ctgaagaagg gcagcattca aagcagaagg ctttggggtg tgtgatacga 2400
   aacgaagcat tggccgtcta gaaggaaaca gctcagaaga aggagactgg agcatggcaa 2460
   gttcgggcac aacatcgacg cgtaagcgct ttaccggcgc agaatttatc gttcatttcc 2520
   tggaacagca gggcattaag attgtgacag gcattccggg cggttctatc ctgcctgttt 2580
   acgatgcctt aagccaaagc acgcaaatcc gccatattct ggcccgtcat gaacagggcg 2640
   cgggctttat cgctcaggga atggcgcgca ccgacggtaa accggcggtc tgtatggcct 2700
   gtagcggacc gggtgcgact aacctggtga ccgccattgc cgatgcgcgg ctggactcca 2760
   tcccgctgat ttgcatcact ggtcaggttc ccgcctcgat gatcggcacc gacgccttcc 2820
   aggaagtgga cacctacggc atctctatcc ccatcaccaa acacaactat ctggtcagac 2880
   atatcgaaga actcccgcag gtcatgagcg atgccttccg cattgcgcaa tcaggccgcc 2940
   caggcccggt gtggatagac attcctaagg atgtgcaaac ggcagttttt gagattgaaa 3000
   cacagcccgc tatggcagaa aaagccgccg cccccgcctt tagcgaagaa agcattcgtg 3060
   acgcagcggc gatgattaac gctgccaaac gcccggtgct ttatctgggc ggcggtgtga 3120
   tcaatgcgcc cgcacgggtg cgtgaactgg cggagaaagc gcaactgcct accaccatga 3180
   ctttaatggc gctgggcatg ttgccaaaag cgcatccgtt gtcgctgggt atgctgggga 3240
   tgcacggcgt gcgcagcacc aactatattt tgcaggaggc ggatttgttg atagtgctcg 3300
   gtgcgcgttt tgatgaccgg gcgattggca aaaccgagca gttctgtccg aatgccaaaa 3360
   tcattcatgt cgatatcgac cgtgcagagc tgggtaaaat caagcagccg cacgtggcga 3420
   ttcaggcgga tgttgatgac gtgctggcgc agttgatccc gctggtggaa gcgcaaccgc 3480
   gtgcagagtg gcaccagttg gtagcggatt tgcagcgtga gtttccgtgt ccaatcccga 3540
   aagcgtgcga tccgttaagc cattacggcc tgatcaacgc cgttgccgcc tgtgtcgatg 3600
   acaatgcaat tatcaccacc gacgttggtc agcatcagat gtggaccgcg caagcttatc 3660
   cgctcaatcg cccacgccag tggctgacct ccggtgggct gggcacgatg ggttttggcc 3720
   tgcctgcggc gattggcgct gcgctggcga acccggatcg caaagtgttg tgtttctccg 3780
   gcgacggcag cctgatgatg aatattcagg agatggcgac cgccagtgaa aatcagctgg 3840
   atgtcaaaat cattctgatg aacaacgaag cgctggggct ggtgcatcag caacagagtc 3900
   tgttctacga gcaaggcgtt tttgccgcca cctatccggg caaaatcaac tttatgcaga 3960
   ttgccgccgg attcggcctc gaaacctgtg atttgaataa cgaagccgat ccgcaggctt 4020
   cattgcagga aatcatcaat cgccctggcc cggcgctgat ccatgtgcgc attgatgccg 4080
   aagaaaaagt ttacccgatg gtgccgccag gtgcggcgaa tactgaaatg gtgggggaat 4140
   aagccatgca aaacacaact catgacaacg taattctgga gctcaccgtt cgcaaccatc 4200
   cgggcgtaat gacccacgtt tgtggccttt ttgcccgccg cgcttttaac gttgaaggca 4260
   ttctttgtct gccgattcag gacagcgaca aaagccatat ctggctactg gtcaatgacg 4320
   accagcgtct ggagcagatg ataagccaaa tcgataagct ggaagatgtc gtgaaagtgc 4380
   agcgtaatca gtccgatccg acgatgttta acaagatcgc ggtgtttttt cagtaacaaa 4440
   cctggttaag cctggctgaa ctgaagaaat aaaataaatc cccggcggcg tttagtcgcc 4500
   ggggttatgt gatccccgaa gatgaaactt attcaatctc ttcacagaca tcctgcgtta 4560
   aacgccgcat aatatctttt cttaacaaaa acttttgtat tttacctgag gtagttcgcg 4620
   gtagtttttc gattaccacg atatgttcag gatatttata ttttgcgacc cgtttacggc 4680
   taaaaaaagc cactacctct tccagcgata atgaatgatg cggcgctttc agcacgacat 4740
   aagcgcatga tcgttcacct aaacgttcat cggacattgc aaccacacag gcatcgtgaa 4800
   ttttaggatg ctgcaataaa atatcttcca cttcacggct gctaatattt tcgccgccgc 4860
   ggacaataat atcttttttg cgtccggtaa tttttatata gccagcctca tccatacggc 4920
   agagatcgcc gctgtaatac cagccttctt catccagggc acgggcggtt a 4971
<210> 51
   <211> 3786
   <212> DNA
   <213> DNA fragment
<400> 51
   aaatgtgctt atttaataat taatttatat atttaatgca ttaattctta acattaattg 60
   atcaataata ttcaccaaat caatatcaaa aaaaatcgca aaacatataa ttcaatacaa 120
   atcatcagga taggttttgc aacgcgtgca ttttgtcccc tttttcctcg ttgattagat 180
   gcaaaaattt atgctgaaat atgtcaaccg atgaaaagcg tcggtagtta agcagaaatt 240
   aatatcgctt actttaacca ccgcagcaca attagctaat tttacggatg cagaactcac 300
   gctggcgctc aagttagtat aaaaaagctg aacgagaaac gtaaaatgat ataaatatca 360
   atatattaaa ttagattttg cataaaaaac agactacata atactgtaaa acacaacata 420
   tgcagtcact atgaatcaac tacttagatg gtattagtga cctgtaacag actgcagtgg 480
   tcgaaaaaaa aagcccgcac tgtcaggtgc gggctttttt ctgtgttaag cttcgacgaa 540
   tttctgccat tcatccgctt attatcactt attcaggcgt agcaccaggc gtttaagggc     600
   accaataact gccttaaaaa aattacgccc cgccctgcca ctcatcgcag tactgttgta     660
   attcattaag cattctgccg acatggaagc catcacagac ggcatgatga acctgaatcg     720
   ccagcggcat cagcaccttg tcgccttgcg tataatattt gcccatggtg aaaacggggg     780
   cgaagaagtt gtccatattg gccacgttta aatcaaaact ggtgaaactc acccagggat     840
   tggctgagac gaaaaacata ttctcaataa accctttagg gaaataggcc aggttttcac     900
   cgtaacacgc cacatcttgc gaatatatgt gtagaaactg ccggaaatcg tcgtggtatt     960
   cactccagag cgatgaaaac gtttcagttt gctcatggaa aacggtgtaa caagggtgaa    1020
   cactatccca tatcaccagc tcaccgtctt tcattgccat acggaattcc ggatgagcat    1080
   tcatcaggcg ggcaagaatg tgaataaagg ccggataaaa cttgtgctta tttttcttta    1140
   cggtctttaa aaaggccgta atatccagct gaacggtctg gttataggta cattgagcaa    1200
   ctgactgaaa tgcctcaaaa tgttctttac gatgccattg ggatatatca acggtggtat    1260
   atccagtgat ttttttctcc attttagctt ccttagctcc tgaaaatctc ggatccgata    1320
   tctagctaga gcgcccggtt gacgctgcta gtgttaccta gcgatttgta tcttactgca    1380
   tgttacttca tgttgtcaat acctgttttt cgtgcgactt atcaggctgt ctacttatcc    1440
   ggagatccac aggacgggtg tggtcgccat gatcgcgtag tcgatagtgg ctccaagtag    1500
   cgaagcgagc aggactgggc ggcggccaaa gcggtcggac agtgctccga gaacgggtgc    1560
   gcatagaaat tgcatcaacg catatagcgc tagcagcacg ccatagtgac tggcgatgct    1620
   gtcggaatgg acgatatccc gcaagaggcc cggcagtacc ggcataacca agcctatgcc    1680
   tacagcatcc agggtgacgg tgccgaggat gacgatgagc gcattgttag atttcataca    1740
   cggtgcctga ctgcgttagc aatttaactg tgataaacta ccgcattaaa gcttatcgat    1800
   gataagctgt caaacatgag aattcgaaat caaataatga ttttattttg actgatagtg    1860
   acctgttcgt tgcaacaaat tgataagcaa tgctttttta taatgccaac ttagtataaa    1920
   aaagcaggct tcaagatctt cacctaccaa acaatgcccc cctgcaaaaa ataaattcat    1980
   ataaaaaaca tacagataac catctgcggt gataaattat ctctggcggt gttgacataa    2040
   ataccactgg cggtgatact gagcacatca gcaggacgca ctgaccacca tgaaggtgac    2100
   gctcttaaaa attaagccct gaagaagggc agcattcaaa gcagaaggct ttggggtgtg    2160
   tgatacgaaa cgaagcattg gccgtctaga aggaaacagc taaggaccca aaccatgagc    2220
   cagcaagtca ttattttcga taccacattg cgcgacggtg aacaggcgtt acaggcaagc    2280
   ttgagtgtga aagaaaaact gcaaattgcg ctggcccttg agcgtatggg tgttgacgtg    2340
   atggaagtcg gtttccccgt ctcttcgccg ggcgattttg aatcggtgca aaccatcgcc    2400
   cgccaggtta aaaacagccg cgtatgtgcg ttagctcgct gcgtggaaaa agatatcgac    2460
   gtggcggccg aatccctgaa agtcgccgaa gccttccgta ttcatacctt tattgccact    2520
   tcgccaatgc acatcgccac caagctgcgc agcacgctgg acgaggtgat cgaacgcgct    2580
   atctatatgg tgaaacgcgc ccgtaattac accgatgatg ttgaattttc ttgcgaagat    2640
   gccgggcgta cacccattgc cgatctggcg cgagtggtcg aagcggcgat taatgccggt    2700
   gccaccacca tcaacattcc ggacaccgtg ggctacacca tgccgtttga gttcgccgga    2760
   atcatcagcg gcctgtatga acgcgtgcct aacatcgaca aagccattat ctccgtacat    2820
   acccacgacg atttgggcct ggcggtcgga aactcactgg cggcggtaca tgccggtgca    2880
   cgccaggtgg aaggcgcaat gaacgggatc ggcgagcgtg ccggaaactg ttccctggaa    2940
   gaagtcatca tggcgatcaa agttcgtaag gatattctca acgtccacac cgccattaat 3000
   caccaggaga tatggcgcac cagccagtta gttagccaga tttgtaatat gccgatcccg 3060
   gcaaacaaag ccattgttgg cagcggcgca ttcgcacact cctccggtat acaccaggat 3120
   ggcgtgctga aaaaccgcga aaactacgaa atcatgacac cagaatctat tggtctgaac 3180
   caaatccagc tgaatctgac ctctcgttcg gggcgtgcgg cggtgaaaca tcgcatggat 3240
   gagatggggt ataaagaaag tgaatataat ttagacaatt tgtacgatgc tttcctgaag 3300
   ctggcggaca aaaaaggtca ggtgtttgat tacgatctgg aggcgctggc cttcatcggt 3360
   aagcagcaag aagagccgga gcatttccgt ctggattact tcagcgtgca gtctggctct 3420
   aacgatatcg ccaccgccgc cgtcaaactg gcctgtggcg aagaagtcaa agcagaagcc 3480
   gccaacggta acggtccggt cgatgccgtc tatcaggcaa ttaaccgcat cactgaatat 3540
   aacgtcgaac tggtgaaata cagcctgacc gccaaaggcc acggtaaaga tgcgctgggt 3600
   caggtggata tcgtcgctaa ctacaacggt cgccgcttcc acggcgtctg cctggctacc 3660
   gatattgtcg agtcatctgc caaagccatg gtgcacgttc tgaacaatat ctggcgtgcc 3720
   gcagaagtcg aaaaagagtt gcaacgcaaa gctcaacaca acgaaaacaa caaggaaacc 3780
   gtgtga 3786
<210> 52
   <211> 2942
   <212> DNA
   <213> DNA fragment
<400> 52
   gtgcctctgg cggatgtacg tttgtcatga gtctcacgct caagttagta taaaaaagct 60
   gaacgagaaa cgtaaaatga tataaatatc aatatattaa attagatttt gcataaaaaa 120
   cagactacat aatactgtaa aacacaacat atgcagtcac tatgaatcaa ctacttagat 180
   ggtattagtg acctgtaaca gactgcagtg gtcgaaaaaa aaagcccgca ctgtcaggtg 240
   cgggcttttt tctgtgttaa gcttcgacga atttctgcca ttcatccgct tattatcact 300
   tattcaggcg tagcaccagg cgtttaaggg caccaataac tgccttaaaa aaattacgcc 360
   ccgccctgcc actcatcgca gtactgttgt aattcattaa gcattctgcc gacatggaag 420
   ccatcacaga cggcatgatg aacctgaatc gccagcggca tcagcacctt gtcgccttgc 480
   gtataatatt tgcccatggt gaaaacgggg gcgaagaagt tgtccatatt ggccacgttt 540
   aaatcaaaac tggtgaaact cacccaggga ttggctgaga cgaaaaacat attctcaata 600
   aaccctttag ggaaataggc caggttttca ccgtaacacg ccacatcttg cgaatatatg 660
   tgtagaaact gccggaaatc gtcgtggtat tcactccaga gcgatgaaaa cgtttcagtt 720
   tgctcatgga aaacggtgta acaagggtga acactatccc atatcaccag ctcaccgtct 780
   ttcattgcca tacggaattc cggatgagca ttcatcaggc gggcaagaat gtgaataaag 840
   gccggataaa acttgtgctt atttttcttt acggtcttta aaaaggccgt aatatccagc 900
   tgaacggtct ggttataggt acattgagca actgactgaa atgcctcaaa atgttcttta 960
   cgatgccatt gggatatatc aacggtggta tatccagtga tttttttctc cattttagct 1020
   tccttagctc ctgaaaatct cggatccgat atctagctag agcgcccggt tgacgctgct   1080
   agtgttacct agcgatttgt atcttactgc atgttacttc atgttgtcaa tacctgtttt   1140
   tcgtgcgact tatcaggctg tctacttatc cggagatcca caggacgggt gtggtcgcca   1200
   tgatcgcgta gtcgatagtg gctccaagta gcgaagcgag caggactggg cggcggccaa   1260
   agcggtcgga cagtgctccg agaacgggtg cgcatagaaa ttgcatcaac gcatatagcg   1320
   ctagcagcac gccatagtga ctggcgatgc tgtcggaatg gacgatatcc cgcaagaggc   1380
   ccggcagtac cggcataacc aagcctatgc ctacagcatc cagggtgacg gtgccgagga   1440
   tgacgatgag cgcattgtta gatttcatac acggtgcctg actgcgttag caatttaact   1500
   gtgataaact accgcattaa agcttatcga tgataagctg tcaaacatga gaattcgaaa   1560
   tcaaataatg attttatttt gactgatagt gacctgttcg ttgcaacaaa ttgataagca   1620
   atgctttttt ataatgccaa cttagtataa aaaagcaggc ttcatagaca ttcgccttct   1680
   tccggtttat tatgttttaa ccacctgccc gtaaacctgg agaaccatcg ctctagaagg   1740
   aaacagctat gtttcaaaaa gttgacgcct acgctggcga cccgattctt acgcttatgg   1800
   agcgttttaa agaagaccct cgcagcgaca aagtgaattt aagtatcggt ctgtactaca   1860
   acgaagacgg aattattcca caactgcaag ccgtggcgga ggcggaagcg cgcctgaatg   1920
   cgcagcctca tggcgcttcg ctttatttac cgatggaagg gcttaactgc tatcgccatg   1980
   ccattgcgcc gctgctgttt ggtgcggacc atccggtact gaaacaacag cgcgtagcaa   2040
   ccattcaaac ccttggcggc tccggggcat tgaaagtggg cgcggatttc ctgaaacgct   2100
   acttcccgga atcaggcgtc tgggtcagcg atcctacctg ggaaaaccac gtagcaatat   2160
   tcgccggggc tggattcgaa gtgagtactt acccctggta tgacgaagcg actaacggcg   2220
   tgcgctttaa tgacctgttg gcgacgctga aaacattacc tgcccgcagt attgtgttgc   2280
   tgcatccatg ttgccacaac ccaacgggtg ccgatctcac taatgatcag tgggatgcgg   2340
   tgattgaaat tctcaaagcc cgcgagctta ttccattcct cgatattgcc tatcaaggat   2400
   ttggtgccgg tatggaagag gatgcctacg ctattcgcgc cattgccagc gctggattac   2460
   ccgctctggt gagcaattcg ttctcgaaaa ttttctccct ttacggcgag cgcgtcggcg   2520
   gactttctgt tatgtgtgaa gatgccgaag ccgctggccg cgtactgggg caattgaaag   2580
   caacagttcg ccgcaactac tccagcccgc cgaattttgg tgcgcaggtg gtggctgcag   2640
   tgctgaatga cgaggcattg aaagccagct ggctggcgga agtagaagag atgcgtactc   2700
   gcattctggc aatgcgtcag gaattggtga aggtattaag cacagagatg ccagaacgca   2760
   atttcgatta tctgcttaat cagcgcggca tgttcagtta taccggttta agtgccgctc   2820
   aggttgaccg actacgtgaa gaatttggtg tctatctcat cgccagcggt cgcatgtgtg   2880
   tcgccgggtt aaatacggca aatgtacaac gtgtggcaaa ggcgtttgct gcggtgatgt   2940
   aa                                                                  2942
<210> 53
   <211> 891
   <212> PRT
   <213> Shigella flexneri
<400> 53
   Met Ala Val Thr Asn Val Ala Glu Leu Asn Ala Leu Val Glu Arg Val
   1 5 10 15
   Lys Lys Ala Gln Arg Glu Tyr Ala Ser Phe Thr Gln Glu Gln Val Asp
   20 25 30
   Lys Ile Phe Arg Ala Ala Ala Leu Ala Ala Ala Asp Ala Arg Ile Pro
   35 40 45
   Leu Ala Lys Met Ala Val Ala Glu Ser Gly Met Gly Ile Val Glu Asp
   50 55 60
   Lys Val Ile Lys Asn His Phe Ala Ser Glu Tyr Ile Tyr Asn Ala Tyr
   65 70 75 80
   Lys Asp Glu Lys Thr Cys Gly Val Leu Ser Glu Asp Asp Thr Phe Gly
   85 90 95
   Thr Ile Thr Ile Ala Glu Pro Ile Gly Ile Ile Cys Gly Ile Val Pro
   100 105 110
   Thr Thr Asn Pro Thr Ser Thr Ala Ile Phe Lys Ser Leu Ile Ser Leu
   115 120 125
   Lys Thr Arg Asn Ala Ile Ile Phe Ser Pro His Pro Arg Ala Lys Asp
   130 135 140
   Ala Thr Asn Lys Ala Ala Asp Ile Val Leu Gln Ala Ala Ile Ala Ala
   145 150 155 160
   Gly Ala Pro Lys Asp Leu Ile Gly Trp Ile Asp Gln Pro Ser Val Glu
   165 170 175
   Leu Ser Asn Ala Leu Met His His Pro Asp Ile Asn Leu Ile Leu Ala
   180 185 190
   Thr Gly Gly Pro Gly Met Val Lys Ala Ala Tyr Ser Ser Gly Lys Pro
   195 200 205
   Ala Ile Gly Val Gly Ala Gly Asn Thr Pro Val Val Ile Asp Glu Thr
   210 215 220
   Ala Asp Ile Lys Arg Ala Val Ala Ser Val Leu Met Ser Lys Thr Phe
   225 230 235 240
   Asp Asn Gly Val Ile Cys Ala Ser Glu Gln Ser Val Val Val Val Asp
   245 250 255
   Ser Val Tyr Asp Ala Val Arg Glu Arg Phe Ala Thr His Gly Gly Tyr
   260 265 270
   Leu Leu Gln Gly Lys Glu Leu Lys Ala Val Gln Asp Val Ile Leu Lys
   275 280 285
   Asn Gly Ala Leu Asn Ala Ala Ile Val Gly Gln Pro Ala Tyr Lys Ile
   290 295 300
   Ala Glu Leu Ala Gly Phe Ser Val Pro Glu Asn Thr Lys Ile Leu Ile
   305                 310                 315                 320
   Gly Glu Val Thr Val Val Asp Glu Ser Glu Pro Phe Ala His Glu Lys
                   325                 330                 335
   Leu Ser Pro Thr Leu Ala Met Tyr Arg Ala Lys Asp Phe Glu Asp Ala
               340                 345                 350
   Val Glu Lys Ala Glu Lys Leu Val Ala Met Gly Gly Ile Gly His Thr
           355                 360                 365
   Ser Cys Leu Tyr Thr Asp Gln Asp Asn Gln Pro Ala Arg Val Ser Tyr
       370                 375                 380
   Phe Gly Gln Lys Met Lys Thr Ala Arg Ile Leu Ile Asn Thr Pro Ala
   385                 390                 395                 400
   Ser Gln Gly Gly Ile Gly Asp Leu Tyr Asn Phe Lys Leu Ala Pro Ser
                   405                 410                 415
   Leu Thr Leu Gly Cys Gly Ser Trp Gly Gly Asn Ser Ile Ser Glu Asn
               420                 425                 430
   Val Gly Pro Lys His Leu Ile Asn Lys Lys Thr Val Ala Lys Arg Ala
           435                 440                 445
   Glu Asn Met Leu Trp His Lys Leu Pro Lys Ser Ile Tyr Phe Arg Arg
       450                 455                 460
   Gly Ser Leu Pro Ile Ala Leu Asp Glu Val Ile Thr Asp Gly His Lys
   465                 470                 475                 480
   Arg Ala Leu Ile Val Thr Asp Arg Phe Leu Phe Asn Asn Gly Tyr Ala
                   485                 490                 495
   Asp Gln Ile Thr Ser Val Leu Lys Ala Ala Gly Val Glu Thr Glu Val
               500                 505                 510
   Phe Phe Glu Val Glu Ala Asp Pro Thr Leu Ser Ile Val Arg Lys Gly
           515                 520                 525
   Ala Glu Leu Ala Asn Ser Phe Lys Pro Asp Val Ile Ile Ala Leu Gly
       530                 535                 540
   Gly Gly Ser Pro Met Asp Ala Ala Lys Ile Met Trp Val Met Tyr Glu
   545                 550                 555                 560
   His Pro Glu Thr His Phe Glu Glu Leu Ala Leu Arg Phe Met Asp Ile
                   565                 570                 575
   Arg Lys Arg Ile Tyr Lys Phe Pro Lys Met Gly Val Lys Ala Lys Met
               580                 585                 590
   Ile Ala Val Thr Thr Thr Ser Gly Thr Gly Ser Glu Val Thr Pro Phe
           595                 600                 605
   Ala Val Val Thr Asp Asp Ala Thr Gly Gln Lys Tyr Pro Leu Ala Asp
       610                 615                 620
   Tyr Ala Leu Thr Pro Asp Met Ala Ile Val Asp Ala Asn Leu Val Met
   625 630 635 640
   Asp Met Pro Lys Ser Leu Cys Ala Phe Gly Gly Leu Asp Ala Val Thr
   645 650 655
   His Ala Met Glu Ala Tyr Val Ser Val Leu Ala Ser Glu Phe Ser Asp
   660 665 670
   Gly Gln Ala Leu Gln Ala Leu Lys Leu Leu Lys Glu Tyr Leu Pro Ala
   675 680 685
   Ser Tyr His Glu Gly Ser Lys Asn Pro Val Ala Arg Glu Arg Val His
   690 695 700
   Ser Ala Ala Thr Ile Ala Gly Ile Ala Phe Ala Asn Ala Phe Leu Gly
   705 710 715 720
   Val Cys His Ser Met Ala His Lys Leu Gly Ser Gln Phe His Ile Pro
   725 730 735
   His Gly Leu Ala Asn Ala Leu Leu Ile Cys Asn Val Ile Arg Tyr Asn
   740 745 750
   Ala Asn Asp Asn Pro Thr Lys Gln Thr Ala Phe Ser Gln Tyr Asp Arg
   755 760 765
   Pro Gln Ala Arg Arg Arg Tyr Ala Glu Ile Ala Asp His Leu Gly Leu
   770 775 780
   Ser Ala Pro Gly Asp Arg Thr Ala Ala Lys Ile Glu Lys Leu Leu Ala
   785 790 795 800
   Trp Leu Glu Thr Leu Lys Ala Glu Leu Gly Ile Pro Lys Ser Ile Arg
   805 810 815
   Glu Ala Gly Val Gln Glu Ala Asp Phe Leu Ala Asn Val Asp Lys Leu
   820 825 830
   Ser Glu Asp Ala Phe Asp Asp Gln Cys Thr Gly Ala Asn Pro Arg Tyr
   835 840 845
   Pro Leu Ile Ser Glu Leu Lys Gln Ile Leu Leu Asp Thr Tyr Tyr Gly
   850 855 860
   Arg Asp Tyr Val Glu Asp Glu Thr Ala Ala Lys Lys Glu Ala Ala Pro
   865 870 875 880
   Ala Lys Ala Glu Lys Lys Ala Lys Lys Ser Ala
   885 890
<210> 54
   <211> 891
   <212> PRT
   <213> Yersinia pestis
<400> 54
   Met Ala Val Thr Asn Val Ala Glu Leu Asn Glu Leu Val Ala Arg Val
   1               5                   10                  15
   Lys Lys Ala Gln Arg Glu Tyr Ala Asn Phe Ser Gln Glu Gln Val Asp
               20                  25                  30
   Lys Ile Phe Arg Ala Ala Ala Leu Ala Ala Ala Asp Ala Arg Ile Pro
           35                  40                  45
   Leu Ala Lys Leu Ala Val Thr Glu Ser Gly Met Gly Ile Val Glu Asp
       50                  55                  60
   Lys Val Ile Lys Asn His Phe Ala Ser Glu Tyr Ile Tyr Asn Ala Tyr
   65                  70                  75                  80
   Lys Asp Glu Lys Thr Cys Gly Ile Leu Cys Glu Asp Lys Thr Phe Gly
                   85                  90                  95
   Thr Ile Thr Ile Ala Glu Pro Ile Gly Leu Ile Cys Gly Ile Val Pro
               100                 105                 110
   Thr Thr Asn Pro Thr Ser Thr Ala Ile Phe Lys Ala Leu Ile Ser Leu
           115                 120                 125
   Lys Thr Arg Asn Gly Ile Ile Phe Ser Pro His Pro Arg Ala Lys Asp
       130                 135                 140
   Ala Thr Asn Lys Ala Ala Asp Ile Val Leu Gln Ala Ala Ile Ala Ala
   145                 150                 155                 160
   Gly Ala Pro Ala Asp Ile Ile Gly Trp Ile Asp Ala Pro Thr Val Glu
                   165                 170                 175
   Leu Ser Asn Gln Leu Met His His Pro Asp Ile Asn Leu Ile Leu Ala
               180                 185                 190
   Thr Gly Gly Pro Gly Met Val Lys Ala Ala Tyr Ser Ser Gly Lys Pro
           195                 200                 205
   Ala Ile Gly Val Gly Ala Gly Asn Thr Pro Val Val Val Asp Glu Thr
       210                 215                 220
   Ala Asp Ile Lys Arg Val Val Ala Ser Ile Leu Met Ser Lys Thr Phe
   225                 230                 235                 240
   Asp Asn Gly Val Ile Cys Ala Ser Glu Gln Ser Ile Ile Val Val Asp
                   245                 250                 255
   Ser Val Tyr Asp Ala Val Arg Glu Arg Phe Ala Ser His Gly Gly Tyr
               260                 265                 270
   Leu Leu Gln Gly Lys Glu Leu Lys Ala Val Gln Asp Ile Ile Leu Lys
           275                 280                 285
   Asn Gly Gly Leu Asn Ala Ala Ile Val Gly Gln Pro Ala Thr Lys Ile
       290                 295                 300
   Ala Glu Met Ala Gly Ile Lys Val Pro Ser Asn Thr Lys Ile Leu Ile
   305 310 315 320
   Gly Glu Val Lys Val Val Asp Glu Ser Glu Pro Phe Ala His Glu Lys
   325 330 335
   Leu Ser Pro Thr Leu Ala Met Tyr Arg Ala Lys Asn Phe Glu Glu Ala
   340 345 350
   Val Glu Lys Ala Glu Lys Leu Val Glu Met Gly Gly Ile Gly His Thr
   355 360 365
   Ser Cys Leu Tyr Thr Asp Gln Asp Asn Gln Thr Ala Arg Val Lys Tyr
   370 375 380
   Phe Gly Asp Lys Met Lys Thr Ala Arg Ile Leu Ile Asn Thr Pro Ala
   385 390 395 400
   Ser Gln Gly Gly Ile Gly Asp Leu Tyr Asn Phe Lys Leu Ala Pro Ser
   405 410 415
   Leu Thr Leu Gly Cys Gly Ser Trp Gly Gly Asn Ser Ile Ser Glu Asn
   420 425 430
   Val Gly Pro Lys His Leu Ile Asn Lys Lys Thr Val Ala Lys Arg Ala
   435 440 445
   Glu Asn Met Leu Trp His Lys Leu Pro Lys Ser Ile Tyr Phe Arg Arg
   450 455 460
   Gly Ser Leu Pro Ile Ala Leu Glu Glu Val Ala Thr Asp Gly Ala Lys
   465 470 475 480
   Arg Ala Phe Ile Val Thr Asp Arg Tyr Leu Phe Asn Asn Gly Tyr Ala
   485 490 495
   Asp Gln Val Thr Ser Val Leu Lys Ser His Gly Ile Glu Thr Glu Val
   500 505 510
   Phe Phe Glu Val Glu Ala Asp Pro Thr Leu Ser Ile Val Arg Lys Gly
   515 520 525
   Ala Glu Gln Met Asn Ser Phe Lys Pro Asp Val Ile Ile Ala Leu Gly
   530 535 540
   Gly Gly Ser Pro Met Asp Ala Ala Lys Ile Met Trp Val Met Tyr Glu
   545 550 555 560
   His Pro Glu Thr His Phe Glu Glu Leu Ala Leu Arg Phe Met Asp Ile
   565 570 575
   Arg Lys Arg Ile Tyr Lys Phe Pro Lys Met Gly Val Lys Ala Lys Leu
   580 585 590
   Val Ala Ile Thr Thr Thr Ser Gly Thr Gly Ser Glu Val Thr Pro Phe
   595 600 605
   Ala Val Val Thr Asp Asp Ala Thr Gly Gln Lys Tyr Pro Leu Ala Asp
   610 615 620
   Tyr Ala Leu Thr Pro Asp Met Ala Ile Val Asp Ala Asn Leu Val Met
   625                 630                 635                 640
   Asn Met Pro Lys Ser Leu Cys Ala Phe Gly Gly Leu Asp Ala Val Thr
                   645                 650                 655
   His Ala Leu Glu Ala Tyr Val Ser Val Leu Ala Asn Glu Tyr Ser Asp
               660                 665                 670
   Gly Gln Ala Leu Gln Ala Leu Lys Leu Leu Lys Glu Phe Leu Pro Ala
           675                 680                 685
   Ser Tyr Asn Glu Gly Ala Lys Asn Pro Val Ala Arg Glu Arg Val His
       690                 695                 700
   Asn Ala Ala Thr Ile Ala Gly Ile Ala Phe Ala Asn Ala Phe Leu Gly
   705                 710                 715                 720
   Val Cys His Ser Met Ala His Lys Leu Gly Ser Glu Phe His Ile Pro
                   725                 730                 735
   His Gly Leu Ala Asn Ala Met Leu Ile Ser Asn Val Ile Arg Tyr Asn
               740                 745                 750
   Ala Asn Asp Asn Pro Thr Lys Gln Thr Ala Phe Ser Gln Tyr Asp Arg
           755                 760                 765
   Pro Gln Ala Arg Arg Arg Tyr Ala Glu Ile Ala Asp His Leu Gly Leu
       770                 775                 780
   Ser Ala Pro Gly Asp Arg Thr Ala Gln Lys Ile Gln Lys Leu Leu Ala
   785                 790                 795                 800
   Trp Leu Asp Glu Ile Lys Ala Glu Leu Gly Ile Pro Ala Ser Ile Arg
                   805                 810                 815
   Glu Ala Gly Val Gln Glu Ala Asp Phe Leu Ala Lys Val Asp Lys Leu
               820                 825                 830
   Ser Glu Asp Ala Phe Asp Asp Gln Cys Thr Gly Ala Asn Pro Arg Tyr
           835                 840                 845
   Pro Leu Ile Ser Glu Leu Lys Gln Ile Leu Met Asp Thr Tyr Tyr Gly
       850                 855                 860
   Arg Glu Tyr Val Glu Glu Phe Asp Arg Glu Glu Glu Val Ala Ala Ala
   865                 870                 875                 880
   Thr Ala Pro Lys Ala Glu Lys Lys Thr Lys Lys
                   885                 890
<210> 55
   <211> 891
   <212> PRT
   <213> Erwinia carotovora
<400> 55
   Met Ala Val Thr Asn Val Ala Glu Leu Asn Ala Leu Val Glu Arg Val
   1 5 10 15
   Lys Lys Ala Gln Gln Glu Phe Ala Thr Tyr Thr Gln Glu Gln Val Asp
   20 25 30
   Lys Ile Phe Arg Ala Ala Ala Leu Ala Ala Ser Asp Ala Arg Ile Pro
   35 40 45
   Leu Ala Lys Met Ala Val Ala Glu Ser Gly Met Gly Ile Val Glu Asp
   50 55 60
   Lys Val Ile Lys Asn His Phe Ala Ser Glu Tyr Ile Tyr Asn Ala Tyr
   65 70 75 80
   Gln Asp Glu Lys Thr Cys Gly Val Leu Ser Thr Asp Asp Thr Phe Gly
   85 90 95
   Thr Ile Thr Ile Ala Glu Pro Ile Gly Leu Ile Cys Gly Ile Val Pro
   100 105 110
   Thr Thr Asn Pro Thr Ser Thr Ala Ile Phe Lys Ala Leu Ile Ser Leu
   115 120 125
   Lys Thr Arg Asn Gly Ile Ile Phe Ser Pro His Pro Arg Ala Lys Asn
   130 135 140
   Ala Thr Asn Lys Ala Ala Asp Ile Val Leu Gln Ala Ala Ile Ala Ala
   145 150 155 160
   Gly Ala Pro Lys Asp Ile Ile Gly Trp Ile Asp Gln Pro Ser Val Asp
   165 170 175
   Leu Ser Asn Gln Leu Met His His Pro Asp Ile Asn Leu Ile Leu Ala
   180 185 190
   Thr Gly Gly Pro Gly Met Val Lys Ala Ala Tyr Ser Ser Gly Lys Pro
   195 200 205
   Ala Ile Gly Val Gly Ala Gly Asn Thr Pro Val Val Ile Asp Glu Thr
   210 215 220
   Ala Asp Ile Lys Arg Ala Val Ala Ser Ile Leu Met Ser Lys Thr Phe
   225 230 235 240
   Asp Asn Gly Val Ile Cys Ala Ser Glu Gln Ser Val Ile Val Val Asp
   245 250 255
   Ser Ala Tyr Asp Ala Val Arg Glu Arg Phe Ala Thr His Gly Gly Tyr
   260 265 270
   Met Leu Lys Gly Lys Glu Leu His Ala Val Gln Gly Ile Leu Leu Lys
   275 280 285
   Asn Gly Ser Leu Asn Ala Asp Ile Val Gly Gln Pro Ala Pro Lys Ile
   290 295 300
   Ala Glu Met Ala Gly Ile Thr Val Pro Ala Asn Thr Lys Val Leu Ile
   305                 310                 315                 320
   Gly Glu Val Thr Ala Val Asp Glu Ser Glu Pro Phe Ala His Glu Lys
                   325                 330                 335
   Leu Ser Pro Thr Leu Ala Met Tyr Arg Ala Lys Asp Phe Asn Asp Ala
               340                 345                 350
   Val Ile Lys Ala Glu Lys Leu Val Ala Met Gly Gly Ile Gly His Thr
           355                 360                 365
   Ser Cys Leu Tyr Thr Asp Gln Asp Asn Gln Pro Glu Arg Val Asn His
       370                 375                 380
   Phe Gly Asn Met Met Lys Thr Ala Arg Ile Leu Ile Asn Thr Pro Ala
   385                 390                 395                 400
   Ser Gln Gly Gly Ile Gly Asp Leu Tyr Asn Phe Lys Leu Ala Pro Ser
                   405                 410                 415
   Leu Thr Leu Gly Cys Gly Ser Trp Gly Gly Asn Ser Ile Ser Glu Asn
               420                 425                 430
   Val Gly Pro Lys His Leu Ile Asn Lys Lys Thr Val Ala Lys Arg Ala
           435                 440                 445
   Glu Asn Met Leu Trp His Lys Leu Pro Lys Ser Ile Tyr Phe Arg Arg
       450                 455                 460
   Gly Ser Leu Pro Ile Ala Leu Glu Glu Val Ala Ser Asp Gly Ala Lys
   465                 470                 475                 480
   Arg Ala Phe Ile Val Thr Asp Arg Phe Leu Phe Asn Asn Gly Tyr Val
                   485                 490                 495
   Asp Gln Val Thr Ser Val Leu Lys Gln His Gly Leu Glu Thr Glu Val
               500                 505                 510
   Phe Phe Glu Val Glu Ala Asp Pro Thr Leu Ser Ile Val Arg Lys Gly
           515                 520                 525
   Ala Glu Gln Met His Ser Phe Lys Pro Asp Val Ile Ile Ala Leu Gly
       530                 535                 540
   Gly Gly Ser Pro Met Asp Ala Ala Lys Ile Met Trp Val Met Tyr Glu
   545                 550                 555                 560
   His Pro Thr Thr His Phe Glu Glu Leu Ala Leu Arg Phe Met Asp Ile
                   565                 570                 575
   Arg Lys Arg Ile Tyr Lys Phe Pro Lys Met Gly Val Lys Ala Lys Met
               580                 585                 590
   Val Ala Ile Thr Thr Thr Ser Gly Thr Gly Ser Glu Val Thr Pro Phe
           595                 600                 605
   Ala Val Val Thr Asp Asp Ala Thr Gly Gln Lys Tyr Pro Leu Ala Asp
       610                 615                 620
   Tyr Ala Leu Thr Pro Asp Met Ala Ile Val Asp Ala Asn Leu Val Met
   625                 630                 635                 640
   Asn Met Pro Lys Ser Leu Cys Ala Phe Gly Gly Leu Asp Ala Val Thr
                   645                 650                 655
   His Ser Leu Glu Ala Tyr Val Ser Val Leu Ala Asn Glu Tyr Ser Asp
               660                 665                 670
   Gly Gln Ala Leu Gln Ala Leu Lys Leu Leu Lys Glu Asn Leu Pro Asp
           675                 680                 685
   Ser Tyr Arg Asp Gly Ala Lys Asn Pro Val Ala Arg Glu Arg Val His
       690                 695                 700
   Asn Ala Ala Thr Ile Ala Gly Ile Ala Phe Ala Asn Ala Phe Leu Gly
   705                 710                 715                 720
   Val Cys His Ser Met Ala His Lys Leu Gly Ser Glu Phe His Ile Pro
                   725                 730                 735
   His Gly Leu Ala Asn Ala Met Leu Ile Ser Asn Val Ile Arg Tyr Asn
               740                 745                 750
   Ala Asn Asp Asn Pro Thr Lys Gln Thr Thr Phe Ser Gln Tyr Asp Arg
           755                 760                 765
   Pro Gln Ala Arg Arg Arg Tyr Ala Glu Ile Ala Asp His Leu Arg Leu
       770                 775                 780
   Thr Ala Pro Ser Asp Arg Thr Ala Gln Lys Ile Glu Lys Leu Leu Asn
   785                 790                 795                 800
   Trp Leu Glu Glu Ile Lys Thr Glu Leu Gly Ile Pro Ala Ser Ile Arg
                   805                 810                 815
   Glu Ala Gly Val Gln Glu Ala Asp Phe Leu Ala Lys Val Asp Lys Leu
               820                 825                 830
   Ser Glu Asp Ala Phe Asp Asp Gln Cys Thr Gly Ala Asn Pro Arg Tyr
           835                 840                 845
   Pro Leu Ile Ser Glu Leu Lys Gln Ile Leu Leu Asp Thr Tyr Tyr Gly
       850                 855                 860
   Arg Lys Phe Ser Glu Glu Val Lys Thr Glu Thr Val Glu Pro Val Ala
   865                 870                 875                 880
   Lys Ala Ala Lys Thr Gly Lys Lys Ala Ala His
                   885                 890
<210> 56
   <211> 878
   <212> PRT
   <213> Salmonella typhimurium
<400> 56
   Met Ala Val Thr Asn Val Ala Glu Leu Asn Ala Leu Val Glu Arg Val
   1               5                   10                  15
   Lys Lys Ala Gln Arg Glu Tyr Ala Ser Phe Thr Gln Glu Gln Val Asp
               20                  25                  30
   Lys Ile Phe Arg Ala Ala Ala Leu Ala Ala Ala Asp Ala Arg Ile Pro
           35                  40                  45
   Leu Ala Lys Met Ala Val Ala Glu Ser Gly Met Gly Ile Val Glu Asp
       50                  55                  60
   Lys Val Ile Lys Asn His Phe Ala Ser Glu Tyr Ile Tyr Asn Ala Tyr
   65                  70                  75                  80
   Lys Asp Glu Lys Thr Cys Gly Val Leu Ser Glu Asp Asp Thr Phe Arg
                   85                  90                  95
   Thr Ile Thr Ile Ala Glu Pro Ile Gly Ile Ile Cys Gly Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Ile Phe Ser Pro His Pro Arg Ala Lys Glu
       130                 135                 140
   Ala Thr Asn Lys Ala Ala Asp Ile Val Ser Lys Ala Leu Ser Leu Pro
   145                 150                 155                 160
   Ala Arg Arg Lys Ile Arg Leu Ala Arg Ser Ile Asn Leu Pro Val Glu
                   165                 170                 175
   Leu Ser Asn Val Asp Ala Pro Pro Gly Tyr Ile Pro Asp Pro Ala Thr
               180                 185                 190
   Gly Ser Arg His Gly Tyr Ser Cys Ile Gln Leu Gly Ser Thr Leu Ser
           195                 200                 205
   Ala Ile Gly Gln Ala Thr Leu Arg Leu Val Leu Met Lys Pro Leu Ile
       210                 215                 220
   Asp Ser Asn Ala Ala Cys Gly Leu Cys Leu Asp Ala Tyr Asn Leu Cys
   225                 230                 235                 240
   Tyr Arg Gly Asn Leu Cys Phe Cys Thr Ser Leu Phe Val Val Asp Ser
                   245                 250                 255
   Ala Tyr Leu His Met Arg Pro Asp Phe Arg Gln His Gly Gly Tyr Met
               260                 265                 270
   Arg Arg Pro Glu Leu Lys Ala Val Gln Arg Tyr Pro Glu Lys Trp Arg
           275                 280                 285
   Ser Glu Arg Ala Ile Val Gly Gln Pro Ala Tyr Lys Ile Ala Glu Leu
       290                 295                 300
   Ala Gly Phe Ser Val Pro Glu Thr Thr Lys Ile Leu Ile Gly Glu Val
   305 310 315 320
   Thr Val Val Asp Glu Ser Glu Pro Phe Ala His Glu Lys Leu Ser Pro
   325 330 335
   Thr Leu Ala Met Tyr Arg Ala Lys Asp Phe Glu Glu Ala Val Glu Lys
   340 345 350
   Ala Glu Lys Leu Val Ala Met Gly Gly Ile Gly His Thr Ser Cys Leu
   355 360 365
   Tyr Thr Asp Gln Asp Asn Gln Pro Glu Arg Val Ala Tyr Phe Gly Gln
   370 375 380
   Met Lys Asn Ala Arg Ile Leu Ile Asn Thr Pro Ala Ser Gln Gly Gly
   385 390 395 400
   Ile Gly Asp Leu Tyr Asn Phe Lys Leu Pro Pro Ser Leu Thr Leu Gly
   405 410 415
   Cys Gly Ser Trp Gly Gly Asn Ser Ile Ser Glu Asn Val Gly Pro Lys
   420 425 430
   His Leu Ile Tyr Lys Lys Thr Val Ala Lys Arg Ala Glu Asn Met Trp
   435 440 445
   His Lys Leu Pro Lys Ser Ile Tyr Phe Arg Arg Gly Ser Leu Pro Ile
   450 455 460
   Ala Leu Asp Glu Val Ile Thr Asp Gly His Lys Arg Ala Leu Ile Val
   465 470 475 480
   Thr Asp Arg Phe Cys Ser Thr Thr Val Ser Asp Arg Ser Leu Cys Ala
   485 490 495
   Glu Arg Arg Gly Val Glu Thr Glu Val Phe Phe Glu Val Glu Ala Ala
   500 505 510
   Asp Pro Thr Leu Ser Val Val Arg Lys Gly Pro Glu Leu Ala Asn Ser
   515 520 525
   Phe Lys Pro Asp Val Ile Ile Ala Val Gly Gly Val Pro Arg Trp Thr
   530 535 540
   Arg Gly Glu Ile Ile Gly Ser Cys Thr Asn His Pro Glu Thr Arg Leu
   545 550 555 560
   Ile Glu Glu Arg Val Arg Phe Met Thr Ser Tyr Arg Ile Tyr Lys Phe
   565 570 575
   Pro Lys Met Val Lys Ala Lys Cys Ser Pro Ser Thr Thr Thr Ser Gly
   580 585 590
   Thr Gly Ser Lys Leu His Arg Leu Arg Leu Cys Pro Thr Thr Leu Leu
   595 600 605
   Pro Gln Lys Tyr Pro Leu Ala Asp Tyr Ala Val Thr Pro Asp Met Ala
   610 615 620
   Ile Val Asp Ala Asn Leu Val Met Asp Met Pro Asn Thr Leu Thr Arg
   625 630 635 640
   Lys Gly Pro Leu His Arg Leu Thr His Ala Met Glu Arg Ile Val Ser
   645 650 655
   Val Leu Ala Ser Gln Ser Asp Gly Gln Ala Leu Gln Ala Leu Lys Glu
   660 665 670
   Tyr Phe Pro Ala Ser Tyr His Glu Gly Lys Asn Pro Val Ala Arg Glu
   675 680 685
   Arg Leu His Ser Ala Ala Thr Ile Ala Pro Ile Ala Phe Ala Asn Ala
   690 695 700
   Phe Leu Gly Val Cys His Trp Met Ala His Lys Leu Pro Ala Gln Leu
   705 710 715 720
   His Ile Pro His Gly Pro Phe Asn Ala Arg Tyr Arg His Ser Val Arg
   725 730 735
   Arg Ala Gln Ser Asn Pro Thr Lys Gln Val Ala Leu Ser Gln Tyr Leu
   740 745 750
   Tyr Asn Phe Ala Ala His Arg Trp Pro Ala Glu Arg Ser Ile Pro Arg
   755 760 765
   Ala Arg Thr Gly Ile Arg Pro Arg Lys Tyr Lys Leu Val Pro Val Ala
   770 775 780
   Leu Cys His Val Lys Gly Ile Lys Ala Asp Leu Gly Ile Pro Lys Ser
   785 790 795 800
   Ile Arg Glu Ala Gly Val Gln Glu Ala Asp Phe Leu Ala His Val Asp
   805 810 815
   Lys Leu Ser Glu Asp Ala Phe Asp Asp Gln Cys Thr Gly Ala Asn Pro
   820 825 830
   Arg Tyr Pro Leu Met Ser Glu Leu Lys Gln Ile Leu Leu Asp Thr Tyr
   835 840 845
   Tyr Gly Arg Asp Phe Thr Glu Gly Glu Val Ala Ala Lys Lys Asp Val
   850 855 860
   Val Ala Ala Pro Lys Ala Glu Lys Lys Ala Lys Lys Ser Ala
   865 870 875
<210> 57
   <211> 867
   <212> PRT
   <213> Lactobacillus plantarum
<400> 57
   Met Ile Lys Thr Glu Lys Asn Gln Thr Ser Lys Val Thr Asp Glu Val
   1               5                   10                  15
   Asp Gln Leu Val Gln Arg Ser Lys Lys Ala Leu Ala Ile Leu Lys Ser
               20                  25                  30
   Tyr Thr Gln Ala Gln Ile Asp Asp Leu Cys Glu Lys Val Ala Val Ala
           35                  40                  45
   Ala Leu Asp Asn His Met Lys Leu Ala Lys Leu Ala Val Glu Glu Thr
       50                  55                  60
   Gly Arg Gly Val Val Glu Asp Lys Ala Ile Lys Asn Ile Tyr Ala Ser
   65                  70                  75                  80
   Glu Tyr Ile Trp Asn Ser Met Arg His Asp Lys Thr Val Gly Val Ile
                   85                  90                  95
   Lys Glu Asp Asp Glu Glu Gln Leu Met Glu Ile Ala Glu Pro Val Gly
               100                 105                 110
   Ile Val Ala Gly Val Thr Pro Val Thr Asn Pro Thr Ser Thr Thr Val
           115                 120                 125
   Phe Lys Thr Leu Ile Ser Leu Lys Gly Arg Asn Thr Ile Val Phe Gly
       130                 135                 140
   Phe His Pro Gln Ala Gln Lys Cys Ser Ser Ala Ala Ala Asp Val Met
   145                 150                 155                 160
   Arg Glu Ala Ile Lys Ala Ala Gly Gly Pro Ala Asp Ala Val Leu Tyr
                   165                 170                 175
   Ile Glu His Pro Ser Ile Glu Ala Thr Asp Ala Leu Met His His Thr
               180                 185                 190
   Asp Val Ala Thr Ile Leu Ala Thr Gly Gly Pro Ser Met Val Thr Ala
           195                 200                 205
   Ala Tyr Ser Ser Gly Lys Pro Ala Leu Gly Val Gly Pro Gly Asn Gly
       210                 215                 220
   Pro Thr Tyr Val Glu Lys Thr Ala Asp Ile Lys Gln Ala Val Asn Asp
   225                 230                 235                 240
   Ile Val Leu Ser Lys Thr Phe Asp Asn Gly Met Ile Cys Ala Ser Glu
                   245                 250                 255
   Asn Ser Ala Ile Ile Asp Lys Glu Ile Tyr Ala Glu Val Lys Ala Glu
               260                 265                 270
   Phe Ile Arg Leu Gly Cys Tyr Tyr Val Lys Pro Lys Asp Val Gln Ala
           275                 280                 285
   Leu Ser Asp Ala Val Ile Asp Pro Asn Arg His Thr Val Arg Gly Pro
       290                 295                 300
   Val Ala Gly Lys Thr Ala Tyr Gln Ile Ala Gln Met Ala Gly Leu Lys
   305                 310                 315                 320
   Asp Val Ala Lys Asp Cys Arg Val Leu Ile Ala Glu Ile Asn Gly Val
   325 330 335
   Gly Ile Lys Tyr Pro Leu Ser Gly Glu Lys Leu Ser Pro Val Leu Thr
   340 345 350
   Val Tyr Lys Ala Asp Ser His Glu Ala Ala Phe Lys Arg Ala Asn Glu
   355 360 365
   Leu Leu His Tyr Gly Gly Leu Gly His Thr Ala Gly Ile His Thr Thr
   370 375 380
   Asp Asp Ala Leu Val Lys Glu Phe Gly Leu Gln Met Pro Ala Cys Arg
   385 390 395 400
   Ile Leu Val Asn Thr Pro Ser Ser Val Gly Gly Leu Gly Asn Ile Tyr
   405 410 415
   Asn Asn Met Ala Pro Ser Leu Thr Leu Gly Thr Gly Ser Tyr Gly Gly
   420 425 430
   Asn Ser Ile Ser His Asn Val Thr Asp Met Asp Leu Ile Asn Ile Lys
   435 440 445
   Thr Val Ala Lys Arg Arg Asn Asn Met Gln Trp Val Lys Met Pro Pro
   450 455 460
   Lys Val Tyr Phe Glu Arg Asn Ser Val Arg Tyr Leu Glu His Met Ala
   465 470 475 480
   Gly Ile Lys Lys Val Phe Leu Val Cys Asp Pro Gly Met Val Glu Phe
   485 490 495
   Gly Tyr Ala Asp Arg Val Thr Ala Val Leu Asn Lys Arg Thr Asp Pro
   500 505 510
   Val Asp Ile Asp Ile Phe Ser Glu Val Glu Pro Asn Pro Ser Thr Asp
   515 520 525
   Thr Val Tyr Lys Gly Val Ala Arg Met Lys Ala Phe Lys Pro Asp Thr
   530 535 540
   Ile Ile Ala Leu Gly Gly Gly Ser Ala Met Asp Ala Ala Lys Gly Met
   545 550 555 560
   Trp Leu Phe Tyr Glu His Pro Glu Ala Ser Phe Leu Gly Ala Lys Gln
   565 570 575
   Lys Phe Leu Asp Ile Arg Lys Arg Thr Tyr Lys Val Pro Val Ser Glu
   580 585 590
   Lys Val Thr Tyr Ile Gly Ile Pro Thr Thr Ser Gly Thr Gly Ser Glu
   595 600 605
   Val Thr Pro Tyr Ala Val Ile Thr Asp Ser Lys Thr His Val Lys Tyr
   610 615 620
   Pro Ile Thr Asp Tyr Ala Met Gln Pro Asp Ile Ala Ile Val Asp Pro
   625 630 635 640
   Gln Phe Val Glu Thr Val Pro Lys Arg Thr Thr Ala Trp Thr Gly Leu
   645 650 655
   Asp Val Ile Thr His Ala Thr Glu Ala Tyr Val Ser Thr Met Ala Ser
   660 665 670
   Asp Phe Thr Arg Gly Trp Ser Ile Gln Ala Leu Gln Leu Ala Phe Lys
   675 680 685
   Tyr Leu Lys Ala Ser Tyr Asp Gly Asp Lys Met Ala Arg Glu Lys Met
   690 695 700
   His Asn Ala Ser Thr Leu Ala Gly Met Ala Phe Ala Asn Ala Phe Leu
   705 710 715 720
   Gly Ile Asn His Ser Ile Ala His Lys Leu Gly Gly Glu Phe Asn Leu
   725 730 735
   Pro His Gly Leu Ala Ile Ala Ile Thr Tyr Pro Gln Val Val Arg Tyr
   740 745 750
   Asn Ala Glu Ile Pro Thr Lys Leu Ala Met Trp Pro Lys Tyr Asn His
   755 760 765
   Asn Thr Ala Leu Ala Asp Tyr Ala Asn Ile Ala Arg Ala Leu Gly Leu
   770 775 780
   Pro Gly Lys Thr Asp Glu Glu Leu Lys Glu Ser Leu Val Lys Ala Tyr
   785 790 795 800
   Ile Asp Leu Ala His Ser Met Asp Val Thr Leu Ser Leu Lys Ala Asn
   805 810 815
   Arg Val Glu Lys Lys His Phe Asp Ala Thr Val Asp Glu Leu Ala Glu
   820 825 830
   Leu Ala Tyr Glu Asp Gln Cys Thr Thr Ala Asn Pro Arg Glu Pro Leu
   835 840 845
   Ile Ser Glu Leu Lys Ala Ile Ile Glu Arg Glu Trp Asp Gly Gln Gly
   850 855 860
   Thr Glu Lys
   865
<210> 58
   <211> 903
   <212> PRT
   <213> Lactococcus lactis
<400> 58
   Met Ala Thr Lys Lys Ala Ala Pro Ala Ala Lys Lys Val Leu Ser Ala
   1 5 10 15
   Glu Glu Lys Ala Ala Lys Phe Gln Glu Val Val Ala Tyr Thr Asp Gln
   20 25 30
   Leu Val Lys Lys Ala Gln Ala Ala Val Leu Lys Phe Glu Gly Tyr Thr
   35 40 45
   Gln Thr Gln Val Asp Thr Ile Val Ala Ala Met Ala Leu Ala Ala Ser
   50 55 60
   Lys His Ser Leu Glu Leu Ala His Glu Ala Val Asn Glu Thr Gly Arg
   65 70 75 80
   Gly Val Val Glu Asp Lys Asp Thr Lys Asn His Phe Ala Ser Glu Ser
   85 90 95
   Val Tyr Asn Ala Ile Lys Asn Asp Lys Thr Val Gly Val Ile Ala Glu
   100 105 110
   Asn Lys Val Ala Gly Ser Val Glu Ile Ala Ser Pro Leu Gly Val Leu
   115 120 125
   Ala Gly Ile Val Pro Thr Thr Asn Pro Thr Ser Thr Ala Ile Phe Lys
   130 135 140
   Ser Leu Leu Thr Ala Lys Thr Arg Asn Ala Ile Val Phe Ala Phe His
   145 150 155 160
   Pro Gln Ala Gln Lys Cys Ser Ser His Ala Ala Lys Ile Val Tyr Asp
   165 170 175
   Ala Ala Ile Glu Ala Gly Ala Pro Glu Asp Phe Ile Gln Trp Ile Glu
   180 185 190
   Val Pro Ser Leu Asp Met Thr Thr Ala Leu Ile Gln Asn Arg Gly Ile
   195 200 205
   Ala Thr Ile Leu Ala Thr Gly Gly Pro Gly Met Val Asn Ala Ala Leu
   210 215 220
   Lys Ser Gly Asn Pro Ser Leu Gly Val Gly Ala Gly Asn Gly Ala Val
   225 230 235 240
   Tyr Val Asp Ala Thr Ala Asn Ile Asp Arg Ala Val Glu Asp Leu Leu
   245 250 255
   Leu Ser Lys Arg Phe Asp Asn Gly Met Ile Cys Ala Thr Glu Asn Ser
   260 265 270
   Ala Val Ile Asp Ala Ser Ile Tyr Asp Glu Phe Val Ala Lys Met Pro
   275 280 285
   Thr Gln Gly Ala Tyr Met Val Pro Lys Lys Asp Tyr Lys Ala Ile Glu
   290 295 300
   Ser Phe Val Phe Val Glu Arg Ala Gly Glu Gly Phe Gly Val Thr Gly
   305 310 315 320
   Pro Val Ala Gly Arg Ser Gly Gln Trp Ile Ala Glu Gln Ala Gly Val
   325 330 335
   Asn Val Pro Lys Asp Lys Asp Val Leu Leu Phe Glu Leu Asp Lys Lys
   340 345 350
   Asn Ile Gly Glu Ala Leu Ser Ser Glu Lys Leu Ser Pro Leu Leu Ser
   355 360 365
   Ile Tyr Lys Ser Glu Thr Arg Glu Glu Gly Ile Glu Ile Val Arg Ser
   370 375 380
   Leu Leu Ala Tyr Gln Gly Ala Gly His Asn Ala Ala Ile Gln Ile Gly
   385 390 395 400
   Ala Met Asp Asp Pro Phe Val Lys Glu Tyr Gly Ile Lys Val Glu Ala
   405 410 415
   Ser Arg Ile Leu Val Asn Gln Pro Asp Ser Ile Gly Gly Val Gly Asp
   420 425 430
   Ile Tyr Thr Asp Ala Met Arg Pro Ser Leu Thr Leu Gly Thr Gly Ser
   435 440 445
   Trp Gly Lys Asn Ser Leu Ser His Asn Leu Ser Thr Tyr Asp Leu Leu
   450 455 460
   Asn Val Lys Thr Val Ala Lys Arg Arg Asn Arg Pro Gln Trp Val Arg
   465 470 475 480
   Leu Pro Lys Glu Ile Tyr Tyr Glu Lys Asn Ala Ile Ser Tyr Leu Gln
   485 490 495
   Glu Leu Pro His Val His Lys Ala Phe Ile Val Ala Asp Pro Gly Met
   500 505 510
   Val Lys Phe Gly Phe Val Asp Lys Val Leu Glu Gln Leu Ala Ile Arg
   515 520 525
   Pro Thr Gln Val Glu Thr Ser Ile Tyr Gly Ser Val Gln Pro Asp Pro
   530 535 540
   Thr Leu Ser Glu Ala Ile Ala Ile Ala Arg Gln Met Asn His Phe Glu
   545 550 555 560
   Pro Asp Thr Val Ile Cys Leu Gly Gly Gly Ser Ala Leu Asp Ala Gly
   565 570 575
   Lys Ile Gly Arg Leu Ile Tyr Glu Tyr Asp Ala Arg Gly Glu Ala Asp
   580 585 590
   Leu Ser Asp Asp Ala Ser Leu Lys Glu Ile Phe Gln Glu Leu Ala Gln
   595 600 605
   Lys Phe Val Asp Ile Arg Lys Arg Ile Ile Lys Phe Tyr His Pro His
   610 615 620
   Lys Ala Gln Met Val Ala Ile Pro Thr Thr Ser Gly Thr Gly Ser Glu
   625 630 635 640
   Val Thr Pro Phe Ala Val Ile Thr Asp Asp Glu Thr His Val Lys Tyr
   645 650 655
   Pro Leu Ala Asp Tyr Gln Leu Thr Pro Gln Val Ala Ile Val Asp Pro
           660                 665                 670
   Glu Phe Val Met Thr Val Pro Lys Arg Thr Val Ser Trp Ser Gly Ile
               675                 680                 685
   Asp Ala Met Ser His Ala Leu Glu Ser Tyr Val Ser Val Met Ser Ser
       690                 695                 700
   Asp Tyr Thr Lys Pro Ile Ser Leu Gln Ala Ile Lys Leu Ile Phe Glu
   705                 710                 715                 720
   Asn Leu Thr Glu Ser Tyr His Tyr Asp Pro Ala His Pro Thr Lys Glu
                   725                 730                 735
   Gly Gln Lys Ala Arg Glu Asn Met His Asn Ala Ala Thr Leu Ala Gly
               740                 745                 750
   Met Ala Phe Ala Asn Ala Phe Leu Gly Ile Asn His Ser Leu Ala His
           755                 760                 765
   Lys Ile Ala Gly Glu Phe Gly Leu Pro His Gly Leu Ala Ile Ala Ile
       770                 775                 780
   Ala Met Pro His Val Ile Lys Phe Asn Ala Val Thr Gly Asn Val Lys
   785                 790                 795                 800
   Phe Thr Pro Tyr Pro Arg Tyr Glu Thr Tyr Arg Ala Gln Glu Asp Tyr
                   805                 810                 815
   Ala Glu Ile Ser Arg Phe Met Gly Phe Ala Gly Lys Glu Asp Ser Asp
               820                 825                 830
   Glu Lys Ala Val Lys Ala Leu Val Ala Glu Leu Lys Lys Leu Thr Asp
           835                 840                 845
   Ser Ile Asp Ile Asn Ile Thr Leu Ser Gly Asn Gly Val Asp Lys Ala
       850                 855                 860
   His Leu Glu Arg Glu Leu Asp Lys Leu Ala Asp Leu Val Tyr Asp Asp
   865                 870                 875                 880
   Gln Cys Thr Pro Ala Asn Pro Arg Gln Pro Arg Ile Asp Glu Ile Lys
                   885                 890                 895
   Gln Leu Leu Leu Asp Gln Tyr
               900

## Description

### Technical field

The present invention relates to the microbiological industry, and specifically to a method for producing an L-amino acid such as L-threonine, L-lysine, L-histidine, L-phenylalanine, L-arginine, L-tryptophan, L-glutamic acid and L-leucine by fermentation using a bacterium with an enhanced activity of alcohol dehydrogenase.

### Background art

Conventionally, L-amino acids are industrially produced by fermentation methods utilizing strains of microorganisms obtained from natural sources, or mutants thereof. Typically, the microorganisms are modified to enhance production yields of L-amino acids.

Many techniques to enhance L-amino acid production yields have been reported, including transformation of microorganisms with recombinant DNA (U.S. Patent No. 4,278,765). Other techniques for enhancing production yields include increasing the activities of enzymes involved in amino acid biosynthesis and/or desensitizing the target enzymes to feedback inhibition by the resulting L-amino acid (U.S. Patent Nos. 4,346,170, 5,661,012, and 6,040,160).

By optimizing the main biosynthetic pathway of a desired compound, further improvement of L-amino acid producing strains can be accomplished. Typically, this is accomplished via supplementation of the bacterium with increasing amounts of a carbon source such as sugars, for example, glucose. Despite the efficiency of glucose transport by PTS, access to the carbon source in a highly productive strain still may be insufficient. Another way to increase productivity of L-amino acid producing strains and decrease the cost of the target L-amino acid is to use an alternative source of carbon, such as alcohol, for example, ethanol.

Alcohol dehydrogenase (ethanol oxidoreductase, AdhE) of *Escherichia coli* is a multifunctional enzyme that catalyzes fermentative production of ethanol by two sequential NADH-dependent reductions of acetyl-CoA, as well as deactivation of pyruvate formate-lyase, which cleaves pyruvate to acetyl-CoA and formate.

AdhE is abundantly synthesized (about 3x10⁴ copies per cell) during anaerobic growth in the presence of glucose and forms helical structures, called spirosomes, which are around 0.22 µm long and contain 40-60 AdhE molecules (Kessler, D., Herth, W., and Knappe, J., J. Biol. Chem., 267, 18073-18079 (1992)). When the *E. coli* cell culture is shifted from anaerobic to aerobic conditions, transcription of the *adhE* gene is reduced and maintained within 10% of the range found under anaerobiosis (Chen, Y. M., and Lin, E. C. C., J. Bacteriol. 173, 8009-8013 (1991); Leonardo, M. R., Cunningham, P. R., and Clark, D. P., J. Bacteriol. 175, 870-878 (1993); Mikulskis, A., Aristarkhov, A., and Lin, E. C. C., J. Bacteriol. 179, 7129-7134 (1997); Membrillo-Hernandez, J., and Lin, E. C. C., J. Bacteriol. 181, 7571-7579 (1999)). Translation is also regulated and requires RNase III (Membrillo-Hernandez, J., and Lin, E. C. C., J. Bacteriol. 181, 7571-7579 (1999); Aristarkhov, A. et al, J. Bacteriol. 178,4327-4332 (1996)). AdhE has been identified as one of the major targets when E. *coli* cells are subjected to hydrogen peroxide stress (Tamarit, J., Cabiscol, E., and Ros, J., J. Biol. Chem. 273, 3027-3032 (1998)).

Despite the reversibility of the two NADH-coupled reactions catalyzed by AdhE, wild-type *E. coli* is unable to grow in the presence of ethanol as the sole source of carbon and energy, because the *adhE* gene is transcribed aerobically at lowered levels (Chen, Y. M. and Lin, E. C. C., J. Bacteriol. 73, 8009-8013 (1991); Leonardo, M. R., Cunningham, P. R. & Clark, D. P., J. Bacteriol. 175 870-878 (1993)) and the half-life of the AdhE protein is shortened during aerobic metabolism by metal-catalyzed oxidation (MCO).

Mutants of *E. coli* capable of aerobic growth on ethanol as the sole carbon and energy source have been isolated and characterized (mutants with the substitution Ala267Thr grew in the presence of ethanol with a doubling time of 240 min; with the substitutions Ala267Thr and Glu568Lys, a doubling time of 90 min at 37°C) (Membrillo-Hernandez, J. et al, J. Biol. Chem. 275, 33869-33875 (2000); Holland-Staley, C. A. et al, J. Bacteriol. 182, 6049-6054 (2000)). Apparently, when the two sequential reactions are catalyzed in a direction opposite to that of the physiological one, acetyl-CoA formation is rate-limiting for wild-type AdhE. The tradeoff for improving the Vₘₐₓ by the A267T substitution in AdhE is decreased thermal enzyme stability and increased sensitivity to MCO damage. The second amino acid substitution, E568K, in AdhE (A267T/E568K) partially restored protein stability and resistance to MCO damage without further improvement of catalytic efficiency in substrate oxidation.

However, there have been no reports to date of using a bacterium of the *Enterobacteriaceae* family which has an enhanced activity of either native alcohol dehydrogenase or mutant alcohol dehydrogenase resistant to aerobic inactivation for increasing the production of L-amino acids by fermentation in a culture medium containing ethanol.

### SUMMARY OF THE INVENTION

Objects of the present invention include enhancing the productivity of L-amino acid-producing strains and providing a method for producing non-aromatic or aromatic L-amino acids using these strains.

This aim was achieved by finding that expressing either the native or mutant *adhE* gene which encodes alcohol dehydrogenase under the control of a promoter which functions under an aerobic cultivation condition enhances production of L-amino acids, for example, L-threonine, L-lysine, L-histidine, L-phenylalanine, L-arginine, L-tryptophan, L-glutamic acid, and/or L-leucine.

It is an object of the present invention to provide a method for producing an L-amino acid comprising:
A) cultivating in a culture medium containing ethanol an L-amino acid-producing bacterium of the *Enterobacteriaceae* family having an alcohol dehydrogenase, and
B) isolating the L-amino acid from the culture medium,
wherein the gene encoding said alcohol dehydrogenase is expressed under the control of a non-native promoter which functions under aerobic cultivation conditions.

It is a further object of the present invention to provide the method described above, wherein said non-native promoter is selected from the group consisting of P_{tac}, P_{lac}, Pₜᵣₚ, P_{trc}, P_{R}, and P_{L}.

It is a further object of the present invention to provide the method described above, wherein said alcohol dehydrogenase is resistant to aerobic inactivation.

It is a further object of the present invention to provide the method described above, wherein said alcohol dehydrogenase originates from a bacterium selected from the group consisting of *Escherichia coli, Erwinia carotovora, Salmonella typhimurium, Shigella flexneri, Yersinia pestis, Pantoea ananatis, Lactobacillus plantarum,* and *Lactococcus lactis.*

It is a further object of the present invention to provide the method described above, wherein said alcohol dehydrogenase comprises the amino acid sequence set forth in SEQ ID NO: 2, except the glutamic acid residue at position 568 is replaced with another amino acid residue other than an aspartic acid residue.

It is a further object of the present invention to provide the method described above, wherein said alcohol dehydrogenase comprises the amino acid sequence set forth in SEQ ID NO: 2, except the glutamic acid residue at position 568 is replaced with a lysine residue.

It is a further object of the present invention to provide the method described above, wherein said alcohol dehydrogenase has at least one additional mutation which is able to improve the growth of said bacterium in a liquid medium which contains ethanol as the sole carbon source.

It is a further object of the present invention to provide the method described above, wherein said additional mutation is selected from the group consisting of:
A) replacement of the glutamic acid residue at position 560 in SEQ ID NO: 2 with another amino acid residue;
B) replacement of the phenylalanine residue at position 566 in SEQ ID NO: 2 with another amino acid residue;
C) replacement of the glutamic acid residue, the methionine residue, the tyrosine residue, the isoleucine residue, and the alanine residue at positions 22, 236, 461, 554, and 786, respectively, in SEQ ID NO: 2 with other amino acid residues; and
D) combinations thereof.

It is a further object of the present invention to provide the method described above, wherein said additional mutation is selected from the group consisting of:
A) replacement of the glutamic acid residue at position 560 in SEQ ID NO: 2 with a lysine residue;
B) replacement of the phenylalanine residue at position 566 in SEQ ID NO: 2 with a valine residue;
C) replacement of the glutamic acid residue, the methionine residue, the tyrosine residue, the isoleucine residue, and the alanine residue at positions 22, 236, 461, 554, and 786 in SEQ ID NO: 2 with a glycine residue, a valine residue, a cysteine residue, a serine residue, and a valine residue, respectively; and
D) combinations thereof.

It is a further obj ect of the present invention to provide the method described above, wherein said bacterium belongs to the genus selected from the group consisting of *Escherichia, Enterobacter, Erwinia, Klebsiella, Pantoea, Providencia, Salmonella, Serratia, Shigella,* and *Morganella.*

It is a further object of the present invention to provide the method described above, wherein said L-amino acid is selected from a group consisting of L-threonine, L-lysine, L-histidine, L-phenylalanine, L-arginine, L-tryptophan, L-glutamic acid, and L-leucine.

### DETAILED DESCRIPTION OF THE PREFERRED EMBODIMENTS

Alcohol dehydrogenase is a Fe²⁺-dependent multifunctional protein with an acetaldehyde-CoA dehydrogenase activity at the N-terminal, an iron-dependent alcohol dehydrogenase activity at the C-terminal, and a pyruvate-formate lyase deactivase activity. Synonyms include B1241, AdhC, and Ana. Under aerobic conditions, the half-life of the active AdhE protein is shortened during aerobic metabolism by metal-catalyzed oxidation.

In the present invention, the phrase "activity of alcohol dehydrogenase" means an activity of catalyzing the reaction ofNAD-dependant oxidation of alcohols into aldehydes or ketones. Alcohol dehydrogenase (EC 1.1.1.1) works well with ethanol, n-propanol, and n-butanol. Activity of alcohol dehydrogenase can be detected and measured by, for example, the method described by Membrillo-Hernandez, J. et al (J. Biol. Chem. 275, 33869-33875 (2000)).

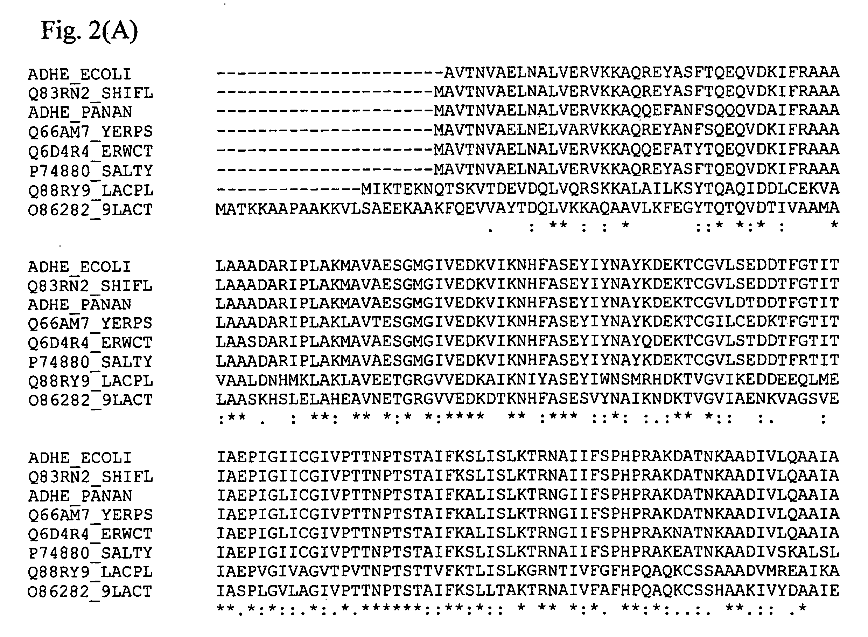

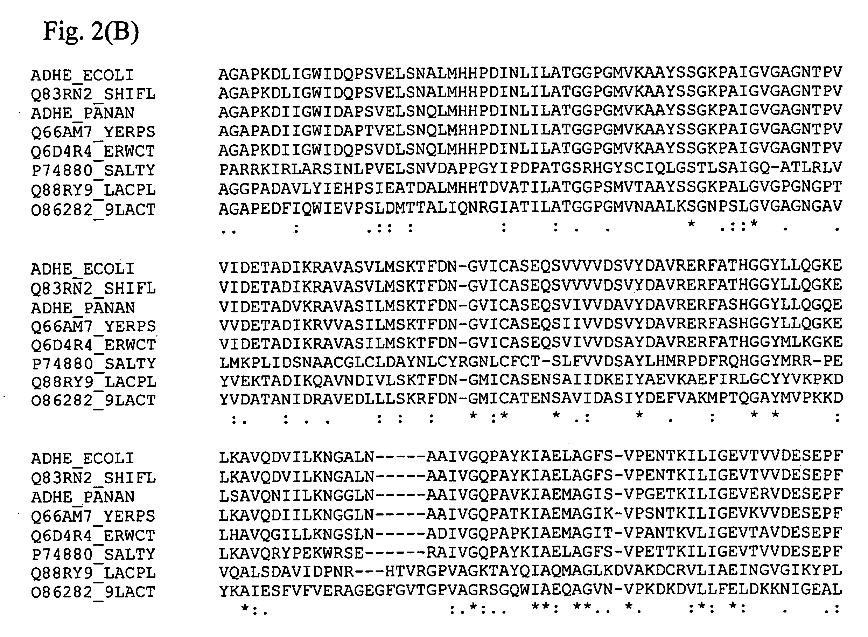

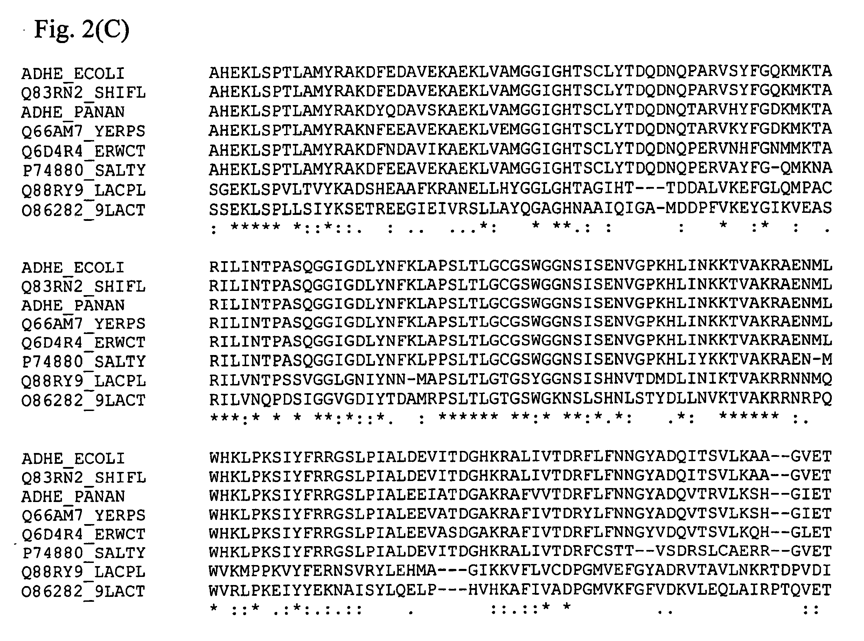

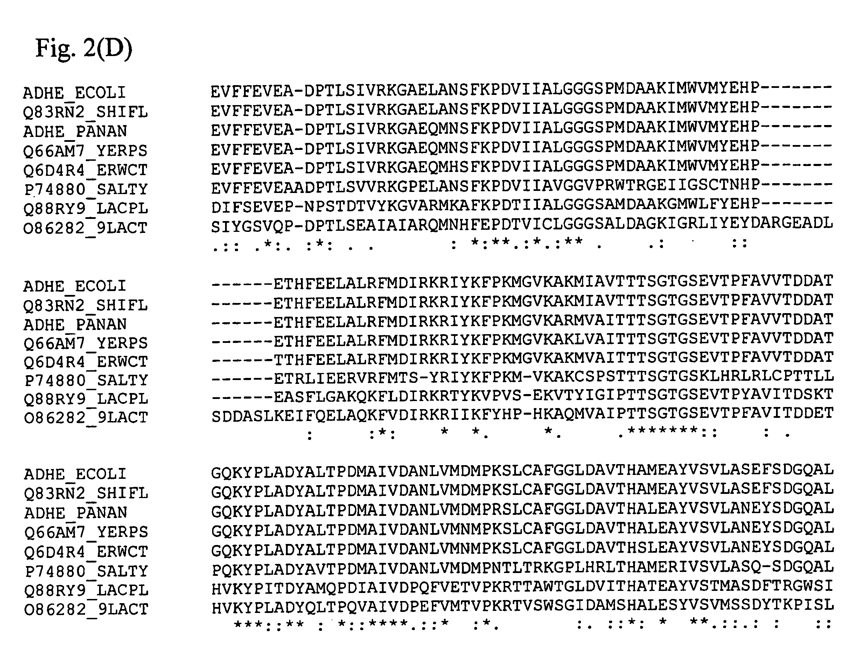

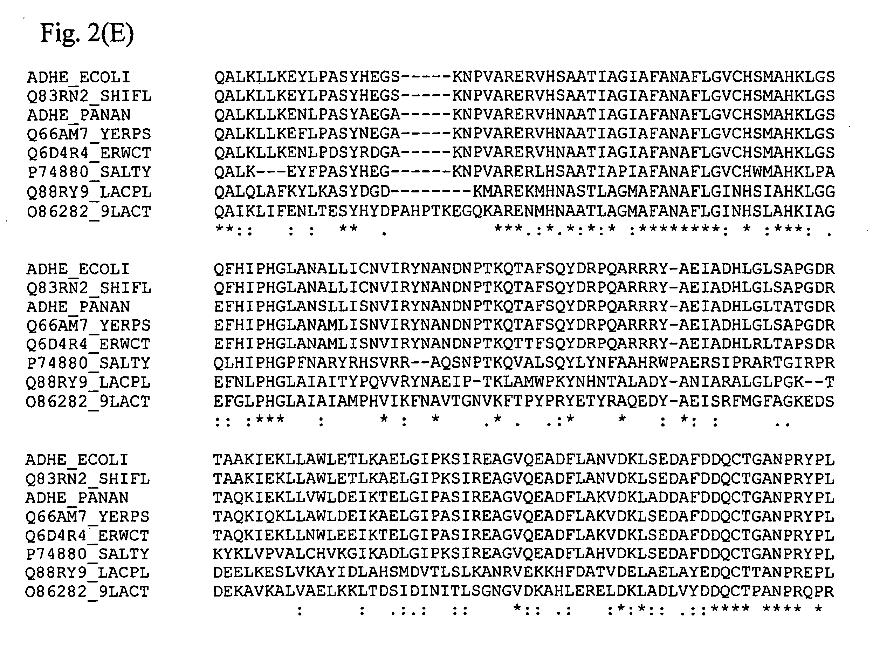

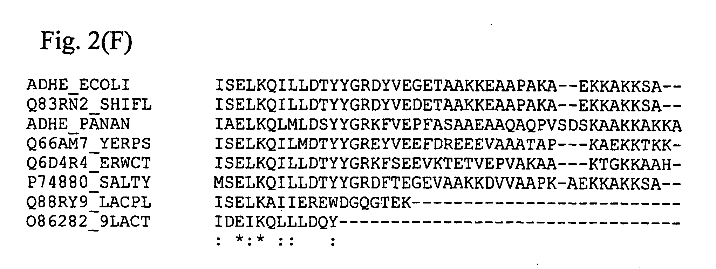

Alcohol dehydrogenase is encoded by the *adhE* gene, and any *adhE* gene derived from or native to bacteria belonging to the genus *Escherichia, Erwinia, Klebsiella, Salmonella, Shigella, Yershinia, Pantoea, Lactobacillus,* and *Lactococcus* may be used as the alcohol dehydrogenase gene in the present invention. Specific examples of the source of the *adhE* gene include bacterial strains such as *Escherichia coli, Erwinia carotovora, Salmonella enterica, Salmonella typhimurium, Shigella flexneri, Yersinia pseudotuberculosis, Pantoea ananatis, Lactobacillus plantarum* and *Lactococcus lactis.* The wild-type *adhE* gene which encodes alcohol dehydrogenase from *Escherichia coli* has been elucidated (nucleotide numbers complementary to numbers 1294669 to 1297344 in the sequence of GenBank accession NC_000913.2, gi: 49175990). The *adhE* gene is located between the *ychG and ychE* ORFs on the chromosome of *E. coli* K-12. Other *adhE* genes which encode alcohol dehydrogenases have also been elucidated: *adhE* gene from *Erwinia carotovora* (nucleotide numbers 2634501 to 2637176 in the sequence of GenBank accession NC_004547.2; gi: 50121254); *adhE* gene from *Salmonella enterica* (nucleotide numbers 1718612 to 1721290 in the sequence of GenBank accession NC_004631.1; gi: 29142095); *adhE* gene from *Salmonella typhimurium* (nucleotide numbers 1 to 2637 in the sequence of GenBank accession U68173.1; gi: 1519723); *adhE* gene from *Shigella flexneri* (nucleotide numbers complement to numbers 1290816 to 1293491in the sequence of GenBank accession NC_004741.1, gi: 30062760); *adhE* gene from *Yersinia pseudotuberculosis* (nucleotide numbers complement to numbers 2478099 to 2480774 in the sequence of GenBank accession NC_006155.1; gi: 51596429), *adhE* gene from *Pantoea ananatis* (SEQ ID NO: 29), *adhE* gene from *Lactobaccillus plantarum* (UniProtKB Entry: Q88RY9_LACPL), *adhE* gene from *Lactococcus lactis* MG1363 (EMBL accession no. AJ001007), and the like (See Figure 2). The nucleotide sequence of the *adhE* gene from *Escherichia coli* is represented by SEQ ID NO: 1. The amino acid sequence encoded by this *adhE* gene is represented by SEQ ID NO: 2.

Therefore, the *adhE* gene can be obtained by PCR (polymerase chain reaction; refer to White, T.J. et al., Trends Genet., 5, 185 (1989)) utilizing primers prepared based on the known nucleotide sequence of the gene from the *E. coli* chromosome. Genes coding for alcohol dehydrogenase from other microorganisms can be obtained in a similar manner.

The *adhE* gene derived from *Escherichia coli* is exemplified by a DNA which encodes the following protein (A) or (B):
(A) a protein which has the amino acid sequence shown in SEQ ID NO: 2; or
(B) a variant protein of the amino acid sequence shown in SEQ ID NO: 2, which has an activity of alcohol dehydrogenase.

The *adhE* gene derived from *Pantoea ananatis* is exemplified by a DNA which encodes the following protein (A) or (B):
(A) a protein which has the amino acid sequence shown in SEQ ID NO: 30; or
(B) a variant protein of the amino acid sequence shown in SEQ ID NO: 30, which has an activity of alcohol dehydrogenase.

The *adhE* gene derived from *Shigella flexneri* is exemplified by a DNA which encodes the following protein (A) or (B):
(A) a protein which has the amino acid sequence shown in SEQ ID NO: 53; or
(B) a variant protein of the amino acid sequence shown in SEQ ID NO: 53, which has an activity of alcohol dehydrogenase.

The *adhE* gene derived from *Yersinia pestis* is exemplified by a DNA which encodes the following protein (A) or (B):
(A) a protein which has the amino acid sequence shown in SEQ ID NO: 54; or
(B) a variant protein of the amino acid sequence shown in SEQ ID NO: 54, which has an activity of alcohol dehydrogenase.

The *adhE* gene derived from *Erwinia carotovora* is exemplified by a DNA which encodes the following protein (A) or (B):
(A) a protein which has the amino acid sequence shown in SEQ ID NO: 55; or
(B) a variant protein of the amino acid sequence shown in SEQ ID NO: 55, which has an activity of alcohol dehydrogenase.

The *adhE* gene derived from *Salmonella typhimurium* is exemplified by a DNA which encodes the following protein (A) or (B):
(A) a protein which has the amino acid sequence shown in SEQ ID NO: 56; or
(B) a variant protein of the amino acid sequence shown in SEQ ID NO: 56, which has an activity of alcohol dehydrogenase.

The *adhE* gene derived from *Lactobacillus plantarum* is exemplified by a DNA which encodes the following protein (A) or (B):
(A) a protein which has the amino acid sequence shown in SEQ ID NO: 57; or
(B) a variant protein of the amino acid sequence shown in SEQ ID NO: 57, which has an activity of alcohol dehydrogenase.

The *adhE* gene derived from *Lactococcus lactis* is exemplified by a DNA which encodes the following protein (A) or (B):
(A) a protein which has the amino acid sequence shown in SEQ ID NO: 58; or
(B) a variant protein of the amino acid sequence shown in SEQ ID NO: 58, which has an activity of alcohol dehydrogenase.

The phrase "variant protein" as used in the present invention means a protein which has changes in the sequence, whether they are deletions, insertions, additions, or substitutions of amino acids, but still maintains alcohol dehydrogenase activity at a useful level. The number of changes in the variant protein depends on the position in the three dimensional structure of the protein or the type of amino acid residue. The number of changes may be 1 to 30, preferably 1 to 15, and more preferably 1 to 5, relative to the protein (A). These changes in the variants are conservative mutations that preserve the function of the protein. In other words, these changes can occur in regions of the protein which are not critical for the function of the protein. This is because some amino acids have high homology to one another so the three dimensional structure or activity is not affected by such a change. Therefore, the protein variant (B) may be one which has an identity of not less than 70 %, preferably not less than 80 %, and more preferably not less than 90 %, and most preferably not less than 95 % with respect to the entire amino acid sequence of alcohol dehydrogenase shown in SEQ ID NO. 2, as long as the activity of the alcohol dehydrogenase is maintained.

Homology between two amino acid sequences can be determined using the well-known methods, for example, the computer program BLAST 2.0, which calculates three parameters: score, identity, and similarity.

The substitution, deletion, insertion, or addition of one or several amino acid residues should be conservative mutation(s) so that the activity is maintained. The representative conservative mutation is a conservative substitution. Examples of conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val.

Data comparing the primary sequences of alcohol dehydrogenase from *Escherichia coli, Shigella flexneri, Pantoea ananatis, Yersinia pestis, Erwinia carotovora, Salmonella typhimurium* (Gram negative bacteria), and *Lactobacillus plantarum, Lactococcus lactis* (Gram positive bacteria) show a high level of homology among these proteins (see Figure 2). From this point of view, substitutions or deletions of the amino acid residues which are identical (marked by asterisk) in all the above-mentioned proteins could be crucial for their function. It is possible to replace similar (marked by colon) amino acids residues by the similar amino acid residues without deterioration of the protein activity. But modifications of other non-conserved amino acid residues may not lead to alteration of the activity of alcohol dehydrogenase.

The DNA which encodes substantially the same protein as the alcohol dehydrogenase described above may be obtained, for example, by modifying the nucleotide sequence of DNA encoding alcohol dehydrogenase (SEQ ID NO: 1), for example, by means of site-directed mutagenesis so that the nucleotide sequence responsible for one or more amino acid residues at a specified site is deleted, substituted, inserted, or added. DNA modified as described above may be obtained by conventionally known mutation treatments. Such treatments include hydroxylamine treatment of the DNA encoding proteins of present invention, or treatment of the bacterium containing the DNA with UV irradiation or a reagent such as N-methyl-N'-nitro-N-nitrosoguanidine or nitrous acid.

A DNA encoding substantially the same protein as alcohol dehydrogenase can be obtained by expressing DNA having a mutation as described above in an appropriate cell, and investigating the activity of any expressed product. A DNA encoding substantially the same protein as alcohol dehydrogenase can also be obtained by isolating a DNA that is able to hybridize with a probe having a nucleotide sequence which contains, for example, the nucleotide sequence shown as SEQ ID NO: 1, under stringent conditions, and encodes a protein having alcohol dehydrogenase activity. The "stringent conditions" referred to herein are conditions under which so-called specific hybrids are formed, and non-specific hybrids are not formed. For example, stringent conditions can be exemplified by conditions under which DNAs having high homology, for example, DNAs having identity of not less than 50%, preferably not less than 60%, more preferably not less than 70%, still more preferably not less than 80%, further preferably not less than 90%, most preferably not less than 95%, are able to hybridize with each other, but DNAs having identity lower than the above are not able to hybridize with each other. Alternatively, stringent conditions may be exemplified by conditions under which DNA is able to hybridize at a salt concentration equivalent to ordinary washing conditions in Southern hybridization, i.e., 1 x SSC, 0.1% SDS, preferably 0.1 x SSC, 0.1% SDS, at 60°C. Duration of washing depends on the type of membrane used for blotting and, as a rule, what is recommended by the manufacturer. For example, recommended duration of washing for the Hybond™ N+ nylon membrane (Amersham) under stringent conditions is 15 minutes. Preferably, washing may be performed 2 to 3 times.

A partial sequence of the nucleotide sequence of SEQ ID NO: 1 can also be used as a probe. Probes may be prepared by PCR using primers based on the nucleotide sequence of SEQ ID NO: 1, and a DNA fragment containing the nucleotide sequence of SEQ ID NO: 1 as a template. When a DNA fragment having a length of about 300 bp is used as the probe, the hybridization conditions for washing include, for example, 50°C, 2 x SSC and 0.1% SDS.

The substitution, deletion, insertion, or addition of nucleotides as described above also includes mutations which naturally occur (mutant or variant), for example, due to variety in the species or genus of bacterium, and which contains the alcohol dehydrogenase.

A wild-type alcohol dehydrogenase may be subject to metal catalyzed oxidatation. Although such a wild-type alcohol dehydrogenase can be used, a mutant alcohol dehydrogenase which is resistant to aerobic inactivation is preferable in the present invention. The phrase "mutant alcohol dehydrogenase which is resistant to aerobic inactivation" means that the mutant alcohol dehydrogenase maintains its activity under aerobic conditions, or the activity is reduced by a negligible amount compared to the wild-type alcohol dehydrogenase.

In case of the *adhE* gene of *E*. *coli,* the wild-type alcohol dehydrogenase comprises the amino acid sequence set forth in SEQ ID NO: 2. An example of a mutation in alcohol dehydrogenase of SEQ ID NO: 2 which results in the protein being resistant to aerobic inactivation is replacement of the glutamic acid residue at position 568 with a lysine residue. However, introduction of a mutation into the *adhE* gene, for example at position 568 in SEQ ID NO: 2, may lead to delay of growth in a liquid medium containing ethanol as a carbon source, and in such a case, it is preferable that the mutant alcohol dehydrogenase have at least one additional mutation which is able to improve the growth of the bacterium in a liquid medium which contains ethanol as the sole carbon source. For example, the growth of *E*. *coli* is improved when the glutamic acid residue at position 568 in the alcohol dehydrogenase of SEQ ID NO: 2 is replaced by another amino acid residue by introducing an additional mutation selected from the group consisting of:
A) replacement of the glutamic acid residue at position 560 in SEQ ID NO: 2 with another amino acid residue, e.g., a lysine residue;
B) replacement of the phenylalanine residue at position 566 in SEQ ID NO: 2 with another amino acid residue, e.g., a valine residue;
C) replacement of the glutamic acid residue, the methionine residue, the tyrosine residue, the isoleucine residue, and the alanine residue at positions 22, 236, 461, 554, and 786, respectively, in SEQ ID NO: 2 with other amino acid residues, e.g., a glycine residue, a valine residue, a cysteine residue, a serine residue, and a valine residue, respectively; and
D) combinations thereof.

The reference to position numbers in a sequence, for example, the phrase "amino acid residues at positions 22, 236, 554, 560, 566, 568 and 786" refers to positions of these residues in the amino acid sequence of the wild-type AdhE from *E. coli.* However, the position of an amino acid residue may change. For example, if an amino acid residue is inserted at the N-terminus portion, the amino acid residue inherently located at position 22 becomes position 23. In such a case, the amino acid residue at original position 22 is the amino acid residue at position 22 in the present invention.

The mutant AdhE may include deletion, substitution, insertion, or addition of one or several amino acids at one or a plurality of positions other than positions identified in A) to C) above, provided that the AdhE activity is not lost or reduced.

The mutant AdhE and mutant *adhE* gene according to the present invention can be obtained from the wild-type *adhE* gene, for example, by site-specific mutagenesis using ordinary methods, such as PCR (polymerase chain reaction; refer to White, T.J. et al., Trends Genet., 5, 185 (1989)) utilizing primers prepared based on the nucleotide sequence of the gene.

Transcription of the *adhE* gene in wild-type *E. coli* is induced only under anaerobic conditions, largely in response to elevated levels of reduced NADH (Leonardo, M. R., Cunningham, P. R. & Clark, D. P., J. Bacteriol. 175 870-878 (1993)).

In the present invention, a bacterial strain used for producing an L-amino acid is modified so that expression of the *adhE* gene is controlled by a non-native promoter, i.e., a promoter that does not control the expression of the *adhE* gene in a wild-type strain. Such modification can be achieved by replacing the native promoter of the *adhE* gene on the chromosome with a non-native promoter which functions under an aerobic cultivation condition so that the *adhE* gene is operably linked with the non-native promoter. As a non-native promoter which functions under aerobic cultivation conditions, any promoter which can express the *adhE* gene above a certain level under aerobic cultivation conditions may be used. With reference to the level of the AdhE protein in the present invention, the activity of alcohol dehydrogenase in the cell free extract measured according to the method by Clark and Cronan (J. Bacteriol. 141 177-183 (1980)) should be 1.5 units or more, preferably 5 units or more, and more preferably 10 units or more, per mg of protein. Aerobic cultivation conditions can be those usually used for cultivation of bacteria in which oxygen is supplied by methods such as shaking, aeration and agitation. Specifically, any promoter which is known to express a gene under aerobic cultivation conditions can be used. For example, promoters of the genes involved in glycosis, the pentose phosphate pathway, TCA cycle, amino acid biosynthetic pathways, etc. can be used. In addition, the P_{tac} promoter, the *lac* promoter, the *trp* promoter, the *trc* promoter, the P_{R}, or the P_{L} promoters of lambda phage are all known to be strong promoters which function under aerobic cultivation conditions, and are preferably used.

The use of a non-native promoter can be combined with the multiplication of gene copies. For example, inserting the *adhE* gene operably linked with a non-native promoter into a vector that is able to function in a bacterium of the *Enterobacteriaceae* family and introducing the vector into the bacterium increases the copy number of the gene in a cell. Preferably, low-copy vectors are used. Examples of low-copy vectors include, but are not limited to, pSC101, pMW 118, pMW119, and the like. The term "low copy vector" is used for vectors, the copy number of which is up to 5 copies per cell. Increasing the copy number of the *adhE* gene can also be achieved by introducing multiple copies of the gene into the chromosomal DNA of the bacterium by, for example, homologous recombination, Mu integration, and the like. Homologous recombination is carried out using a sequence which is present in multiple copies as targets on the chromosomal DNA. Sequences having multiple copies on the chromosomal DNA include, but are not limited to, repetitive DNA, or inverted repeats existing at the end of a transposable element. Also, as disclosed in U.S. Patent No. 5,595,889, it is possible to incorporate the *adhE* gene into a transposon, and allow it to be transferred to introduce multiple copies of the gene into the chromosomal DNA. In these instances, the *adhE* gene can be placed under the control of a promoter which functions under aerobic cultivation conditions. Alternatively, the effect of a promoter can be enhanced by, for example, introducing a mutation into the promoter to increase the transcription level of a gene located downstream of the promoter. Furthermore, it is known that the substitution of several nucleotides in the spacer between the ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the *rhtA23* mutation is an A-for-G substitution at the -1 position relative to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances *rhtA* gene expression and, as a consequence, increases resistance to threonine, homoserine, and some other substances transported out of cells.

Moreover, it is also possible to introduce a nucleotide substitution into a promoter region of the *adhE* gene on the bacterial chromosome, which results in stronger promoter function. The alteration of the expression control sequence can be performed, for example, in the same manner as the gene substitution using a temperature-sensitive plasmid, as disclosed in International Patent Publication WO 00/18935 and Japanese Patent Application Laid-Open No. 1-215280.

In the present invention, "L-amino acid-producing bacterium" means a bacterium which has an ability to produce and secrete an L-amino acid into a medium, when the bacterium is cultured in the medium. The L-amino acid-producing ability may be imparted or enhanced by breeding. The term "L-amino acid-producing bacterium" as used herein also means a bacterium which is able to produce and cause accumulation of an L-amino acid in a culture medium in an amount larger than a wild-type or parental strain of the bacterium, for example, *E. coli,* such as *E. coli* K-12, and preferably means that the bacterium is able to cause accumulation in a medium of an amount not less than 0.5 g/L, more preferably not less than 1.0 g/L of the target L-amino acid. The term "L-amino acid" includes L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. L-threonine, L-lysine, L-histidine, L-phenylalanine, L-arginine, L-tryptophan, L-glutamic acid, and L-leucine are particularly preferred.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella, Morganella, Yersinia,* etc.. Specifically, those classified into the *Enterobacteriaceae* family according to the taxonomy used by the NCBI (National Center for Biotechnology Information) database (http://www.ncbi.nlm.nih.gov/TaxonomyBrowser/wwwtax.cgi?id=91347) can be used. A bacterium belonging to the genus *Escherichia* or *Pantoea* is preferred. The phrase "a bacterium belonging to the genus *Escherichia"* means that the bacterium is classified into the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited, however, for example, bacteria described by Neidhardt, F.C. et al. (Escherichia coli and Salmonella typhimurium, American Society for Microbiology, Washington D.C., 1208, Table 1) are encompassed by the present invention.

The bacterium belonging to the genus *Pantoea* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii,* or the like, based on the nucleotide sequence analysis of 16S rRNA etc. (Int. J. Syst. Bacteriol., 43, 162-173 (1993)).

The bacterium of the present invention encompasses a strain of the *Enterobacteriaceae* family which has an ability to produce an L-amino acid and has been modified so that the gene encoding an alcohol dehydrogenase is expressed under the control of a promoter which functions under aerobic cultivation conditions. In addition, the bacterium of the present invention encompasses a strain of the *Enterobacteriaceae* family which has an ability to produce an L-amino acid and does not have a native activity of alcohol dehydrogenase, but has been transformed with a DNA fragment encoding alcohol dehydrogenase.

In the present invention, the amount of accumulated L-amino acid, for example, L-threonine, L-lysine, L-histidine, L-phenylalanine, L-arginine, L-tryptophan, L-glutamic acid, or L-leucine, can be significantly increased in a culture medium containing ethanol as a carbon source as a result of expressing the gene encoding an alcohol dehydrogenase under the control of a promoter which functions under aerobic cultivation conditions.

### L-amino acid-producing bacteria

As a bacterium of the present invention which is modified to have mutant alcohol dehydrogenase of the present invention, bacteria which are able to produce either an aromatic or a non-aromatic L-amino acids may be used.

The bacterium of the present invention can be obtained by introducing the gene encoding the mutant alcoholdehydrogenase of the present invention in a bacterium which inherently has the ability to produce L-amino acids. Alternatively, the bacterium of present invention can be obtained by imparting the ability to produce L-amino acids to a bacterium already having the mutant alcohol dehydrogenase.

### L-threonine-producing bacteria

Examples of parent strains which can be used to derive the L-threonine-producing bacteria of the present invention include, but are not limited to, strains belonging to the genus *Escherichia,* such as *E. coli* TDH-6/pVIC40 (VKPM B-3996) (U.S. Patent No. 5, 175, 107, U.S. Patent No. 5,705,371), *E. coli* 472T23/pYN7 (ATCC 98081) (U.S. Patent No.5,631,157), *E. coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E. coli* MG442 (Gusyatiner et al., Genetika (in Russian), 14, 947-956 (1978)), *E. coli* VL643 and VL2055 (EP 1149911 A), and the like.

The strain TDH-6 is deficient in the *thrC* gene, as well as being sucrose-assimilative, and the *ilvA* gene in this strain has a leaky mutation. This strain also has a mutation in the *rhtA* gene, which imparts resistance to high concentrations of threonine or homoserine. The strain B-3996 contains the plasmid pVIC40 which was obtained by inserting a *thrA*BC* operon which includes a mutant *thrA* gene into a RSF1010-derived vector. This mutant *thrA* gene encodes aspartokinase homoserine dehydrogenase I which has substantially desensitized feedback inhibition by threonine. The strain B-3996 was deposited on November 19, 1987 in the All-Union Scientific Center of Antibiotics (Russia, 117105 Moscow, Nagatinskaya Street, 3-A) under the accession number RIA 1867. The strain was also deposited in the Russian National Collection of Industrial Microorganisms (VKPM) (Russia, 117545 Moscow, 1 Dorozhny proezd, 1) on April 7, 1987 under the accession number VKPM B-3996.

*E. coli* VKPM B-5318 (EP 0593792B) also may be used as a parent strain to derive L-threonine-producing bacteria of the present invention. The strain B-5318 is prototrophic with regard to isoleucine, and a temperature-sensitive lambda-phage C 1 repressor and P_{R} promoter replaces the regulatory region of the threonine operon in the plasmid pVIC40 harbored by the strain. The strain VKPM B-5318 was deposited in the Russian National Collection of Industrial Microorganisms (VKPM) on May 3, 1990 under accession number of VKPM B-5318.

Preferably, the bacterium of the present invention is additionally modified to enhance expression of one or more of the following genes:
- the mutant thrA gene which codes for aspartokinase-homoserine dehydrogenase I resistant to feed back inhibition by threonine;
- the thrB gene which codes for homoserine kinase;
- the thrC gene which codes for threonine synthase;
- the *rhtA* gene which codes for a putative transmembrane protein;
- the asd gene which codes for aspartate-β-semialdehyde dehydrogenase; and
- the *aspC* gene which codes for aspartate aminotransferase (aspartate transaminase);

The *thrA* gene which encodes aspartokinase-homoserine dehydrogenase I of *Escherichia coli* has been elucidated (nucleotide positions 337 to 2799, GenBank accession no.NC_000913.2, gi: 49175990). The *thrA* gene is located between the *thrL* and *thrB* genes on the chromosome of *E*. *coli* K-12. The *thrB* gene which encodes homoserine kinase of *Escherichia coli* has been elucidated (nucleotide positions 2801 to 3733, GenBank accession NC_000913.2, gi: 49175990). The *thrB* gene is located between the *thrA* and *thrC* genes on the chromosome *of E. coli* K-12. The *thrC* gene which encodes threonine synthase of *Escherichia coli* has been elucidated (nucleotide positions 3734 to 5020, GenBank accession NC_000913.2, gi: 49175990). The *thrC* gene is located between the *thrB* gene and the *yaaX* open reading frame on the chromosome of *E*. *coli* K-12. All three genes function as a single threonine operon. To enhance expression of the threonine operon, the attenuator region which affects the transcription is desirably removed from the operon (WO2005/049808, WO2003/097839).

A mutant *thrA* gene which codes for aspartokinase homoserine dehydrogenase I resistant to feedback inhibition by threonine, as well as the *thrB* and *thrC* genes can be obtained as one operon from the well-known plasmid pVIC40, which is present in the threonine producing *E. coli* strain VKPM B-3996. Plasmid pVIC40 is described in detail in U.S. Patent No. 5,705,371.

The *rhtA* gene is located at 18 min on the *E. coli* chromosome close to the *glnHPQ* operon, which encodes components of the glutamine transport system. The *rhtA* gene is identical to ORF1 (*ybiF* gene, nucleotide positions 764 to 1651, GenBank accession number AAA218541, gi:440181), and is located between the *pexB* and *ompX* genes. The DNA sequence expressing a protein encoded by the ORF1 has been designated the *rhtA* gene (rht: resistance to homoserine and threonine). Also, it is known that the *rhtA23* mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A). Hereinafter, the *rhtA23* mutation is marked as *rhtA*.*

The *asd* gene of *E*. *coli* has already been elucidated (nucleotide positions 3572511 to 3571408, GenBank accession NC_000913.1, gi:16131307), and can be obtained by PCR (polymerase chain reaction; refer to White, T.J. et al., Trends Genet., 5, 185 (1989)) utilising primers prepared based on the nucleotide sequence of the gene. The *asd* genes of other microorganisms can be obtained in a similar manner.

Also, the *aspC* gene of *E. coli* has already been elucidated (nucleotide positions 983742 to 984932, GenBank accession NC_000913.1, gi:16128895), and can be obtained by PCR. The *aspC* genes of other microorganisms can be obtained in a similar manner.

### L-lysine-producing bacteria

Examples of L-lysine-producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine is present in the medium. Examples of the L-lysine analogue include, but are not limited to, oxalysine, lysine hydroxamate, S-(2-aminoethyl)-L-cysteine (AEC), γ-methyllysine, α-chlorocaprolactam, and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC 196 may be used as an L-lysine producing bacterium of *Escherichia coli.* This bacterial strain was bred by conferring AEC resistance to the strain W3110, which was derived from *Escherichia coli* K-12. The resulting strain was designated *Escherichia coli* AJ13069 and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of parent strains which can be used to derive L-lysine-producing bacteria of the present invention also include strains in which expression of one or more genes encoding an L-lysine biosynthetic enzyme are enhanced. Examples of such genes include, but are not limited to, genes encoding dihydrodipicolinate synthase (*dapA*), aspartokinase (*lysC*), dihydrodipicolinate reductase (*dapB*), diaminopimelate decarboxylase (*lysA*), diaminopimelate dehydrogenase (*ddh*) (U.S. Patent No. 6,040,160), phosphoenolpyruvate carboxylase (*ppc*), aspartate semialdehyde dehydrogenase (*asd*), and aspartase (*aspA*) (EP 1253195 A). In addition, the parent strains may have an increased level of expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (W02005/073390), or combinations thereof.

Examples of parent strains for deriving L-lysine-producing bacteria of the present invention also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (W02005/010175).

Examples of L-lysine producing strains include *E. coli* WC196ΔcadAΔldc/pCABD2 (WO2006/078039). This strain was obtained by introducing the plasmid pCABD2, which is disclosed in U.S. Patent No. 6,040,160, into the strain WC196 with the disrupted *cadA* and *ldcC* genes, which encode lysine decarboxylase. The plasmid pCABD2 contains the *dapA* gene of *E. coli* coding for a dihydrodipicolinate synthase having a mutation which desensitizes feedback inhibition by L-lysine, the *lysC* gene of *E. coli* coding for aspartokinase III having a mutation which desensitizes feedback inhibition by L-lysine, the *dapB* gene *E. coli* coding for a dihydrodipicolinate reductase, and the *ddh* gene of Corynebacterium glutamicum coding for diaminopimelate dehydrogenase..

### L-cysteine-producing bacteria

Examples of parent strains which can be used to derive L-cysteine-producing bacteria of the present invention include, but are not limited to, strains belonging to the genus *Escherichia,* such as *E. coli* JM15 which is transformed with different *cysE* alleles coding for feedback-resistant serine acetyltransferases (U.S. Patent No. 6,218,168, Russian patent application 2003121601); *E. coli* W3110 which over-expresses genes which encode proteins suitable for secreting substances toxic for cells (U.S. Patent No. 5,972,663); *E. coli* strains having lowered cysteine desulfohydrase activity (JP11155571A2); *E. coli* W3110 with increased activity of a positive transcriptional regulator for cysteine regulon encoded by the *cysB* gene (WO0127307A1), and the like.

### L-leucine-producing bacteria

Examples of parent strains which can be used to derive L-leucine-producing bacteria of the present invention include, but are not limited to, strains belonging to the genus *Escherichia,* such as *E. coli* strains resistant to leucine (for example, the strain 57 (VKPM B-7386, U.S. Patent No. 6,124,121)) or leucine analogs including β-2-thienylalanine, 3-hydroxyleucine, 4-azaleucine, 5,5,5-trifluoroleucine (JP 62-34397 B and JP 8-70879 A); *E. coli* strains obtained by the genetic engineering methods such as those described in WO96/06926; *E. coli* H-9068 (JP 8-70879 A), and the like.

The bacterium of the present invention may be improved by enhancing the expression of one or more genes involved in L-leucine biosynthesis. Examples include genes of the *leuABCD* operon, which are preferably represented by a mutant *leuA* gene coding for isopropylmalate synthase which is not subject to feedback inhibition by L-leucine (US Patent 6,403,342). In addition, the bacterium of the present invention may be improved by enhancing the expression of one or more genes coding for proteins which excrete L-amino acids from the bacterial cell. Examples of such genes include the b2682 and b2683 genes (*ygaZH* genes) (EP 1239041 A2).

### L-histidine-producing bacteria

Examples of parent strains which can be used to derive L-histidine-producing bacteria of the present invention include, but are not limited to, strains belonging to the genus *Escherichia,* such as *E. coli* strain 24 (VKPM B-5945, RU2003677), *E. coli* strain 80 (VKPM B-7270, RU2119536), *E. coli* NRRL B-12116 - B12121 (U.S. Patent No. 4,388,405), *E. coli* H-9342 (FERM BP-6675) and H-9343 (FERM BP-6676) (U.S. Patent No. 6,344,347), *E. coli* H-9341 (FERM BP-6674) (EP1085087), *E. coli* AI80/pFM201 (U,S. Patent No. 6,258,554), and the like.

Examples of parent strains which can be used to derive L-histidine-producing bacteria of the present invention also include strains in which expression of one or more genes encoding an L-histidine biosynthetic enzyme are enhanced. Examples of such genes include genes encoding ATP phosphoribosyltransferase (*hisG*), phosphoribosyl AMP cyclohydrolase (*hisI*), phosphoribosyl-ATP pyrophosphohydrolase (*hisIE*), phosphoribosylformimino-5-aminoimidazole carboxamide ribotide isomerase (*hisA*), amidotransferase (*hisH*), histidinol phosphate aminotransferase (*hisC*), histidinol phosphatase (*hisB*), histidinol dehydrogenase (*hisD*), and so forth.

It is known that the L-histidine biosynthetic enzymes encoded by *hisG* and *hisBHAFI* are inhibited by L-histidine, and therefore an L-histidine-producing ability can also be efficiently enhanced by introducing a mutation into any of these genes which confer resistance to the feedback inhibition into enzymes encoded by the genes (Russian Patent Nos. 2003677 and 2119536).

Specific examples of strains having an L-histidine-producing ability include *E. coli* FERM P-5038 and 5048 which have been transformed with a vector carrying a DNA encoding an L-histidine-biosynthetic enzyme (JP 56-005099 A), *E. coli* strains transformed with *rht,* a gene for an amino acid-exporter (EP1016710A), *E. coli* 80 strain imparted with sulfaguanidine, DL-1,2,4-triazole-3-alanine, and streptomycin-resistance (VKPM B-7270, Russian Patent No. 2119536), and so forth.

### L-glutamic acid-producing bacteria

Examples of parent strains which can be used to derive L-glutamic acid-producing bacteria of the present invention include, but are not limited to, strains belonging to the genus *Escherichia,* such as *E. coli* VL334thrC⁺(EP 1172433). *E. coli* VL334 (VKPM B-1641) is an L-isoleucine and L-threonine auxotrophic strain having mutations in the *thrC* and *ilvA* genes (U.S. Patent No. 4,278,765). A wild-type allele of the *thrC* gene was transferred using general transduction with a bacteriophage P1 grown on the wild-type *E. coli* strain K12 (VKPM B-7) cells. As a result, an L-isoleucine auxotrophic strain VL334thrC⁺ (VKPM B-8961), which is able to produce L-glutamic acid, was obtained.

Examples of parent strains which can be used to derive the L-glutamic acid-producing bacteria of the present invention include, but are not limited to, strains which are deficient in α-ketoglutarate dehydrogenase activity, or strains in which expression of one or more genes encoding an L-glutamic acid biosynthetic enzyme are enhanced. Examples of such genes include genes encoding glutamate dehydrogenase (*gdh*), glutamine synthetase (*glnA*), glutamate synthetase (*gltAB*), isocitrate dehydrogenase (*icdA*), aconitate hydratase (*acnA, acnB*), citrate synthase (*gltA*), phosphoenolpyruvate carboxylase (*ppc*), pyruvate dehydrogenase (*aceEF, lpdA*), pyruvate kinase (*pykA, pykF*), phosphoenolpyruvate synthase (*ppsA*), enolase (*eno*), phosphoglyceromutase (*pgmA, pgmI*), phosphoglycerate kinase (*pgk*), glyceraldehyde-3-phophate dehydrogenase (*gapA*), triose phosphate isomerase (*tpiA*), fructose bisphosphate aldolase (*fbp*), phosphofructokinase (*pfkA, pfkB*), glucose phosphate isomerase (*pgi*), and so forth.

Examples of strains which have been modified so that expression of the citrate synthetase gene and/or the phosphoenolpyruvate carboxylase gene are reduced, and/or are deficient in α-ketoglutarate dehydrogenase activity include those disclosed in EP1078989A, EP955368A, and EP952221A.

Examples of parent strains which can be used to derive the L-glutamic acid-producing bacteria of the present invention also include strains having decreased or eliminated activity of an enzyme that catalyzes synthesis of a compound other than L-glutamic acid by branching off from an L-glutamic acid biosynthesis pathway. Examples of such enzymes include isocitrate lyase (*aceA*), α-ketoglutarate dehydrogenase (*sucA*), phosphotransacetylase (*pta*), acetate kinase (*ack*), acetohydroxy acid synthase (*ilvG*), acetolactate synthase (*ilvI*), formate acetyltransferase (*pfl*), lactate dehydrogenase (*ldh*), and glutamate decarboxylase (*gadAB*). Bacteria belonging to the genus *Escherichia* deficient in α-ketoglutarate dehydrogenase activity or having a reduced α-ketoglutarate dehydrogenase activity and methods for obtaining them are described in U.S. Patent Nos. 5,378,616 and 5,573,945. Specifically, these strains include the following:
*E. coli* W3110sucA::Km^{R}
*E. coli* AJ12624 (FERM BP-3853)
*E. coli* AJ12628 (FERM BP-3854)
*E. coli* AJ12949 (FERM BP-4881)
*E. coli* W3110sucA::Km^{R} is obtained by disrupting the α-ketoglutarate dehydrogenase gene (hereinafter referred to as "*sucA* gene") of *E*. *coli* W3110. This strain is completely deficient in the α-ketoglutarate dehydrogenase activity.

Other examples of L-glutamic acid-producing bacteria include those which belong to the genus *Escherichia* and have resistance to an aspartic acid antimetabolite. These strains can also be deficient in the α-ketoglutarate dehydrogenase activity and include, for example, *E. coli* AJ13199 (FERM BP-5807) (U.S. Patent No. 5.908,768), FFRM P-12379, which additionally has a low L-glutamic acid decomposing ability (U.S. Patent No. 5,393,671), AJ13138 (FERM BP-5565) (U.S. Patent No. 6,110,714), and the like.

Examples of L-glutamic acid-producing bacteria, include mutant strains belonging to the genus *Pantoea* which are deficient in α-ketoglutarate dehydrogenase activity or have decreased α-ketoglutarate dehydrogenase activity, and can be obtained as described above. Such strains include *Pantoea ananatis* AJ13356. (U.S. Patent No. 6,331,419). *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 under an accession number of FERM P-16645. It was then converted to an international deposit under the provisions of Budapest Treaty on January 11, 1999 and received an accession number of FERM BP-6615. *Pantoea ananatis* AJ13356 is deficient in the α-ketoglutarate dehydrogenase activity as a result of disruption of the αKGDH-E1 subunit gene (*sucA*). The above strain was identified as *Enterobacter agglomerans* when it was isolated and deposited as *Enterobacter agglomerans* AJ13356. However, it was recently re-classified as *Pantoea ananatis* on the basis of nucleotide sequencing of 16S rRNA and so forth. Although AJ13356 was deposited at the aforementioned depository as *Enterobacter agglomerans,* for the purposes of this specification, they are described as *Pantoea ananatis.*

### L-phenylalanine-producing bacteria

Examples of parent strains which can be used to derive L-phenylalanine-producing bacteria of the present invention include, but are not limited to, strains belonging to the genus *Escherichia,* such as *E. coli* AJ12739 (tyrA::Tn10, tyrR) (VKPM B-8197), *E. coli* HW1089 (ATCC 55371) harboring the mutant *pheA34* gene (U.S. Patent No. 5,354,672), *E. coli* MWEC101-b (KR8903681), *E. coli* NRRL B-12141, NRRL B-12145, NRRL B-12146 and NRRL B-12147 (U.S. Patent No. 4,407,952). Also, as a parent strain, *E. coli* K-12 [W3110 (tyrA)/pPHAB (FERM BP-3566), *E. coli* K-12 [W3110 (tyrA)/pPHAD] (FERM BP-12659), *E. coli* K-12 [W3110 (tyrA)/pPHATerm] (FERM BP-12662) and *E. coli* K-12 [W3110 (tyrA)/pBR-aroG4, pACMAB] named as AJ 12604 (FERM BP-3579) may be used (EP 488424 B1). Furthermore, L-phenylalanine producing bacteria belonging to the genus *Escherichia* with an enhanced activity of the protein encoded by the *yedA* gene or the *yddG* gene may also be used (U.S. patent applications 2003/0148473 A1 and 2003/0157667 A1).

### L-tryptophan-producing bacteria

Examples of parent strains which can be used to derive the L-tryptophan-producing bacteria of the present invention include, but are not limited to, strains belonging to the genus *Escherichia,* such as *E. coli* JP4735/pMU3028 (DSM10122) and JP6015/pMU91 (DSM10123) which is deficient in tryptophanyl-tRNA synthetase encoded by the mutant *trpS* gene (U.S. Patent No. 5,756,345), *E. coli* SV164 (pGH5) having a *serA* allele encoding phosphoglycerate dehydrogenase which is not subject to feedback inhibition by serine and a *trpE* allele encoding anthranilate synthase which is not subject to feedback inhibition by tryptophan (U.S. Patent No. 6,180,373), *E. coli* AGX17 (pGX44) (NRRL B-12263) and AGX6(pGX50)aroP (NRRL B-12264) which is deficient in the enzyme tryptophanase (U.S. Patent No. 4,371,614), *E*. *coli* AGX17/pGX50,pACKG4-pps in which a phosphoenolpyruvate-producing ability is enhanced (WO9708333, U.S. Patent No. 6,319,696), and the like. L-tryptophan-producing bacteria belonging to the genus *Escherichia* which have enhanced activity of the protein encoded by the *yedA* or *yddG* genes may also be used (U.S. patent applications 2003/0148473 A1 and 2003/0157667 A1).

Examples of parent strains which can be used to derive the L-tryptophan-producing bacteria of the present invention also include strains in which one or more activities are enhanced of the following enzymes: anthranilate synthase (*trpE*), phosphoglycerate dehydrogenase (*serA*), and tryptophan synthase (*trpAB*). The anthranilate synthase and phosphoglycerate dehydrogenase are both subject to feedback inhibition by L-tryptophan and L-serine, therefore a mutation desensitizing the feedback inhibition may be introduced into these enzymes. Specific examples of strains having such a mutation include *E. coli* SV164 which harbors desensitized anthranilate synthase and a transformant strain obtained by introducing into *E. coli* SV164 the plasmid pGH5 (WO 94/08031), which contains a mutant *serA* gene encoding feedback-desensitized phosphoglycerate dehydrogenase.

Examples of parent strains which can be used to derive the L-tryptophan-producing bacteria of the present invention also include strains which have been transformed with the tryptophan operon containing a gene encoding desensitized anthranilate synthase (JP 57-71397 A, JP 62-244382 A, U.S. Patent No. 4,371,614). Moreover, L-tryptophan-producing ability may be imparted by enhancing expression of a gene which encodes tryptophan synthase, among tryptophan operons (*trpBA*). Tryptophan synthase consists of α and β subunits which are encoded by the *trpA* and *trpB* genes, respectively. In addition, L-tryptophan-producing ability may be improved by enhancing expression of the isocitrate lyase-malate synthase operon (WO2005/103275).

### L-proline-producing bacteria

Examples of parent strains which can be used to derive L-proline-producing bacteria of the present invention include, but are not limited to, strains belonging to the genus *Escherichia,* such as *E. coli* 702ilvA (VKPM B-8012) which is deficient in the *ilvA* gene and is able to produce L-proline (EP 1172433). The bacterium of the present invention may be improved by enhancing the expression of one or more genes involved in L-proline biosynthesis. Examples of such genes include the *proB* gene coding for glutamate kinase which is desensitized to feedback inhibition by L-proline (DE Patent 3127361). In addition, the bacterium of the present invention may be improved by enhancing the expression of one or more genes coding for proteins responsible for secreting L-amino acids from the bacterial cell. Such genes are exemplified by the b2682 and b2683 genes (*ygaZH* genes) (EP 1239041 A2).

Examples of bacteria belonging to the genus *Escherichia,* which have an activity to produce L-proline include the following *E. coli* strains: NRRL B-12403 and NRRL B-12404 (GB Patent 2075056), VKPM B-8012 (Russian patent application 2000124295), plasmid mutants described in DE Patent 3127361, plasmid mutants described by Bloom F.R. et al (The 15th Miami winter symposium, 1983, p.34), and the like.

### L-arginine-producing bacteria

Examples of parent strains which can be used to derive L-arginine-producing bacteria of the present invention include, but are not limited to, strains belonging to the genus *Escherichia,* such as *E. coli* strain 237 (VKPM B-7925) (U.S. Patent Application 2002/058315 A1) and derivatives thereof harboring mutant N-acetylglutamate synthase (Russian Patent Application No. 2001112869), *E. coli* strain 382 (VKPM B-7926) (EP1170358A1), an arginine-producing strain transformed with the *argA* gene encoding N-acetylglutamate synthetase (EP1170361A1), and the like.

Examples of parent strains which can be used to derive L-arginine producing bacteria of the present invention also include strains in which expression of one or more genes encoding an L-arginine biosynthetic enzyme are enhanced. Examples of such genes include genes encoding N-acetylglutamyl phosphate reductase (*argC*), ornithine acetyl transferase (*argJ*), N-acetylglutamate kinase (*argB*), acetylornithine transaminase (*argD*), ornithine carbamoyl transferase (*argF*), argininosuccinic acid synthetase (*argG*), argininosuccinic acid lyase (*argH*), carbamoyl phosphate synthetase (*carAB*), and so forth.

### L-valine-producing bacteria

Example of parent strains which can be used to derive L-valine-producing bacteria of the present invention include, but are not limited to, strains which have been modified to overexpress the *ilvGMEDA* operon (U.S. Patent No. 5,998,178). It is desirable to remove the region of the *ilvGMEDA* operon responsible for attenuation so that the produced L-valine cannot attenuate expression of the operon. Furthermore, the *ilvA* gene in the operon is desirably disrupted so that threonine deaminase activity is decreased.

Examples of parent strains which can be used to derive L-valine-producing bacteria of the present invention also include mutants of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 has been deposited in the Russian National Collection of Industrial Microorganisms (VKPM) (Russia, 117545 Moscow, 1 Dorozhny Proezd, 1) on June 24, 1988 under accession number VKPM B-4411.

Furthermore, mutants requiring lipoic acid for growth and/or lacking H⁺-ATPase can also be used as parent strains (WO96/06926).

### L-isoleucine-producing bacteria

Examples of parent strains which can be used to derive L-isoleucine producing bacteria of the present invention include, but are not limited to, mutants having resistance to 6-dimethylaminopurine (JP 5-304969 A), mutants having resistance to an isoleucine analogue such as thiaisoleucine and isoleucine hydroxamate, and mutants additionally having resistance to DL-ethionine and/or arginine hydroxamate (JP 5-130882 A). In addition, recombinant strains transformed with genes encoding proteins involved in L-isoleucine biosynthesis, such as threonine deaminase and acetohydroxate synthase, can also be used as parent strains (JP 2-458 A, FR 0356739, and U.S. Patent No. 5,998,178).

The method for producing an L-amino acid of the present invention includes the steps of cultivating the bacterium of the present invention in a culture medium, allowing L-amino acid to accumulate in the culture medium, and collecting L-amino acid from the culture medium. Furthermore, the method of present invention includes a method for producing L-threonine, L-lysine, L-histidine, L-phenylalanine, L-arginine, L-tryptophan, L-glutamic acid, or L-leucine, including the steps of cultivating the bacterium of the present invention in a culture medium, allowing L-threonine, L-lysine, L-histidine, L-phenylalanine, L-arginine, L-tryptophan, L-glutamic acid, or L-leucine to accumulate in the culture medium, and collecting L-threonine, L-lysine, L-histidine, L-phenylalanine, L-arginine, L-tryptophan, L-glutamic acid, or L-leucine from the culture medium.

In the present invention, the cultivation, collection, and purification of L-amino acids from the medium and the like may be performed by conventional fermentation methods wherein an L-amino acid is produced using a bacterium.

The culture medium may be either synthetic or natural, so long as the medium includes a carbon source, a nitrogen source, minerals, and if necessary, appropriate amounts of nutrients which the bacterium requires for growth. The carbon source may include various carbohydrates such as glucose and sucrose, various organic acids and alcohols, such as ethanol. According to the present invention ethanol can be used as the sole carbon source or mixed with carbohydrates, such as glucose and sucrose. As the nitrogen source, various ammonium salts such as ammonia and ammonium sulfate, other nitrogen compounds such as amines, a natural nitrogen source such as peptone, soybean-hydrolysate, and digested fermentative microorganisms can be used. As minerals, potassium monophosphate, magnesium sulfate, sodium chloride, ferrous sulfate, manganese sulfate, calcium chloride, and the like, can be used. As vitamins, thiamine, yeast extract, and the like may be used. Additional nutrients may be added to the medium, if necessary. For example, if the bacterium requires an L-amino acid for growth (L-amino acid auxotrophy), a sufficient amount of the L-amino acid may be added to the cultivation medium.

The cultivation is preferably performed under aerobic conditions such as a shaking culture, and stirring culture with aeration, at a temperature of 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the target L-amino acid can be collected and purified by ion-exchange, concentration, and/or crystallization methods.

### BRIEF DESCRIPTION OF THE DRAWINGS

Figure 1 shows the structure of the upstream region of the *adhE* gene in the chromosome of *E*. *coli* and the structure of an integrated DNA fragment containing the *cat* gene and a P_{L-tac} promoter.
Figure 2 shows the alignment of the primary sequences of alcohol dehydrogenase from *Escherichia coli* (ADHE_ECOLI, SEQ ID NO: 2), *Shigella flexneri* (Q83RN2_SHIFL, SEQ ID NO: 53), *Pantoea ananatis* (ADHE PANAN, SEQ ID NO: 30), *Yersinia pestis* (Q66AM7_YERPS, SEQ ID NO: 54), *Erwinia carotovora* (Q6D4R4_ERWCT, SEQ ID NO: 55), *Salmonella typhimurium* (P74880_SALTY, SEQ ID NO: 56), *Lactobacillus plantarum* (Q88RY9_LACPL, SEQ ID NO: 57) and *Lactococcus lactis* (086282_9LACT, SEQ ID NO: 58). The alignment was done by using the PIR Multiple Alignment program (http://pir.georgetown.edu). The identical amino acids are marked by asterisk (*), similar amino acids are marked by colon (:).
Figure 3 shows growth curves of modified strains grown on the minimal M9 medium containing ethanol (2% or 3%) as a sole carbon source.
Figure 4 shows growth curves of modified strains grown on the minimal M9 medium containing a mixture of glucose (0.1 weight %) and ethanol (0.1 volume %).
Figure 5 shows comparison of growth curves of strains having mutant adhE* gene under control of the native promoter, or P_{L-tac} promoter grown on the minimal M9 medium containing ethanol (2% or 3%) as a sole carbon source.

### Examples

The present invention will be more concretely explained below with reference to the following non-limiting examples.

### Example 1. Preparation of E. coli MG1655 Δtdh, rhtA*

The L-threonine producing *E. coli* strain MG1655 Δtdh, rhtA* (pVIC40) was constructed by inactivation of the native *tdh* gene encoding threonine dehydrogenase in *E. coli* MG1655 (ATCC 700926) using the *cat* gene followed by introduction of an rhtA23 mutation (*rhtA**) which confers resistance to high concentrations of threonine (>40 mg/ml) and homoserine (>5 mg/ml). Then, the resulting strain was transformed with plasmid pVIC40 from *E. coli* VKPM B-3996. The plasmid pVIC40 is described in detail in U.S. Patent No. 5,705,371.

To replace the native *tdh* gene, a DNA fragment carrying the chloramphenicol resistance marker (Cm^{R}) encoded by the *cat* gene was integrated into the chromosome of *E*. *coli* MG1655 in place of the native gene by the method described by Datsenko K.A. and Wanner B.L. (Proc. Natl. Acad. Sci. USA, 2000, 97, 6640-6645) which is also called "Red-mediated integration" and/or "Red-driven integration". The recombinant plasmid pKD46 (Datsenko, K.A., Wanner, B.L., Proc. Natl. Acad. Sci. USA, 2000, 97, 6640-6645) with the thermosensitive replicon was used as the donor of the phage λ-derived genes responsible for the Red-mediated recombination system. *E. coli* BW25113 containing the recombinant plasmid pKD46 can be obtained from the *E*. *coli* Genetic Stock Center, Yale University, New Haven, USA, the accession number of which is CGSC7630.

A DNA fragment containing a Cm^{R} marker encoded by the *cat* gene was obtained by PCR using the commercially available plasmid pACYC184 (GenBank/EMBL accession number X06403, "Fermentas", Lithuania) as the template, and primers P1 (SEQ ID NO: 3) and P2 (SEQ ID NO: 4). Primer P1 contains 35 nucleotides homologous to the 5'-region of the *tdh* gene introduced into the primer for further integration into the bacterial chromosome. Primer P2 contains 32 nucleotides homologous to the 3'-region of the *tdh* gene introduced into the primer for further integration into the bacterial chromosome.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C for 40 sec; and the final elongation for 5 min at 72 °C. Then, the amplified DNA fragment was purified by agarose gel-electrophoresis, extracted using "GenElute Spin Columns" (Sigma, USA), and precipitated by ethanol.

The obtained DNA fragment was used for electroporation and Red-mediated integration into the bacterial chromosome of *E*. *coli* MG1655/pKD46.

MG1655/pKD46 cells were grown overnight at 30 °C in liquid LB-medium containing ampicillin (100 µg/ml), then diluted 1:100 by SOB-medium (Yeast extract, 5 g/l; NaCl, 0.5 g/l; Tryptone, 20 g/l; KCI, 2.5 mM; MgCl₂, 10 mM) containing ampicillin (100 µg/ml) and L-arabinose (10 mM) (arabinose is used for inducing the plasmid containing the genes of the Red system) and grown at 30 °C to reach the optical density of the bacterial culture OD₆₀₀=0.4-0.7. The grown cells from 10 ml of the bacterial culture were washed 3 times with ice-cold de-ionized water, followed by suspension in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated for 2 hours at 37°C, and then were spread onto L-agar containing 25 µg/ml of chloramphenicol. Colonies grown for 24 hours were tested for the presence of Cm^{R} marker instead of the native *tdh* gene by PCR using primers P3 (SEQ ID NO: 5) and P4 (SEQ ID NO: 6). For this purpose, a freshly isolated colony was suspended in 20µl water and then 1µl of obtained suspension was used for PCR. The temperature profile was the following: initial DNA denaturation for 5 min at 95 °C; then 30 cycles of denaturation at 95 °C for 30 sec, annealing at 55 °C for 30 sec and elongation at 72 °C for 30 sec; the final elongation for 5 min at 72 °C. A few Cm^{R} colonies tested contained the desired 1104 bp DNA fragment, confirming the presence of Cm^{R} marker DNA instead of 1242 bp fragment of *tdh* gene. One of the obtained strains was cured of the thermosensitive plasmid pKD46 by culturing at 37 °C and the resulting strain was named *E*. *coli* MG1655Δtdh.

Then, the rhtA23 mutation from the strain VL614rhtA23 (Livshits V.A. et al, 2003, Res. Microbiol., 154:123-135) was introduced into the obtained strain MG1655 Δtdh resulting in strain MG1655 Δtdh, rhtA*. The rhtA23 is a mutation which confers resistance to high concentrations of threonine (>40 mg/ml) and homoserine (>5 mg/ml). For that purpose the strain MG1655 Δtdh was infected with phage P1ᵥᵢᵣ grown on the donor strain VL614rhtA23. The transductants were selected on M9 minimal medium containing 8 mg/ml homoserine and 0.4% glucose as the sole carbon source.

### Example 2. Construction of E. coli MG1655::P_{L-tac}adhE

*E. coli* MG1655::P_{L-tac}adh was obtained by replacement of the native promoter region of the *adhE* gene in the strain MG1655 by P_{L-tac} promoter.

To replace the native promoter region of the *adhE* gene, the DNA fragment carrying a P_{L-tac} promoter and chloramphenicol resistance marker (Cm^{R}) encoded by the *cat* gene was integrated into the chromosome of *E*. *coli* MG1655 in the place of the native promoter region by the method described by Datsenko K.A. and Wanner B.L. (Proc. Natl. Acad. Sci. USA, 2000, 97, 6640-6645), which is also called "Red-mediated integration" and/or "Red-driven integration".

A fragment containing the P_{L-tac} promoter and the *cat* gene was obtained by PCR using chromosomal DNA of *E. coli* MG1655P_{L-tac}xylE (WO2006/043730) as a template. The nucleotide sequence of the P_{L-tac} promoter is presented in the Sequence listing (SEQ ID NO: 7). Primers P5 (SEQ ID NO: 8) and P6 (SEQ ID NO: 9) were used for PCR amplification. Primer P5 contains 40 nucleotides complementary to the region located 318 bp upstream of the start codon of the *adhE* gene introduced into the primer for further integration into the bacterial chromosome and primer P6 contains a 39 nucleotides identical to 5'-sequence of the *adhE* gene.

PCR was provided using the "Gene Amp PCR System 2700" amplificatory (Applied Biosystems). The reaction mixture (total volume - 50 µl) consisted of 5 µl of 10x PCR-buffer with 15 mM MgCl₂ ("Fermentas", Lithuania), 200 µM each of dNTP, 25 pmol each of the exploited primers and 1 U of Taq-polymerase ("Fermentas", Lithuania). Approximately 20 ng of the *E. coli* MG1655P_{L-tac}xylE genomic DNA was added in the reaction mixtures as a template for PCR.

The temperature profile was the following: initial DNA denaturation for 5 min at 95 °C, followed by 35 cycles of denaturation at 95 °C for 30 sec, annealing at 54 °C for 30 sec, elongation at 72 °C for 1.5 min and the final elongation for 5 min at 72 °C. Then, the amplified DNA fragment was purified by agarose gel-electrophoresis, extracted using "GenElute Spin Columns" ("Sigma", USA) and precipitated by ethanol. The obtained DNA fragment was used for electroporation and Red-mediated integration into the bacterial chromosome of the *E. coli* MG1655/pKD46.

MG1655/pKD46 cells were grown overnight at 30 °C in the liquid LB-medium containing ampicillin (100 µg/ml), then diluted 1:100 by SOB-medium (Yeast. extract, 5 g/l; NaCl, 0.5 g/l; Tryptone, 20 g/l; KCI, 2.5 mM; MgCl₂, 10 mM) containing ampicillin (100 µg/ml) and L-arabinose (10 mM) (arabinose is used for inducing the plasmid encoding genes of the Red system) and grown at 30 °C to reach the optical density of the bacterial culture OD₆₀₀=0,4-0.7. The grown cells from 10 ml of the bacterial culture were washed 3 times with ice-cold de-ionized water, followed by suspension in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions.

Shocked cells were added to 1-ml of SOC medium (Sambrook et al, "Molecular Cloning A Laboratory Manual, Second Edition", Cold Spring Harbor Laboratory Press (1989)), incubated for 2 hours at 37°C, and then were spread onto L-agar containing 25 µg/ml of chloramphenicol.

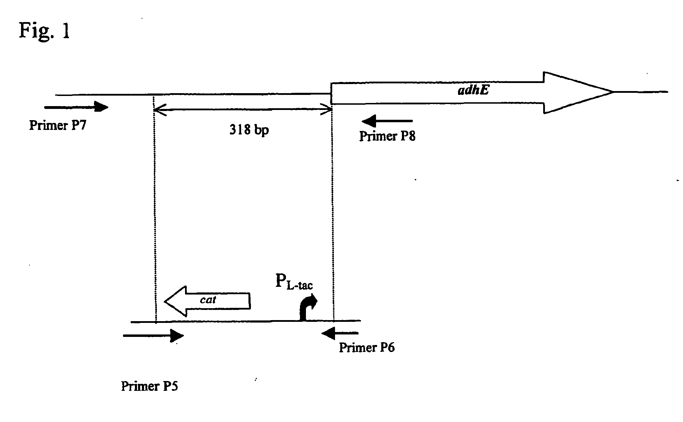

About 100 resulting clones were selected on M9 plates with 2% ethanol as the sole carbon source. Some clones which grew on M9 plates with 2% ethanol in 36 hours were chosen and tested for the presence of Cm^{R} marker instead of the native promoter region of the *adhE* gene by PCR using primers P7 (SEQ ID NO: 10) and P8 (SEQ ID NO: 11). For this purpose, a freshly isolated colony was suspended in 20µl water and then 1µl of the obtained suspension was used for PCR. The temperature profile follows: initial DNA denaturation for 10 min at 95 °C; then 30 cycles of denaturation at 95°C for 30 sec, annealing at 54 °C for 30 sec and elongation at 72°C for 1.5min; the final elongation for 1 min at 72°C. A few Cm^{R} colonies tested contained the desired ∼1800 bp DNA fragment, confirming the presence of Cm^{R} marker DNA instead of 520 bp native promoter region of *adhE* gene. One of the obtained strains was cured of the thermosensitive plasmid pKD46 by culturing at 37 °C and the resulting strain was named *E. coli* MG1655::P_{L-tac}adhE (See Figure 1).

### Example 3. Construction of E. coli MG1655Δtdh, rhtA*, P_{L-tac}adhE

*E. coli* MG1655Δtdh, rhtA*, P_{L-tac}adhE was obtained by transduction of the P_{L-tac} promoter from the strain MG1655::P_{L-tac}adhE into strain MG1655Δtdh, rhtA*.

The strain MG1655Δtdh, rhtA* was infected with phage Plᵥᵢᵣ grown on the donor strain MG1655::P_{L-tac}adhE, and the strain MG1655Δtdh, rhtA*, P_{L-tac}adhE was obtained. This strain was checked for growth on M9 plates with 2% ethanol as the sole carbon source. The growth rate was the same as for the strain MG1655::P_{L-tac}adhE.

### Example 4. The effect of increasing the adhE gene expression on L-threonine production

### To evaluate the effect of enhancing expression of the adhE gene on L-threonine production, both E. coli strains MG1655Δtdh, rhtA*, P_{L-tac}adhE and MG1655Δtdh, rhtA* were transformed with plasmid pVIC40.

The strain MG1655Δtdh, rhtA*, P_{L-tac}adhE (pVIC40) and a parent strain MG1655Δtdh, rhtA* (pVIC40) were each cultivated at 37 °C for 18 hours in a nutrient broth and 0.3 ml of each of the obtained cultures was inoculated into 3 ml of fermentation medium having the following composition in a 20x200 mm test tube and cultivated at 34°C for 48 hours with a rotary shaker. Data from at least 10 independent experiments are shown on Tables 1 and 2.

Fermentation medium composition (g/l):

| | |
|---|---|
| Ethanol | 24 or 16 |
| Glucose | 0 (Table 1) or 3 (Table 2) |
| (NH₄)₂SO₄ | 16 |
| K₂HPO₄ | 0.7 |
| MgSO₄·7H₂O | 1.0 |
| MnSO₄·5H₂O | 0.01 |
| FeSO₄·7H₂O | 0.01 |
| Thiamine hydrochloride | 0.002 |
| Yeast extract | 1.0 |
| L-isoleucine | 0.01 |
| CaCO₃ | 33 |

| | |
|---|---|
| MgSO₄·7H₂O and CaCO₃ were each sterilized separately. | |

It can be seen from the Tables 1 and 2, MG1655Δtdh, rhtA*, P_{L-tac}adhE was able to accumulate a higher amount of L-threonine as compared with MG1655Δtdh, rhtA*. Moreover, MG1655Δtdh, rhtA*, P_{L-tac}adhE was able to grow on the medium containing ethanol as the sole carbon source and cause accumulation of L-threonine, whereas MG1655Δtdh, rhtA* exhibited very poor growth and productivity in the medium containing ethanol as the sole carbon source.

### Example 5. Construction of E. coli MG1655ΔadhE

This strain was constructed by inactivation of the native *adhE* gene in *E*. *coli* MG1655 by the *kan* gene.

To inactivate (or disrupt) the native *adhE* gene, the DNA fragment carrying kanamycin resistance marker (Km^{R}) encoded by the *kan* gene was integrated into the chromosome of *E*. *coli* MG1655 (ATCC 700926) in place of the native gene by the method described by Datsenko K.A. and Wanner B.L. (Proc. Natl. Acad. Sci. USA, 2000, 97,6640-6645) which is also called "Red-mediated integration" and/or "Red-driven integration".

A DNA fragment containing a Km^{R} marker (kan gene) was obtained by PCR using the commercially available plasmid pACYC177 (GenBank/EMBL accession number X06402, "Fermentas", Lithuania) as the template, and primers P9 (SEQ ID NO: 12) and P10 (SEQ ID NO: 13). Primer P9 contains 40 nucleotides homologous to the region located 318 bp upstream of the start codon of the *adhE* gene introduced into the primer for further integration into the bacterial chromosome. Primer P10 contains 41 nucleotides homologous to the 3'-region of the *adhE* gene introduced into the primer for further integration into the bacterial chromosome.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C for 40 sec; and the final elongation for 5 min at 72 °C. Then, the amplified DNA fragment was purified by agarose gel-electrophoresis, extracted using "GenElute Spin Columns" ("Sigma", USA) and precipitated by ethanol.

The obtained DNA fragment was used for electroporation and Red-mediated integration into the bacterial chromosome of the *E. coli* MG1655/pKD46.

MG1655/pKD46 cells were grown overnight at 30 °C in liquid LB-medium containing ampicillin (100 µg/ml), then diluted 1:100 by SOB-medium (Yeast extract, 5 g/l; NaCl, 0.5 g/l; Tryptone, 20 g/l; KCI, 2.5 mM; MgCl₂, 10 mM) containing ampicillin (100 µg/ml) and L-arabinose (10 mM) (arabinose is used for inducing the plasmid encoding genes of Red system) and grown at 30 °C to reach the optical density of the bacterial culture OD₆₀₀=0,4-0.7. The grown cells from 10 ml of the bacterial culture were washed 3 times by the ice-cold de-ionized water, followed by suspension in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium (Sambrook et al, "Molecular Cloning A Laboratory Manual, Second Edition", Cold Spring Harbor Laboratory Press (1989)), incubated for 2 hours at 37°C, and then were spread onto L-agar containing 20 µg/ml of kanamycin. Colonies grown within 24 hours were tested for the presence of Km^{R} marker instead of the native *adhE* gene by PCR using primers P11 (SEQ ID NO: 14) and P12 (SEQ ID NO: 15). For this purpose, a freshly isolated colony was suspended in 20µl water and then 1µl of obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72 °C for 30 sec; the final elongation for 5 min at 72 °C. A few Km^{R} colonies tested contained the desired about 1030 bp DNA fragment, confirming the presence of Km^{R} marker DNA instead of the 3135 bp fragment of *adhE* gene. One of the obtained strains was cured of the thermosensitive plasmid pKD46 by culturing at 37 °C and the resulting strain was named *E*. *coli* MG1655ΔadhE.

### Example 6. Construction of E. coli MG1655::P_{L-tac}adhE*

*E. coli* MG1655::P_{L-tac}adhE* was obtained by introduction of the Glu568Lys (E568K) mutation into the *adhE* gene. First, 1.05 kbp fragment of the *adhE* gene carrying the E568K mutation was obtained by PCR using the genomic DNA of *E*. *coli* MG1655 as the template and primers P13 (SEQ ID NO: 16) and P12 (SEQ ID NO: 15). Primer P15 homologous to 1662-1701 bp and 1703-1730 bp regions of the *adhE* gene and includes the substitution **g/a** (position 1702 bp) shown as bold and primer P12 homologous to 3'-end of the *adhE* gene. PCR was provided using the "Gene Amp PCR System 2700" amplificatory (Applied Biosystems). The reaction mixture (total volume - 50 µl) consisted of 5 µl of 10x PCR-buffer with MgCl₂ ("TaKaRa", Japan), 250 µM each of dNTP, 25 pmol each of the exploited primers and 2.5 U of Pyrobest DNA polymerase ("TaKaRa", Japan). Approximately 20 ng of the *E. coli* MG1655 genomic DNA was added in the reaction mixtures as a template for PCR. The temperature profile was the following: initial DNA denaturation for 5 min at 95 °C, followed by 35 cycles of denaturation at 95 °C for 30 sec, annealing at 54 °C for 30 sec, elongation at 72 °C for 1min and the final elongation for 5 min at 72 °C. The fragment obtained was purified by agarose gel-electrophoresis, extracted using "GenElute Spin Columns" ("Sigma", USA) and precipitated with ethanol.

In the second step, the fragment containing the P_{L-tac} promoter with the mutant *adhE* gene and marked by the *cat* gene, which provides chloramphenicol resistance, was obtained by PCR using the genomic DNA of *E. coli* MG1655::P_{L-tac}adhE as the template (see Example 2), primer P11 (SEQ ID NO: 14) and a 1.05 kbp fragment carrying a mutant sequence (see above) as a second primer. Primer P11 is homologous to the region located at 402-425 bp upstream of the start codon of the *adhE* gene. PCR was provided using the "Gene Amp PCR System 2700" amplificatory (Applied Biosystems). The reaction mixture (total volume - 50 µl) consisted of 5 µl of 10x PCR-buffer ("TaKaRa", Japan), 25mM MgCl₂, 250 µM each of dNTP, 10 ng of the primer P11, 1µg of the 1.05 kbp fragment as a second primer and 2.5U of TaKaRa LA DNA polymerase ("TaKaRa", Japan). Approximately 20 ng of the *E. coli* MG1655::P_{L-tac}adhE genomic DNA was added to the reaction mixture as a template for PCR. The temperature profile was the following: initial DNA denaturation for 5 min at 95 °C, followed by 35 cycles of denaturation at 95 °C for 30 sec, annealing at 54 °C for 30 sec, elongation at 72 °C for 3.5 min and the final elongation for 7 min at 72 °C. The resulting fragment was purified by agarose gel-electrophoresis, extracted using "GenElute Spin Columns" ("Sigma", USA) and precipitated by ethanol.

To replace the native region of the *adhE* gene, the DNA fragment carrying a P_{L-tac} promoter with the mutant *adhE* and chloramphenicol resistance marker (Cm^{R}) encoded by the *cat* gene (cat-P_{L-tac}adhE*, 4.7 kbp) was integrated into the chromosome of *E*. *coli* MG1655ΔadhE by the method described by Datsenko K.A. and Wanner B.L. (Proc. Natl. Acad. Sci. USA, 2000, 97, 6640-6645) which is also called "Red-mediated integration" and/or "Red-driven integration". MG1655 ΔadhE/pKD46 cells were grown overnight at 30 °C in liquid LB-medium containing ampicillin (100 µg/ml), then diluted 1:100 by SOB-medium (Yeast extract, 5 g/l; NaCl, 0.5 g/l; Tryptone, 20 g/l; KCI, 2.5 mM; MgCl₂, 10 mM) containing ampicillin (100 µg/ml) and L-arabinose (10 mM) (arabinose is used for inducing the plasmid encoding genes of the Red system) and grown at 30 °C to reach the optical density of the bacterial culture OD₆₀₀=0,4-0.7. The grown cells from 10 ml of the bacterial culture were washed 3 times by the ice-cold de-ionized water, followed by suspension in 100 µl of the water. 10 µl of DNA fragment (300 ng) dissolved in the de-ionized water was added to the cell suspension. The electroporation was performed by "Bio-Rad" electroporator (USA) (No. 165-2098, version 2-89) according to the manufacturer's instructions.

Shocked cells were added to 1-ml of SOC medium (Sambrook et al, "Molecular Cloning A Laboratory Manual, Second Edition", Cold Spring Harbor Laboratory Press (1989)), incubated for 2 hours at 37 °C, and then were spread onto L-agar containing 25 µg/ml of chloramphenicol.

The clones obtained were selected on M9 plates with 2% ethanol as the sole carbon source.

The runaway clone was chosen and the full gene sequence was verified. The row of mutations was revealed as follows: Glu568Lys (gag - aag), Ile554Ser (atc - agc), Glu22Gly (gaa -gga), Met236Val (atg - gtg), Tyr461 Cys (tac - tgc), Ala786Val (gca - gta). This clone was named MG1655::P_{L-tac}adhE*.

### Example 7. Construction of E. coli MG1655Δtdh, rhtA*, P_{L-tac}adhE*

*E. coli* MG1655Δtdh, rhtA*, P_{L-tac}adhE* was obtained by transduction of the P_{L-tac} adhE* mutation from the strain MG1655::P_{L-tac}adhE*.

The strain MG1655Δtdh, rhtA* was infected with phage P1ᵥᵢᵣ grown on the donor strain MG1655::P_{L-tac}adhE* and the strain MG1655Δtdh, rhtA*, P_{L-tac}adhE* was obtained. This strain was checked for growth on M9 plates with 2% ethanol as a sole carbon source. The growth rate was the same as for the strain MG1655::P_{L-tac}adhE*.

### Example 8. Construction of E. coli MG1655Δtdh, rhtA*, P_{L-tac}adhE-Lys568

A second attempt to obtain a single mutant *adhE* having the Glu568Lys mutation was performed. For that purpose *E*. *coli* strain MG1655Δtdh, rhtA*, P_{L-tac}adhE-wtΔ34 was constructed.

*E. coli* MG1655Δtdh, rhtA*, P_{L-tac}adhE-wtΔ34 was obtained by replacement of a 34bp fragment of the *adhE* gene (the region from 1668 to 1702 bp, inclusive of the triplet encoding Glu568) in *E*. *coli* MG1655Δtdh, rhtA*, P_{L-tac}adhE-wt (wt means a wild type) with *kan* gene. The *kan* gene was integrated into the chromosome of *E*. *coli* MG1655Δtdh, rhtA*, P_{L}-_{tac}adhE-wt by the method, described by Datsenko K.A. and Wanner B.L. (Proc.Natl.Acad.Sci.USA, 2000, 97, 6640-6645) which is also called "Red-mediated integration" and/or "Red-driven integration".

A DNA fragment containing a Km^{R} marker encoded by the *kan* gene was obtained by PCR using the commercially available plasmid pACYC177 (GenBank/EMBL accession number X06402, "Fermentas", Lithuania) as the template, and primers P 14 (SEQ ID NO: 17) and P15 (SEQ ID NO: 18). Primer P14 contains 41 nucleotides identical to the region from 1627 to 1668 bp of *adhE* gene and primer P 15 contains 39 nucleotides complementary to the region from 1702 to 1740 bp of *adhE* gene introduced into the primers for further integration into the bacterial chromosome.

PCR was provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 5 ng of the plasmid DNA was added in the reaction mixture as a template DNA for the PCR amplification. The temperature profile was the following: initial DNA denaturation for 5 min at 95 °C, followed by 25 cycles of denaturation at 95 °C for 30 sec, annealing at 55 °C for 30 sec, elongation at 72 °C 50 sec and the final elongation for 5 min at 72 °C. Then, the amplified DNA fragment was purified by agarose gel-electrophoresis, extracted using "GenElute Spin Columns" ("Sigma", USA) and precipitated by ethanol.

Colonies obtained were tested for the presence of Km^{R} marker by PCR using primers P16 (SEQ ID NO: 19) and P17 (SEQ ID NO: 20). For this purpose, a freshly isolated colony was suspended in 20µl water and then 1µl of the obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72° C for 45 sec; the final elongation for 5 min at 72 °C. A few Km^{R} colonies tested contained the desired 1200 bp DNA fragment, confirming the presence of Km^{R} marker DNA instead of 230 bp fragment of native *adhE* gene. One of the obtained strains was cured of the thermosensitive plasmid pKD46 by culturing at 37 °C and the resulting strain was named as *E. coli* MG1655Δtdh, rhtA*,P_{L-tac}adhE-wtΔ34.

Then, to replace the kanamycin resistance marker (Km^{R}) encoded by *kan* gene with a fragment of the *adhE* gene encoding the Glu568Lys mutation, the oligonucleotides P18 (SEQ ID NO: 21) and P19 (SEQ ID NO: 22) carrying the appropriate mutation were integrated into the chromosome of *E*. *coli* MG1655Δtdh, rhtA, P_{L-tac}adhE-wt Δ34 by the method "Red-mediated integration" and/or "Red-driven integration" (Yu D., Sawitzke J. et al., Recombineering with overlapping single-stranded DNA oligonucleotides: Testing of recombination intermediate, PNAS, 2003, 100(12), 7207-7212). Primer P18 contains 75 nucleotides identical to the region from 1627 to 1702 bp of *adhE* gene and primer P 19 contains 75 nucleotides complementary to the region from 1668 to 1740 bp of *adhE* gene, both primers inclusive of the triplet encoding Lys568 instead of Glu568.

The clones were selected on M9 minimal medium containing 2% ethanol and 25mg/ml succinate as a carbon source.

Colonies were tested for the absence of Km^{R} marker by PCR using primers P16 (SEQ ID NO: 19) and P17 (SEQ ID NO: 20). For this purpose, a freshly isolated colony was suspended in 20µl water and then 1µl of the obtained suspension was used for PCR. The temperature profile follows: initial DNA denaturation for 5 min at 95 °C; then 30 cycles of denaturation at 95 °C for 30 sec, annealing at 55 °C for 30 sec and elongation at 72°C for 25 sec; the final elongation for 5 min at 72 °C. A few Km^{S} colonies tested contained the desired 230 bp DNA fragment of *adhE* gene, confirming the absence of Km^{R} marker DNA instead of 1200 bp fragment. Several of the obtained strains was cured of the thermosensitive plasmid pKD46 by culturing at 37 °C and the resulting strain was named as *E. coli* MG1655Δtdh, rhtA, P_{L-tac}adhE-Lys568.

The presence of the Glu568Lys mutation was confirmed by sequencing, for example, cl.18 has a single mutation Glu568Lys. Addditionally it was found that some clones (#1, 13) contained additional mutations: cl. 1 - Glu568Lys, Phe566Val; cl.13 - Glu568Lys, Glu560Lys.

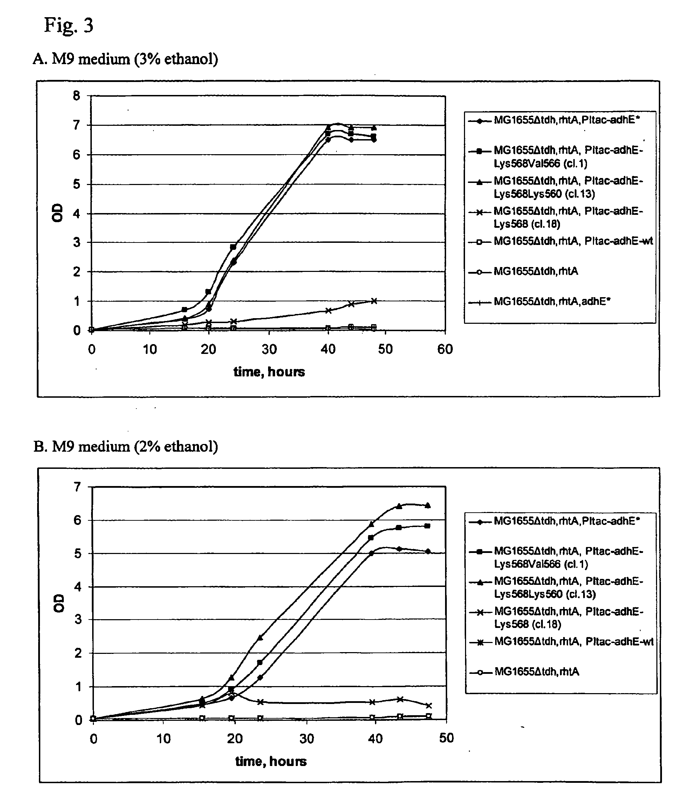

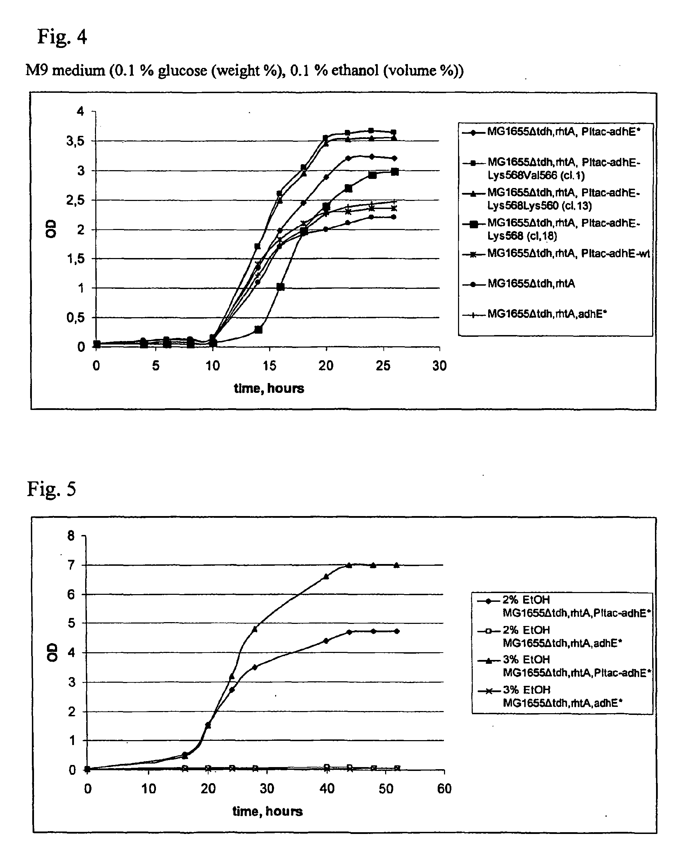

For strains MG1655Δtdh, rhtA*,P_{L-tac}adhE-Lys568 (cl.18), MG1655Δtdh, rhtA*,P_{L}-_{tac}adhE-Lys568,Val566 (cl.1), MG1655Δtdh, rhtA*,P_{L-tac}adhE-Lys568,Lys560 (cl.13) and MG1655Δtdh, rhtA*,P_{L-tac}adhE*, the growth curves were studied (Figures 3 and 4).

The strains were grown in M9 medium with ethanol as a sole carbon source and in M9 medium with glucose and ethanol (molar ratio 1:3)

### Example 9. Construction of E. coli MG1655Δtdh, rhtA*, adhE*

The *E. coli* strain MG1655Δtdh, rhtA*, adhE* was obtained by reconstruction of the native *adhE* promoter in strain MG1655Δtdh, rhtA*, P_{L-tac}adhE*. A DNA fragment carrying a P_{L-tac} promoter and chloramphenicol resistance marker (Cm^{R}) encoded by *cat* gene in the chromosome of the strain MG1655Δtdh, rhtA*, P_{L-tac}adhE* was replaced by a fragment carrying native *adhE* promoter and kanamycin resistance marker (Km^{R}) encoded by the *kan* gene. Native P_{adhE} was obtained by PCR using a DNA of the strain MG1655 as a template and primers P20 (SEQ ID NO: 23) and P21 (SEQ ID NO: 24). Primer P20 contains an *EcoRI* recognition site at the 5'-end thereof, which is necessary for further joining to the *kan* gene and primer P21 contains 30 nucleotides homologous to 5'-region of the adhE gene (from 50 bp to 20 bp).

A DNA fragment containing a Km^{R} marker encoded by the *kan* gene was obtained by PCR using the commercially available plasmid pACYC177 (GenBank/EMBL accession number X06402, "Fermentas", Lithuania) as the template, and primers P22 (SEQ ID NO: 25) and P23 (SEQ ID NO: 26). Primer P22 contains 41 nucleotides homologous to the region located 425 bp upstream of the start codon of the *adhE* gene introduced into the primer for further integration into the bacterial chromosome and primer P23 contains an *Eco*RI recognition site at the 3'-end thereof, which is necessary for further joining to the P_{adhE} promoter.

PCR were provided using the "Gene Amp PCR System 2700" amplificatory (Applied Biosystems). The reaction mixture (total volume - 50 µl) consisted of 5 µl of 10x PCR-buffer with 25 mM MgCl₂ ("Fermentas", Lithuania), 200 µM each of dNTP, 25 pmol each of the exploited primers and 1 U of Taq-polymerase ("Fermentas", Lithuania). Approximately 20 ng of genomic DNA or 5 ng of the plasmid DNA were added in the reaction mixture as a template for the PCR amplification. The temperature profile was the following: initial DNA denaturation for 5 min at 95 °C, followed by 35 cycles of denaturation for P_{adhE} or 25 cycles of denaturation for *kan* gene at 95 °C for 30 sec, annealing at 55 °C for 30 sec, elongation at 72 °C for 20 sec for Ptac promoter and 50 sec for *kan* gene; and the final elongation for 5 min at 72 °C. Then, the amplified DNA fragments were purified by agarose gel-electrophoresis, extracted using "GenElute Spin Columns" ("Sigma", USA) and precipitated by ethanol.

Each of the two above-described DNA fragments was treated with *EcoR*I restrictase and ligated. The ligation product was amplified by PCR using primers P21 and P22. The amplified *kan*-P_{adhE} DNA fragment was purified by agarose gel-electrophoresis, extracted using "GenElute Spin Columns" ("Sigma", USA) and precipitated by ethanol. The obtained DNA fragment was used for electroporation and Red-mediated integration into the bacterial chromosome of the *E. coli* MG1655Δtdh::rhtA*, P_{L-tac}adhE*/pKD46.

MG1655Atdh::rhtA*,P_{L-tac}dhE*/pKD46 cells were grown overnight at 30 °C in the liquid LB-medium with addition of ampicillin (100 µg/ml), then diluted 1:100 by the SOB-medium (Yeast extract, 5 g/l; NaCl, 0.5 g/l; Tryptone, 20 g/l; KCl, 2.5 mM; MgCl₂, 10 mM) with addition of ampicillin (100 µg/ml) and L-arabinose (10 mM) (arabinose was used for inducing the plasmid encoding genes of Red system) and grown at 30 °C to reach the optical density of the bacterial culture OD₆₀₀=0,4-0.7. The grown cells from 10 ml of the bacterial culture were washed 3 times by the ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions.

Shocked cells were added to 1-ml of SOC medium (Sambrook et al, "Molecular Cloning A Laboratory Manual, Second Edition", Cold Spring Harbor Laboratory Press (1989)), incubated for 2 hours at 37 °C, and then were spread onto L-agar containing 20 µg/ml of kanamycin.

Colonies grown within 24 h were tested for the presence of P_{adhE} -Km^{R} marker instead of P_{L-tac}-Cm^{R}-marker by PCR using primers P24 (SEQ ID NO: 27) and P25 (SEQ ID NO: 28). For this purpose, a freshly isolated colony was suspended in 20µl water and then 1 µl of obtained suspension was used for PCR. The temperature profile follows: initial DNA denaturation for 5 min at 95 °C; then 30 cycles of denaturation at 95 °C for 30 sec, annealing at 54 °C for 30 sec and elongation at 72 °C for 1.0 min; the final elongation for 5 min at 72 °C. A few Km^{R} colonies tested contained the desired 1200 bp DNA fragment, confirming the presence of native P_{adhE} promoter and Km^{R} -marker DNA. Some of these fragments were sequenced. The structure of the native P_{adhE} promoter was confirmed. One of the strains containing the mutant *adhE* gene under the control of anative promoter was cured of the thermosensitive plasmid pKD46 by culturing at 37 °C and the resulting strain was named as *E*. *coli* MG1655Δtdh, rhtA*, adhE*.

The ability of all the obtained strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L}-_{tac}adhE-Lys568, Val566 (cl.1); MG1655Δtdh, rhtA*, adhE* and parental strain MG1655Δtdh, rhtA* to grow on the minimal medium M9 containing ethanol as a sole carbon source was investigated. It was shown that the parental strain MG1655Δtdh, rhtA* and the strain with enhanced expression of wild-type alcohol dehydrogenase were unable to grow on the medium containing ethanol (2% or 3%) as a sole carbon source (Figure 3, A and B). Strain MG1655Δtdh, rhtA*, P_{L-tac}adhE-Lys568 (cl.18) containing the single mutation in the alcohol dehydrogenase described early (Membrillo-Hernandez, J. et al, J. Biol. Chem. 275, 33869-33875 (2000)) exhibited very poor growth in the same medium. But strains containing mutations in the alcohol dehydrogenase in addition to mutation Glu568Lys exhibited good growth (Figure 3, A and B). All the above strains were able to grow on the minimal medium M9 containing a mixture of glucose and ethanol, but strains with enhanced expression of the mutant alcohol dehydrogenase containing mutations in addition to mutation Glu568Lys exhibited better growth (Figure 4).

It was also shown that strain MG1655Δtdh, rhtA*, adhE* containing the alcohol dehydrogenase with 5 mutations under the control of the native promoter was unable to grow on the minimal medium M9 containing ethanol (2% or 3%) as a sole carbon source. Enhanced expression of the gene encoding for said alcohol dehydrogenase is necessary for good growth (Figure 5).

### Example 10. The effect of increasing the mutant adhE gene expression on L-threonine production

To evaluate the effect of enhancing expression of the mutant *adhE* gene on threonine production, *E. coli* strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566 (cl.1); MG1655Δtdh, rhtA*, adhE* and parental strain MG1655Δtdh, rhtA* were transformed with plasmid pVIC40.

These strains and the parent strain MG1655Δtdh, rhtA* (pVIC40) were cultivated at 37 °C for 18 hours in a nutrient broth and 0.3 ml of each of the obtained cultures was inoculated into 3 ml of fermentation medium (see Example 4) in a 20x200 mm test tube and cultivated at 34 °C for 48 hours with a rotary shaker. Data from at least 10 independent experiments are shown on Tables 1 and 2.

It can be seen from the Tables 1 and 2, mutant alcohol dehydrogenase was able to cause accumulation of a higher amount of L-threonine as compared with MG1655Δtdh, rhtA* in which neither expression of a wild-type nor a mutant alcohol dehydrogenase was increased or even with MG1655Δtdh, rhtA*, or P_{L-tac}adhE, in which expression of wild-type alcohol dehydrogenase was increased. Such higher accumulation of L-threonine during fermentation was observed in the medium containing either a mixture of glucose and ethanol, or just ethanol as the sole carbon source.

**Table 1**

| Strain | 3% ethanol | | 2% ethanol | |
|---|---|---|---|---|
| | OD₅₄₀ | Thr, g/l | OD₅₄₀ | Thr, g/l |
| MG1655Δtdh,rhtA* (pVIC40) | 1.6±0.1 | <0.1 | 1.4±0.1 | <0.1 |
| MG1655Δtdh, rhtA*,P_{L-tac}adhE(wt) (pVIC40) | 7.9±0.3 | 1.1±0.1 | 7.6±0.2 | 0.9±0.1 |
| MG1655Δtdh, rhtA*, P_{L-tac}adhE-Lys568(pVIC40)(cl.18) | 14.7±0.3 | 3.3±0.1 | 13.7±0.4 | 2.3±0.3 |
| MG1655Δtdh, rhtA*, P_{L-tac}adhE-Lys568, Val566(pVIC40) (cl.1) | 14.2±0.4 | 3.2±0.2 | 12.5±0.3 | 2.1±0.3 |
| MG1655Δtdh, rhtA*, P_{L-tac}adhE*(pVIC40) | 17.0±0.3 | 3.9±0.2 | 14.3±0.3 | 2.8±0.1 |
| MG1655Δtdh, rhtA*, adhE*(pVIC40) | 2.8±0.2 | <0.1 | 2.1±0.1 | < 0.1 |

**Table 2**

| Strain | 2,7% ethanol+0,3% glucose | |
|---|---|---|
| | OD₅₄₀ | Thr, g/l |
| MG1655Δtdh, rhtA* (pVIC40) | 6.6±0.2 | 0.9±0.2 |
| MG1655Δtdh, rhtA*,P_{L-tac}adhE(wt) (pVIC40) | 13.4±0.3 | 1.4±0.3 |
| MG1655Δtdh, rhtA*, P_{L-tac}adhE-Lys568 (pVIC40) (cl.18) | 16.1±0.4 | 2.6±0.2 |
| MG1655Δtdh, rhtA*, P_{L-tac}adhE-Lys568, Val566(pVIC40) (cl.1) | 15.5±0.3 | 2.9 ±0.2 |
| MG1655Δtdh, rhtA*, P_{L-tac}adhE*(pVIC40) | 18.8±0.4 | 2.8±0.1 |
| MG1655Δtdh, rhtA*, adhE*(pVIC40) | 5.8±0.1 | 0.8±0.3 |

Test-tube fermentation was carried out without reversion of evaporated ethanol.

### Example 11. The effect of increasing adhE gene expression on L-lysine production

To test the effect of enhanced expression of the *adhE* gene under the control of P_{L-tac} promoter on lysine production, the DNA fragments from the chromosome of the above-described strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566 (cl.1); MG1655Δtdh, rhtA*, adhE* were transferred to the lysine-producing *E*. *coli* strain WC1960ΔcadAΔldc (pCABD2) by P1 transduction (Miller, J.H. (1972) Experiments in Molecular Genetics, Cold Spring Harbor Lab. Press, Plainview, NY). pCABD2 is a plasmid comprising the *dapA* gene coding for a dihydrodipicolinate synthase having a mutation which desensitizes feedback inhibition by L-lysine, the *lysC* gene coding for aspartokinase III having a mutation which desensitizes feedback inhibition by L-lysine, the *dapB* gene coding for a dihydrodipicolinate reductase, and the *ddh* gene coding for diaminopimelate dehydrogenase (U.S. Patent No. 6,040,160).

The resulting strains and the parent strain WC196ΔcadAΔldc (pCABD2) were spread on L-medium plates containing 20 mg/l of streptomycin at 37 °C, and cells corresponding to 1/8 of a plate were inoculated into 20 ml of the fermentation medium containing the required drugs in a 500 ml-flask. The cultivation can be carried out at 37 °C for 48 hours by using a reciprocal shaker at the agitation speed of 115 rpm. After the cultivation, the amounts of L-lysine and residual ethanol in the medium can be measured by a known method (Bio-Sensor BF-5, manufactured by Oji Scientific Instruments). Then, the yield of L-lysine relative to consumed ethanol can be calculated for each of the strains.

The composition of the fermentation medium (g/l) was as follows:

| | |
|---|---|
| Ethanol | 20.0 |
| (NH₄)₂SO₄ | 24.0 |
| K₂HPO₄ | 1.0 |
| MgSO₄·7H₂O | 1.0 |
| FeSO₄·7H₂O | 0.01 |
| MnSO₄·5H₂O | 0.01 |
| Yeast extract | 2.0 |

pH is adjusted to 7.0 by KOH and the medium was autoclaved at 115°C for 10 min. Ethanol and MgSO₄ 7H₂O were sterilized separately. CaCO₃ was dry-heat sterilized at 180°C for 2 hours and added to the medium at a final concentration of 30 g/l. Data from two parallel experiments are shown on Table 3.

**Table 3**

| Strain | 2% ethanol | |
|---|---|---|
| | OD₆₀₀ | Lys, g/l |
| WC196ΔcadAΔldc (pCABD2) | 1.1±0.0 | 0.2±0.0 |
| WC196ΔcadAΔldc,P_{L-tac}adhE(wt) (pCABD2) | 1.5±0.1 | 0.4±0.1 |
| MG1655ΔcadAΔldc, P_{L-tac}adhE-Lys568 (pCABD2) (cl.18) | 5.2±0.4 | 1.3±0.1 |
| MG1655ΔcadAΔldc, P_{L-tac}adhE-Lys568, Val566(pCABD2) (cl.1) | 1.6±0.0 | 0.8 ±0.3 |
| MG1655ΔcadAΔldc, P_{L-tac}adhE*(pCABD2) | 5.9±0.2 | 1.8±0.1 |

It can be seen from Table 3 that mutant alcohol dehydrogenases and a wild-type alcohol dehydrogenase was able to cause growth enhancement and accumulation of a higher amount of L-lysine as compared with WC 196ΔcadAΔldc (pCABD2), in which neither expression of a wild-type nor a mutant alcohol dehydrogenase was increased.

### Example 12. Construction of E. coli MG1655ΔargR,P_{L-tac}adhE*

### 1. Construction of the strain MG1655ΔargR

This strain was constructed by inactivation of the native *argR* gene, which encodes a repressor of the L-arginine biosynthetic pathway in *E. coli* MG1655 by the *kan* gene. To replace the native *argR* gene, the DNA fragment carrying a kanamycin resistance marker (Km^{R}) encoded by the *kan* gene was integrated into the chromosome of *E*. *coli* MG1655 (ATCC 700926) in place of the native *argR* gene by the Red-driven integration.

A DNA fragment containing a Km^{R} marker encoded by the *kan* gene was obtained by PCR using the commercially available plasmid pACYC177 (GenBank/EMBL accession number X06402, "Fermentas", Lithuania) as a template, and primers P26 (SEQ ID NO: 31) and P27 (SEQ ID NO: 32). Primer P26 contains 40 nucleotides homologous to the 5'-region of the *argR* gene introduced into the primer for further integration into the bacterial chromosome. Primer P27 contains 41 nucleotides homologous to the 3'-region of the *argR* gene introduced into the primer for further integration into the bacterial chromosome. The temperature profile was the following: initial DNA denaturation for 5 min at 95 °C, followed by 25 cycles of denaturation at 95 °C for 30 sec, annealing at 55 °C for 30 sec, elongation at 72 °C for 40 sec; and the final elongation for 5 min at 72 °C. Then, the amplified DNA fragment was purified by agarose gel-electrophoresis, extracted using "GenElute Spin Columns" ("Sigma", USA) and precipitated by ethanol.

The obtained DNA fragment was used for electroporation and Red-mediated integration into the bacterial chromosome of the *E*. *coli* MG1655/pKD46.

MG1655/pKD46 cells were grown overnight at 30 °C in the liquid LB-medium with addition of ampicillin (100µg/ml), then diluted 1:100 by the SOB-medium (Yeast extract, 5 g/l; NaCl, 0.5 g/l; Tryptone, 20 g/l; KCI, 2.5 mM; MgCl₂, 10 mM) with the addition of ampicillin (100 µg/ml) and L-arabinose (10 mM) (arabinose is used for inducing the plasmid encoding genes of Red system) and grown at 30 °C to reach the optical density of the bacterial culture OD₆₀₀=0,4-0.7. The grown cells from 10 ml of the bacterial culture were washed 3 times by the ice-cold de-ionized water, followed by suspending in 100 µl of the water. 10 µl of DNA fragment (100 ng) dissolved in the de-ionized water was added to the cell suspension. The electroporation was performed by "Bio-Rad" electroporator (USA) (No. 165-2098, version 2-89) according to the manufacturer's instructions. Shocked cells were added to 1-ml of SOC medium, incubated 2 hours at 37°C, and then were spread onto L-agar containing 25 µg/ml of chloramphenicol. Colonies grown within 24 h were tested for the presence of Km^{R} marker instead of the native *argR* gene by PCR using primers P28 (SEQ ID NO: 33) and P29 (SEQ ID NO: 34). For this purpose, a freshly isolated colony was suspended in 20µl water and then 1µl of obtained suspension was used for PCR. The temperature profile was the following: initial DNA denaturation for 5 min at 95 °C; then 30 cycles of denaturation at 95 °c for 30 sec, annealing at 55 °C for 30 sec and elongation at 72 °C for 30 sec; the final elongation for 5 min at 72 °C. A few Km^{R} colonies tested contained the desired 1110 bp DNA fragment, confirming the presence of Km^{R} marker DNA instead of 660 bp fragment of *argR* gene. One of the obtained strains was cured from the thermosensitive plasmid pKD46 by culturing at 37 °C and the resulting strain was named *E. coli* MG1655ΔargR.

### 2. Construction of E. coli MG1655ΔargR,P_{L-tac}adhE*.

*E. coli* MG16550argR,P_{L-tac}adhE* was obtained by transduction of the P_{L-tac} adhE* mutation from the strain MG1655::P_{L-tac}adhE*.

The strain MG1655ΔargR was infected with phage P1ᵥᵢᵣ grown on the donor strain MG1655::P_{L-tac}adhE* and the strain MG1655ΔargR,P_{L-tac}adhE* was obtained. This strain was checked for growth on M9 plates with 2% ethanol as a sole carbon source. The growth rate was the same as for the strain MG1655::P_{L-tac}adhE* (36 h).

### Example 13. Construction of the pMW119-ArgA4 plasmid

*ArgA* gene with a single mutation provide the fbr (feedback resistant) phenotype (JP2002253268, EP1170361) and under the control of its own promoter was cloned into pMW119 vector.

The *argA* gene was obtained by PCR using the plasmid pKKArgA-r4 (JP2002253268, EP1170361) as a template, and primers P30 (SEQ ID NO: 35) and P31 (SEQ ID NO: 36). Sequence of the primer P30 homologous to the 5'-region of the *argA* gene located 20 bp upstream and 19 bp downstream of the start codon of the *argA* gene. Primer P31 contains 24 nucleotides homologous to the 3'-region of *argA* gene and *HindIII* restriction site introduced for further cloning into the pMW 119/*BamHI-HindIII* vector.

Sequence of the P_{argA} promoter was obtained by PCR using *E. coli* MG1655 as a template, and primers P32 (SEQ ID NO: 37) and P33 (SEQ ID NO: 38). Primer P32 contains 30 nucleotides homologous to the 5'-untranslated region of the *argA* gene located 245 bp upstream of the start codon, and moreover this sequence includes *BamHI* recognition site. Primer P33 contains 24 nucleotides homologous to the 5'-region of the *argA* gene located 20 bp upstream of the start codon and start codon itself. The temperature profile was the following: initial DNA denaturation for 5 min at 95 °C, followed by 25 cycles of denaturation at 95 °C for 30 sec, annealing at 54 °C for 30 sec, elongation at 72 °C for 1 min 20 sec (for *ArgA* gene) or 20 sec(for P_{argA} promoter) and the final elongation for 5 min at 72 °C. Then, the amplified DNA fragments was purified by agarose gel-electrophoresis, extracted using "GenElute Spin Columns" ("Sigma", USA) and precipitated by ethanol.

P_{argA}*ArgA* fragment was obtained by PCR using both the above-described DNA fragments: P_{argA} promoter and *ArgA* gene. First, the reaction mixture (total volume - 100 µl) consisted of 10 µl of 10x PCR-buffer with 25 mM MgCl₂ (Sigma, USA), 200 µM each of dNTP and 1 U of Accu-Taq DNA polymerase (Sigma, USA). The *argA* fragment (25 ng) and P_{argA} (5 ng) were used as a template DNA and as primers simultaneously. Next, primers P31 and P32 were added in reaction mixture. The temperature profile was the following: 1 st step - initial DNA denaturation for 5 min at 95 °C, followed by 10 cycles of denaturation at 95 °C for 30 sec, annealing at 53 °C for 30 sec, elongation at 72 °C for 1 min, 2nd step - 15 cycles of denaturation at 95 °C, annealing at 54 °C for 30 sec, elongation at 72 °C for 1 min 30 sec. The amplified DNA fragments was purified by agarose gel-electrophoresis, extracted using "GenElute Spin Columns" ("Sigma", USA), precipitated by ethanol, treated with *BamHI* and *HindIII* and ligated with pMW 119/ *BamHI- HindIII* vector. As a result the plasmid pMW 119-ArgA4 was obtained.

### Example 14. The effect of increasing adhE gene expression on L-arginine production

To evaluate the effect of enhancing expression of the mutant *adhE* gene on L-arginine production, *E. coli* strains MG1655ΔargR P_{L-tac}adhE* and MG1655ΔargR were each transformed by plasmid pMW 119- ArgA4. 10 obtained colonies of each sort of transformants were cultivated at 37°C for 18hours in a nutrient broth supplemented with 150 mg/l of Ap and 0.1 ml of each of the obtained cultures was inoculated into 2 ml of fermentation medium in a 20x200 mm test tube and cultivated at 32°C for 96 hours with a rotary shaker. After cultivation, the amount of L-arginine which accumulates in the medium was determined by paper chromatography using the following mobile phase: butanol : acetic acid : water = 4 : 1 : 1 (v/v). A solution (2%) of ninhydrin in acetone was used as a visualizing reagent. A spot containing L-arginine was cut out, L-arginine was eluted in 0.5 % water solution of CdCl₂, and the amount of L-arginine was estimated spectrophotometrically at 540 nm. The results of ten independent test tube fermentations are shown in Table 4. As follows from Table 4, MG1655ΔargR P_{L- tac}adhE* produced a higher amount of L-arginine, as compared with MG1655ΔargR P_{L-tac}adhE*, both in medium with supplemented glucose and without it.

The composition of the fermentation medium was as follows (g/l):

| | |
|---|---|
| Ethanol | 20 |
| Glucose | 0 / 5 |
| (NH₄)₂SO₄ | 25 |
| K₂HPO₄ | 2 |
| MgSO₄·7H₂O | 1.0 |
| Thiamine hydrochloride | 0.002 |
| Yeast extract | 5.0 |
| CaCO₃ | 33 |

| | |
|---|---|
| MgSO₄·7H₂O, ethanol and CaCO₃ were each sterilized separately. | |

**Table 4**

| Strain | Ethanol (2%) | | Ethanol (2%) and glucose (5%) | |
|---|---|---|---|---|
| | OD₅₅₀ | Amount of L-arginine, g/l | OD₅₅₀ | Amount of L-arginine, g/l |
| MG1655ΔargR(pMW-argAm4) | 1.3±0,2 | <0.1 | 8.0±0.4 | 1.5±0.2 |
| MG 1655ΔargRcat-P_{L-tac}-adhE* (pMW-argAm4) | 7.2±0.4 | 0.8±0.3 | 13.4±0.3 | 1.9±0.2 |

### Example 15. Construction of the L-leucine producing E. coli strain NS1391

The strain NS1391 was obtained as follows.

At first, a strain having inactivated acetolactate synthase genes (combination *of AilvIH* and *ΔilvGM* deletions) was constructed. The *ilvIH* genes (ΔilvIH::cat) were deleted from the wild-type strain *E. coli K12* (VKPM B-7) by P1 transduction (Sambrook et al, "Molecular Cloning A Laboratory Manual, Second Edition", Cold Spring Harbor Laboratory Press (1989). *E. coli* MG1655 ΔilvIH::cat was used as a donor strain. Deletion of the *ilvIH* operon in the strain MG1655 was conducted by means of the Red-driven integration. According to this procedure, the PCR primers P34 (SEQ ID NO: 39) and P35 (SEQ ID NO: 40) homologous to the both region adjacent to the *ilvIH* operon and gene conferring chloramphenicol resistance in the template plasmid were constructed. The plasmid pMW-attL-Cm-attR (PCT application WO 05/010175) was used as a template in a PCR reaction. Conditions for PCR were following: denaturation step for 3 min at 95 °C; profile for two first cycles: 1 min at 95 °C, 30 sec at 34 °C, 40 sec at 72 °C; profile for the last 30 cycles: 30 sec at 95 °C, 30 sec at 50 °C, 40 sec at 72 °C; final step: 5 min at 72 °C. Obtained 1713 bp PCR product was purified in agarose gel and used for electroporation of *E. coli* MG1655/pKD46. Chloramphenicol resistant recombinants were selected after electroporation and verified by means of PCR with locus-specific primers P36 (SEQ ID NO: 41) and P37 (SEQ ID NO: 42). Conditions for PCR verification were the following: denaturation step for 3 min at 94 °C; profile for the 30 cycles: 30 sec at 94 °C, 30 sec at 53 °C, 1 min 20 sec at 72 °C; final step: 7 min at 72 °C. PCR product, obtained in the reaction with the chromosomal DNA from parental IlvIH⁺ strain MG1655 as a template, was 2491 nt in length. PCR product, obtained in the reaction with the chromosomal DNA from mutant MG1655 ΔilvIH::cat strain as a template, was 1823 nt in length. As a result the strain MG1655 ΔilvIH::cat was obtained. After deletion of *ilvIH* genes (ΔilvIH::cat) from *E. coli K12* (VKPM B-7) by P1 transduction, Cm^{R} transductants were selected. As a result the strain B-7 ΔilvIH::cat was obtained. To eliminate the chloramphenicol resistance marker from B-7 ΔilvIH::cat, cells were transformed with the plasmid pMW118-int-xis (Ap^{R}) (W02005/010175). Ap^{R} clones were grown on LB agar plates containing 150 mg/l ampicillin at 30°C. Several tens of Ap^{R} clones were picked up and tested for chloramphenicol sensitivity. The plasmid pMW118-int-xis was eliminated from Cm^{S} cells by incubation on LB agar plates at 42 °C. As a result, the strain B-7 ΔilvIH was obtained.

The *ilvGM* genes (ΔilvGM::cat) were deleted from*E* .*coli* B-7 ΔilvIH by P1 transduction. E. *coli* MG1655 ΔilvGM::cat was used as a donor strain. The *ilvGM* operon was deleted from the strain MG1655 by Red-driven integration. According to this procedure, the PCR primers P38 (SEQ ID NO: 43) and P39 (SEQ ID NO: 44) homologous to both the region adjacent to the *ilvGM* operon and the gene conferring chloramphenicol resistance in the template plasmid were constructed. The plasmid pMW-attL-Cm-attR (PCT application WO 05/010175) was used as a template in the PCR reaction. Conditions for PCR were the following: denaturation step for 3 min at 95 °C; profile for two first cycles: 1 min at 95 °C, 30 sec at 34 °C, 40 sec at 72 °C; profile for the last 30 cycles: 30 sec at 95 °C, 30 sec at 50 °C, 40 sec at 72 °C; final step: 5 min at 72 °C.

The obtained 1713 bp PCR product was purified in agarose gel and used for electroporation of E. *coli* MG1655/pKD46. Chloramphenicol resistant recombinants were selected after electroporation and verified by means of PCR with locus-specific primers P40 (SEQ ID NO: 45) and P41 (SEQ ID NO: 46). Conditions for PCR verification were the following: denaturation step for 3 min at 94 °C; profile for the 30 cycles: 30 sec at 94 °C, 30 sec at 54 °C, 1 min 30 sec at 72 °C; final step: 7 min at 72 °C. PCR product, obtained in the reaction with the chromosomal DNA from the parental strain MG1655 as a template, was 2209 nt in length. The PCR product, obtained in the reaction with the chromosomal DNA from mutant MG1655 ΔilvGm::cat strain as a template, was 1941 nt in length. As a result, the strain MG1655 ΔilvGm::cat was obtained. After deletion of *ilvGM* genes (ΔilvGm::cat) from *E.coli* B-7 ΔilvIH by P1 transduction, CmR transductants were selected. As a result the strain B-7 ΔilvIH ΔilvBN ΔilvGM::cat was obtained. The chloramphenicol resistance marker was eliminated from B-7 ΔilvIH ΔilvBN ΔilvGM::cat as described above. As a result, the strain B-7 ΔilvIH ΔilvGM was obtained.

The native regulator region of the *ilvBN* operon was replaced with the phage lambda P_{L} promoter by the Red-driven integration. For that purpose, the strain B7 ΔilvIH ΔilvGM with the sole AHAS I was used as an initial strain for such modification. According to the procedure of Red-driven integration, the PCR primers P42(SEQ ID NO: 47) and P43 (SEQ ID NO:48) were constructed. Oligonucleotide P42 (SEQ ID NO: 47) was homologous to the region upstream of the *ilvB* gene and the region adjacent to the gene conferring antibiotic resistance which was present in the chromosomal DNA of BW25113 cat-P_{L}-yddG. Oligonucleotide P43 (SEQ ID NO: 48) was homologous to both the *ilvB* region and the region downstream from the P_{L} promoter which was present in the chromosome of BW25113 cat-P_{L}-yddG. Obtaining BW25113 cat-P_{L}-yddG has been described in detail previously (EP1449918A1, Russian patent RU2222596). The chromosomal DNA of strain BW25113 cat-P_{L}-yddG was used as a template for PCR. Conditions for PCR were the following: denaturation for 3 min at 95 °C; profile for two first cycles: 1 min at 95 °C, 30 sec at 34 °C, 40 sec at 72 °C; profile for the last 30 cycles: 30 sec at 95 °C, 30 sec at 50 °C, 40 sec at 72 °C; final step: 5 min at 72 °C. As a result, the PCR product was obtained (SEQ ID NO: 49), purified in agarose gel, and used for electroporation of *E. coli* B-7 ΔilvIH ΔilvGM, which contains the plasmid pKD46 with temperature sensitive replication. Electrocompetent cells were prepared as follows: *E. coli* strain B-7 ΔilvIH ΔilvGM was grown overnight at 30 °C in LB medium containing ampicillin (100 mg/l), and the culture was diluted 100 times with 5 ml of SOB medium (Sambrook et al, "Molecular Cloning A Laboratory Manual, Second Edition", Cold Spring Harbor Laboratory Press (1989)) with ampicillin and L-arabinose (1 mM). The cells were grown with aeration at 30 °C to an OD₆₀₀ of ≈0.6 and then made electrocompetent by concentrating 100-fold and washing three times with ice-cold deionized H₂O. Electroporation was performed using 70 µl of cells and ≈100 ng of PCR product. Following electroporation, the cells were incubated with 1 ml of SOC medium (Sambrook et al, "Molecular Cloning A Laboratory Manual, Second Edition", Cold Spring Harbor Laboratory Press (1989)) at 37 °C for 2.5 h and after that plated onto L-agar and were grown at 37 °C to select Cm^{R} recombinants. Then, to eliminate the pKD46 plasmid, 2 passages on L-agar with Cm at 42 °C were performed and the obtained colonies were tested for sensitivity to ampicillin.

The obtained strain B7 ΔilvIH ΔilvGM cat-P_{L}-ilvBN was valine sensitive. New valine resistant spontaneous mutants of AHAS I were obtained from this strain. Strains which grew better on 1 g/l of valine were characterized.

Valine resistance mutations which were resistance to isoleucine were obtained, as well. Variants with a specific activity which was more than that of the wild-type were obtained. The nucleotide sequence of the mutant operons for mutant ilvBN4 was determined. It was revealed that IlvBN4 contained one point mutation in IlvN: N17K Asn-Lys (codon aac was replaced with aag). Obtained strain B7 ΔilvIH ΔilvGM cat-P_{L}-ilvBN4 was used for the following constructions.

Then, cat-P_{L}-ilvBN4 DNA fragment was transferred from *E .coli* B7 ΔilvIH ΔilvGM cat-P_{L}-ilvBN4 into *E .coli* MG1655 mini-Mu::scrKYABR (EP application 1149911) by P1 transduction. As a result the strain ESP214 was obtained. The chloramphenicol resistance marker was eliminated from the strain ESP214 as described above. As a result, the strain ESP215 was obtained.

Then the DNA fragment shown in (SEQ ID NO: 50) was used for electroporation of the strain ESP215/pKD46 for the purpose of subsequent integration into chromosome. This DNA fragment contained regions complementary to the 3' region of the gene *b1701* and to the 5' region of the gene *b1703* (these genes are adjacent to the gene *pps),* which are necessary for integration into the chromosome. It also contained an excisable chloramphenicol resistance marker *cat,* and a mutant *ilvBN4* operon under the control of the constitutive promoter P_{L}. Electroporation was performed as described above. Selected Cm^{R} recombinants contained a deletion of the gene *pps* as a result of the integration of *cat-P_{L}-ilvBN4* fragment into the chromosome. Thus the strain ESP216 was obtained. The chloramphenicol resistance marker was eliminated from the strain ESP216 as described above. As a result, the strain ESP217 was obtained.

At the next step, the mutant *leuA* gene (Gly479 -> Cys) under the control of the constitutive promoter P_{L} was introduced into the strain ESP217. The DNA fragment shown in (SEQ ID NO: 51) was used for electroporation of the strain ESP217/pKD46 for the purpose of subsequent integration into the chromosome. This DNA fragment contained the 35nt-region, which is necessary for integration into the chromosome and homologous to the upstream region of the gene *leuA.* It also contained an excisable region complementary to the sequence of chloramphenicol resistance marker *cat,* and the mutant *leuA* (Gly479 -> Cys) gene under the control of the constitutive promoter P_{L}. Electroporation was performed as described above. Selected Cm^{R} recombinants contained the mutant gene *leuA* (Gly479 -> Cys) under the control of the constitutive promoter P_{L} integrated into the chromosome. Thus, the strain ESP220 was obtained. The chloramphenicol resistance marker was eliminated from the strain ESP220 as described above. As a result, the strain ESP221 was obtained.

Then, the DNA fragment shown in SEQ ID NO: 52 was used for electroporation of the strain ESP221/pKD46 for the purpose of subsequent integration into the chromosome. This DNA fragment contained the 35nt-region homologous to the upstream region of the gene *tyrB,* which is necessary for integration into the chromosome. It also contained an excisable region complementary to the sequence of chloramphenicol resistance marker *cat* and the gene *tyrB* with a modified regulatory(-35) region. Electroporation was performed as described above. Selected Cm^{R} recombinants contained the gene *tyrB* with the modified regulatory(-35) region. Thus, the strain NS1390 was obtained. The chloramphenicol resistance marker was eliminated from the strain NS1390 as described above. As a result, the strain NS 1391 was obtained. Leucine producing strain NS1391 was used for further work.

### Example 16. The effect of increasing the mutant adhE gene expression on L-leucine production

To test the effect of enhanced expression of the *adhE* gene under the control of a P_{L-tac} promoter on L-leucine production, DNA fragments from the chromosome of the above-described strain MG1655 P_{L-tac}adhE* were transferred to the L-leucine producing *E. coli* strain NS1391 by P1 transduction (Miller, J.H. (1972) Experiments in Molecular Genetics, Cold Spring Harbor Lab. Press, Plainview, NY) to obtain the strain NS 1391 P_{L-tac}adhE*.

Both *E. coli* strains, NS1391 and NS1391 P_{L-tac}adhE*, were cultured for 18-24 hours at 37°C on L-agar plates. To obtain a seed culture, the strains were grown on a rotary shaker (250 rpm) at 32°C for 18 hours in 20x200-mm test tubes containing 2 ml of L-broth supplemented with 4% sucrose. Then, the fermentation medium was inoculated with 0.21 ml of seed material (10%). The fermentation was performed in 2 ml of a minimal fermentation medium in 20x200-mm test tubes. Cells were grown for 48-72 hours at 32°C with shaking at 250 rpm. The amount of L-leucine was measured by paper chromatography (liquid phase composition: butanol - acetic acid - water = 4:1:1). The results of ten independent test tube fermentations are shown in Table 5. As follows from Table 5, NS1391 P_{L-tac}adhE* produced a higher amount of L-leucine, as compared with NS1391, in media containing different concentrations of ethanol.

The composition of the fermentation medium (g/l) (pH 7.2) was as follows:

| | |
|---|---|
| Glucose | 60.0 |
| Ethanol | 0/10.0/20.0/30.0 |
| (NH₄)₂SO₄ | 25.0 |
| K₂HPO₄ | 2.0 |
| MgSO₄·7H₂O | 1.0 |
| Thiamine | 0.01 |
| CaCO₃ | 25.0 |

| | |
|---|---|
| Glucose, ethanol and CaCO₃ were sterilized separately. | |

**Table 5**

| Strain | Glucose (6%) | | | | | | | |
|---|---|---|---|---|---|---|---|---|
| | without ethanol | | +1% ethanol | | +2% ethanol | | +3% ethanol | |
| | Leu,g/l | OD₅₅₀ | Leu,g/l | OD₅₅₀ | Leu,g/l | OD₅₅₀ | Leu,g/l | OD₅₅₀ |
| NS1391 | 5.0±0.1 | 34.2±0.6 | 4.8±0.1 | 31.7±0.4 | 3.9±0.2 | 31.3±0.6 | 4.0±0.1 | 28.0±0.6 |
| NS1391 P_{L- tac}-adhE* | 5.1±0.1 | 31.1±0.2 | 5.9±0.1 | 29.3±0.3 | 4.9±0.1 | 27.3±0.6 | 4.6±0.1 | 22.8±0.2 |

### Example 17. The effect of the increasing the adhE gene expression on L-phenylalanine production

To test the effect of enhanced expression of the *adhE* gene under the control of a P_{L-tac} promoter on phenylalanine production, the DNA fragments from the chromosome of the above-described strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566 (cl.1); MG1655Δtdh, rhtA*, adhE* can be transferred to the phenylalanine-producing *E. coli* strain AJ12739 by P1 transduction (Miller, J.H. (1972) Experiments in Molecular Genetics, Cold Spring Harbor Lab. Press, Plainview, NY). The strain AJ12739 has been deposited in the Russian National Collection of Industrial Microorganisms (VKPM) (Russia, 117545 Moscow, 1 Dorozhny proezd, 1) on November 6, 2001 under accession number VKPM B-8197 and then converted to a deposit under the Budapest Treaty on August 23, 2002

The resulting strains and the parent strain AJ12739 can each be cultivated at 37 °C for 18 hours in a nutrient broth, and 0.3 ml of the obtained cultures can each be inoculated into 3 ml of a fermentation medium in a 20 x 200 mm test tube and cultivated at 37 °C for 48 hours with a rotary shaker. After cultivation, the amount of phenylalanine which accumulates in the medium can be determined by TLC. 10 x 15 cm TLC plates coated with 0.11 mm layers of Sorbfil silica gel without fluorescent indicator (Stock Company Sorbpolymer, Krasnodar, Russia) can be used. The Sorbfil plates can be developed with a mobile phase: propan-2-ol : ethylacetate : 25% aqueous ammonia : water = 40 : 40 : 7 : 16 (v/v). A solution (2%) of ninhydrin in acetone can be used as a visualizing reagent.

The composition of the fermentation medium (g/l):

| | |
|---|---|
| Ethanol | 20.0 |
| (NH₄)₂SO₄ | 16.0 |
| K₂HPO₄ | 0.1 |
| MgSO₄·7H₂O | 1.0 |
| FeSO₄·7H₂O | 0.01 |
| MnSO₄·5H₂O | 0.01 |
| Thiamine HCl | 0.0002 |
| Yeast extract | 2.0 |
| Tyrosine | 0.125 |
| CaCO₃ | 20.0 |

Ethanol and magnesium sulfate are sterilized separately. CaCO₃ dry-heat sterilized at 180 °C for 2 hours. pH is adjusted to 7.0.

### Example 18. The effect of increasing the adhE gene expression on L-tryptophan production

To test the effect of enhanced expression of the *adhE* gene under the control of a P_{L-tac} promoter on tryptophan production, the DNA fragments from the chromosome of the above-described strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566(cl.1); MG1655Δtdh, rhtA*, adhE* can be transferred to the tryptophan-producing *E. coli* strain SV164 (pGH5) by P1 transduction (Miller, J.H. (1972) Experiments in Molecular Genetics, Cold Spring Harbor Lab. Press, Plainview, NY). The strain SV164 has the *trpE* allele encoding anthranilate synthase which is not subject to feedback inhibition by tryptophan. The plasmid pGH5 harbors a mutant *serA* gene encoding phosphoglycerate dehydrogenase which is not subject to feedback inhibition by serine. The strain SV164 (pGH5) is described in detail in U.S. Patent No. 6,180,373 or European patent 0662143.

The resulting strains and the parent strain SV 164 (pGH5) can each be cultivated with shaking at 37 °C for 18 hours in 3 ml of nutrient broth supplemented with 20 mg/l of tetracycline (marker of pGH5 plasmid). 0.3 ml of the obtained cultures can each be inoculated into 3 ml of a fermentation medium containing tetracycline (20 mg/l) in 20 x 200 mm test tubes, and cultivated at 37 °C for 48 hours with a rotary shaker at 250 rpm. After cultivation, the amount of tryptophan which accumulates in the medium can be determined by TLC as described in Example 17. The fermentation medium components are set forth in Table 6, but should be sterilized in separate groups A, B, C, D, E, F, and H, as shown, to avoid adverse interactions during sterilization.

**Table 6**

| Solutions | Component | Final concentration, g/l |
|---|---|---|
| A | KH₂PO₄ | 1.5 |
| | NaCl | 0.5 |
| | (NH₄)₂SO₄ | 1.5 |
| | L-Methionine | 0.05 |
| | L-Phenylalanine | 0.1 |
| | L-Tyrosine | 0.1 |
| | Mameno (total N) | 0.07 |
| B | Ethanol | 20.0 |
| | MgSO₄·7H₂O | 0.3 |
| C | CaCl₂ | 0.011 |
| D | FeSO₄·7H₂O | 0.075 |
| | Sodium citrate | 1.0 |
| E | Na₂MoO₄·2H₂O | 0.00015 |
| | H₃BO₃ | 0.0025 |
| | CoCl₂·6H₂O | 0.00007 |
| | CuSO₄·5H₂O | 0.00025 |
| | MnCl₂·4H₂O | 0.0016 |
| | ZnSO₄·7H₂O | 0.0003 |
| F | Thiamine HCl | 0.005 |
| G | CaCO₃ | 30.0 |
| H | Pyridoxine | 0.03 |

| | | |
|---|---|---|
| Solution A had a pH of 7.1, adjusted by NH₄OH. | | |

### Example 19. The effect of the increasing the adhE gene expression on L-histidine production

To test the effect of enhanced expression of the *adhE* gene under the control of a P_{L-tac} promoter on histidine production, the DNA fragments from the chromosome of the above-described strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566(cl.1); MG1655Δtdh, rhtA*, adhE* can be transferred to the histidine-producing *E. coli* strain 80 by P1 transduction (Miller, J.H. (1972) Experiments in Molecular Genetics, Cold Spring Harbor Lab. Press, Plainview, NY). The strain 80 has been described in Russian patent 2119536 and deposited in the Russian National Collection of Industrial Microorganisms (Russia, 117545 Moscow, 1 Dorozhny proezd, 1) on October 15, 1999 under accession number VKPM B-7270 and then converted to a deposit under the Budapest Treaty on July 12, 2004.

The resulting strains and the parent strain 80 can each be cultivated in L broth for 6 hours at 29 °C. Then, 0.1 ml of obtained culture can each be inoculated into 2 ml of fermentation medium in a 20x200mm test tube and cultivated for 65 hours at 29 °C with a rotary shaker (350 rpm). After cultivation, the amount of histidine which accumulates in the medium can be determined by paper chromatography. The paper can be developed with a mobile phase: n-butanol : acetic acid : water = 4 : 1 : 1 (v/v). A solution of ninhydrin (0.5%) in acetone can be used as a visualizing reagent.

The composition of the fermentation medium (pH 6.0) (g/l):

| | |
|---|---|
| Ethanol | 20.0 |
| Mameno (soybean hydrolyzate) | 0.2 as total nitrogen |
| L-proline | 1.0 |
| (NH₄)₂SO₄ | 25.0 |
| KH₂PO₄ | 2.0 |
| MgSO₄·7H₂O | 1.0 |
| FeSO₄·7H₂O | 0.01 |
| MnSO₄ | 0.01 |
| Thiamine | 0.001 |
| Betaine | 2.0 |
| CaCO₃ | 60.0 |

| | |
|---|---|
| Ethanol, proline, betaine and CaCO₃ are sterilized separately. pH is adjusted to 6.0 before sterilization. | |

### Example 20. The effect of increasing the adhE gene expression on L-glutamic acid production

To test the effect of enhanced expression of the *adhE* gene under the control of a P_{L-tac} promoter on glutamic acid production, the DNA fragments from the chromosome of the above-described strains MG1655Δtdh, rhtA*, P_{L-tac}adhE; MG1655Δtdh, rhtA*, P_{L-tac}adhE*; MG1655Δtdh, rhtA*, P_{L-tac}adhE-Lys568 (cl.18); MG1655Δtdh, rhtA*, P_{L-tac}adhE-Lys568, Val566 (cl.1); MG1655Δtdh, rhtA*, adhE* can be transferred to the glutamic acid-producing *E. coli* strain VL334thrC⁺ (EP1172433) by P1 transduction (Miller, J.H. (1972) Experiments in Molecular Genetics, Cold Spring Harbor Lab. Press, Plainview, NY). The strain VL334thrC⁺ has been deposited in the Russian National Collection of Industrial Microorganisms (VKPM) (Russia, 117545 Moscow, 1 Dorozhny proezd, 1) on December 6, 2004 under the accession number VKPM B-8961 and then converted to a deposit under the Budapest Treaty on December 8, 2004.

The resulting strains and the parent strain VL334thrC⁺ can each be cultivated with shaking at 37 °C for 18 hours in 3 ml of nutrient broth. 0.3 ml of the obtained cultures can each be inoculated into 3 ml of a fermentation medium in 20 x 200 mm test tubes, and cultivated at 37 °C for 48 hours with a rotary shaker at 250 rpm.

The composition of the fermentation medium (pH 7.2) (g/l):

| | |
|---|---|
| Ethanol | 20.0 |
| Ammonium sulfate | 25.0 |
| KH₂PO₄ | 2.0 |
| MgSO₄·7H₂O | 1.0 |
| Thiamine | 0.0001 |
| L-isoleucine | 0.05 |
| CaCO₃ | 25.0 |

| | |
|---|---|
| Ethanol and CaCO₃ were sterilized separately. | |

### Industrial Applicability

According to the present invention, production of an L-amino acid by a bacterium of the *Enterobacteriaceae* family can be enhanced.

## Claims

1. A method for producing an L-amino acid by fermentation comprising:
A) cultivating in a culture medium containing ethanol an L-amino acid-producing bacterium of the *Enterobacteriaceae* family having an alcohol dehydrogenase, and
B) isolating the L-amino acid from the culture medium,
wherein the gene encoding said alcohol dehydrogenase is expressed under the control of a non-native promoter which functions under aerobic cultivation conditions.

2. The method according to claim 1, wherein said non-native promoter is selected from the group consisting of P_{tac}, P_{lac}, Pₜᵣₚ, P_{trc}, P_{R}, and P_{L}.

3. The method according to claim 1 or 2, wherein said alcohol dehydrogenase is resistant to aerobic inactivation.

4. The method according to any one of claims 1 to 3, wherein said alcohol dehydrogenase originates from a bacterium selected from the group consisting of *Escherichia coli, Erwinia carotovora, Salmonella typhimurium, Shigella flexneri, Yersinia pestis, Pantoea ananatis, Lactobacillus plantarum,* and *Lactococcus lactis.*

5. The method according to any one of claims 1 to 4, wherein said alcohol dehydrogenase comprises the amino acid sequence set forth in SEQ ID NO: 2, except the glutamic acid residue at position 568 is replaced with another amino acid residue other than an aspartic acid residue.

6. The method according to any one of claims 1 to 4, wherein said alcohol dehydrogenase comprises the amino acid sequence set forth in SEQ ID NO: 2, except the glutamic acid residue at position 568 is replaced with a lysine residue.

7. The method according to claim 5 or 6, wherein said alcohol dehydrogenase has at least one additional mutation which is able to improve the growth of said bacterium in a liquid medium which contains ethanol as the sole carbon source.

8. The method according to claim 7, wherein said additional mutation is selected from the group consisting of:
A) replacement of the glutamic acid residue at position 560 in SEQ ID NO: 2 with another amino acid residue;
B) replacement of the phenylalanine residue at position 566 in SEQ ID NO: 2 with another amino acid residue;
C) replacement of the glutamic acid residue, the methionine residue, the tyrosine residue, the isoleucine residue and the alanine residue at positions 22, 236, 461, 554, and 786, respectively, in SEQ ID NO: 2 with other amino acid residues; and
D) combinations thereof.

9. The method according to claim 7, wherein said additional mutation is selected from the group consisting of:
A) replacement of the glutamic acid residue at position 560 in SEQ ID NO: 2 with a lysine residue;
B) replacement of the phenylalanine residue at position 566 in SEQ ID NO: 2 with a valine residue;
C) replacement of the glutamic acid residue, the methionine residue, the tyrosine residue, the isoleucine residue and the alanine residue at positions 22, 236, 461, 554, and 786, respectively, in SEQ ID NO: 2 with a glycine residue, a valine residue, a cysteine residue, a serine residue, and a valine residue, respectively; and
D) combinations thereof.

10. The method according to any one of claims 1 to 9, wherein said L-amino acid-producing bacterium belongs to a genus selected from the group consisting of *Escherichia, Enterobacter, Erwinia, Klebsiella, Pantoea, Providencia, Salmonella, Serratia, Shigella,* and *Morganella.*

11. The method according to any one of claims 1 to 10, wherein said L-amino acid is selected from the group consisting of L-threonine, L-lysine, L-histidine, L-phenylalanine, L-arginine, L-tryptophan, L-glutamic acid, and L-leucine.

## Patentansprüche

1. Verfahren zur Herstellung einer L-Aminosäure durch Fermentation, umfassend:
A) Kultivieren eines L-Aminosäure-herstellenden Bakteriums der Enterobacteriaceae-Familie, das eine Alkoholdehydrogenase besitzt, in einem Kulturmedium, das Ethanol enthält, und
B) Isolieren der L-Aminosäure aus dem Kulturmedium,
wobei das Gen, das die Alkoholdehydrogenase kodiert, unter der Kontrolle eines nicht-nativen Promotors exprimiert ist, welcher unter aeroben Kultivierungsbedingungen funktioniert.

2. Verfahren gemäß Anspruch 1, wobei der nicht-native Promotor ausgewählt ist aus der Gruppe bestehend aus P_{tac}, P_{lac}, Pₜᵣₚ, P_{trc}, P_{R} und P_{L}.

3. Verfahren gemäß Anspruch 1 oder 2, wobei die Alkoholdehydrogenase gegenüber aerober Inaktivierung beständig ist.

4. Verfahren gemäß mindestens einem der Ansprüche 1 bis 3, wobei die Alkoholdehydrogenase einem Bakterium entstammt, welches ausgewählt ist aus der Gruppe bestehend aus Escherichia coli, Erwinia carotovora, Salmonella typhimurium, Shigella flexneri, Yersinia pestis, Pantoea ananatis, Lactobacillus plantarum und Lactococcus lactis.

5. Verfahren gemäß mindestens einem der Ansprüche 1 bis 4, wobei die Alkoholdehydrogenase die in SEQ ID NO: 2 dargelegte Aminosäuresequenz umfasst, bis auf dass der Glutaminsäure-Rest an Position 568 mit einem anderen Aminosäurerest ersetzt ist, der anders als ein Asparaginsäure-Rest ist.

6. Verfahren gemäß mindestens einem der Ansprüche 1 bis 4, wobei die Alkoholdehydrogenase die in SEQ ID NO: 2 dargelegte Aminosäuresequenz umfasst, bis auf dass der Glutaminsäure-Rest an Position 568 mit einem Lysin-Rest ersetzt ist.

7. Verfahren gemäß Anspruch 5 oder 6, wobei die Alkoholdehydrogenase wenigstens eine zusätzliche Mutation besitzt, die in der Lage ist, das Wachstum des Bakteriums in einem flüssigen Medium zu verbessern, das Ethanol als die einzige Kohlenstoffquelle enthält.

8. Verfahren gemäß Anspruch 7, wobei die zusätzliche Mutation ausgewählt ist aus der Gruppe bestehend aus:
A) Ersatz des Glutaminsäure-Rests an Position 560 in SEQ ID NO: 2 mit einem anderen Aminosäure-Rest;
B) Ersatz des Phenylalanin-Rests an Position 566 in SEQ ID NO: 2 mit einem anderen Aminosäure-Rest;
C) Ersatz des Glutaminsäure-Rests, des Methionin-Rests, des Tyrosin-Rests, des Isoleucin-Rests und des Alanin-Rests an den Positionen 22, 236, 461, 554 bzw. 786 in SEQ ID NO: 2 mit anderen Aminosäure-Resten; und
D) Kombinationen davon.

9. Verfahren gemäß Anspruch 7, wobei die zusätzliche Mutation ausgewählt ist aus der Gruppe bestehend aus:
A) Ersatz des Glutaminsäure-Rests an Position 560 in SEQ ID NO: 2 mit einem Lysin-Rest;
B) Ersatz des Phenylalanin-Rests an Position 566 in SEQ ID NO: 2 mit einem Valin-Rest;
C) Ersatz des Glutaminsäure-Rests, des Methionin-Rests, des Tyrosin-Rests, des Isoleucin-Rests und des Alanin-Rests an den Positionen 22, 236, 461, 554 bzw. 786 in SEQ ID NO: 2 mit einem Glycin-Rest, einem Valin-Rest, einem Cystein-Rest, einem SerinRest bzw. einem Valin-Rest; und
D) Kombinationen davon.

10. Verfahren gemäß mindestens einem der Ansprüche 1 bis 9, wobei das L-Aminosäure-herstellende Bakterium zu einer Gattung gehört, die ausgewählt ist aus der Gruppe bestehend aus Escherichia, Enterobacter, Erwinia, Klebsiella, Pantoea, Providencia, Salmonella, Serratia, Shigella und Morganella.

11. Verfahren gemäß mindestens einem der Ansprüche 1 bis 10, wobei die L-Aminosäure ausgewählt ist aus der Gruppe bestehend aus L-Threonin, L-Lysin, L-Histidin, L-Phenylalanin, L-Arginin, L-Tryptophan, L-Glutaminsäure und L-Leucin.

## Revendications

1. Procédé de production d'un acide L-aminé par fermentation, comprenant les étapes consistant :
A) à mettre en culture dans un milieu de culture contenant de l'éthanol une bactérie productrice d'acide L-aminé de la famille des Enterobacteriaceae possédant un alcool déshydrogénase, et
B) à isoler l'acide L-aminé du milieu de culture,
dans lequel le gène codant pour ledit alcool déshydrogénase est exprimé sous le contrôle d'un promoteur non natif qui fonctionne sous des conditions de culture aérobies.

2. Procédé selon la revendication 1, dans lequel ledit promoteur non natif est choisi dans le groupe constitué de P_{tac}, P_{lac}, Pₜᵣₚ, P_{trc}, P_{R}, et P_{L}.

3. Procédé selon la revendication 1 ou 2, dans lequel ledit alcool déshydrogénase est résistant à l'inactivation aérobie.

4. Procédé selon l'une quelconque des revendications 1 à 3, dans lequel ledit alcool déshydrogénase est issu d'une bactérie choisie dans le groupe constitué d'Escherichia coli, d'Erwinia carotovora, de Salmonella typhimurium, de Shigella flexneri, de Yersinia pestis, de Pantoea ananatis, de Lactobacillus plantarum, et de Lactococcus lactis.

5. Procédé selon l'une quelconque des revendications 1 à 4, dans lequel ledit alcool déshydrogénase comprend la séquence d'acides aminés présentée dans la SEQ ID NO : 2, à l'exception du résidu d'acide glutamique à la position 568 qui est remplacé par un autre résidu d'acide aminé autre qu'un résidu d'acide aspartique.

6. Procédé selon l'une quelconque des revendications 1 à 4, dans lequel ledit alcool déshydrogénase comprend la séquence d'acides aminés présentée dans la SEQ ID NO : 2, à l'exception du résidu d'acide glutamique à la position 568 qui est remplacé par un résidu de lysine.

7. Procédé selon la revendication 5 ou 6, dans lequel ledit alcool déshydrogénase a au moins une mutation supplémentaire qui est capable d'améliorer la croissance de ladite bactérie dans un milieu liquide qui contient de l'éthanol comme étant la seule source de carbone.

8. Procédé selon la revendication 7, dans lequel ladite mutation supplémentaire est choisie dans le groupe constitué :
A) d'un remplacement du résidu d'acide glutamique à la position 560 dans la SEQ ID NO : 2 par un autre résidu d'acide aminé ;
B) d'un remplacement du résidu de phénylalanine à la position 566 dans la SEQ ID NO : 2 par un autre résidu d'acide aminé ;
C) d'un remplacement du résidu d'acide glutamique, du résidu de méthionine, du résidu de tyrosine, du résidu d'isoleucine et du résidu d'alanine aux positions 22, 236, 461, 554, et 786, respectivement, dans la SEQ ID NO : 2 par d'autres résidus d'acides aminés ; et
D) des combinaisons de ceux-ci.

9. Procédé selon la revendication 7, dans lequel ladite mutation supplémentaire est choisie dans le groupe constitué :
A) d'un remplacement du résidu d'acide glutamique à la position 560 dans la SEQ ID NO : 2 par un résidu de lysine ;
B) d'un remplacement du résidu de phénylalanine à la position 566 dans la SEQ ID NO : 2 par un résidu de valine ;
C) d'un remplacement du résidu d'acide glutamique, du résidu de méthionine, du résidu de tyrosine, du résidu d'isoleucine et du résidu d'alanine aux positions 22, 236, 461, 554, et 786, respectivement, dans la SEQ ID NO : 2 par un résidu de glycine, un résidu de valine, un résidu de cystéine, un résidu de sérine, et un résidu de valine, respectivement ; et
D) des combinaisons de ceux-ci

10. Procédé selon l'une quelconque des revendications 1 à 9, dans lequel ladite bactérie productrice d'acide L-aminé appartient à un genre choisi dans le groupe constitué d'Escherichia, d'Enterobacter, d'Erwinia, de Klebsiella, de Pantoea, de Providencia, de Salmonella, de Serratia, de Shigella, et de Morganella.

11. Procédé selon l'une quelconque des revendications 1 à 10, dans lequel ledit acide L-aminé est choisi dans le groupe constitué de L-thréonine, de L-lysine, de L-histidine, de L-phénylalanine, de L-arginine, de L-tryptophane, d'acide L-glutamique et de L-leucine
